# EUROPEAN PATENT SPECIFICATION

(11) **EP 3 874 275 B1**
(45) Date of publication and mention of the grant of the patent: **23.11.2022**
(21) Application number: 19791288.4
(22) Date of filing: 30.10.2019
(51) Int. Cl.: G01N 33/68

(54) **BIOMARKERS OF SUBCLINICAL ATHEROSCLEROSIS**
BIOMARKER FÜR SUBKLINISCHE ATHEROSKLEROSE
BIOMARQUEURS DE L'ATHÉROSCLÉROSE SUBCLINIQUE

(30) Priority: 31.10.2018 EP 18382778; 12.07.2019 EP 19382594
(43) Date of publication of application: 08.09.2021
(73) Proprietor: Centro Nacional de Investigaciones Cardiovasculares Carlos III (F.S.P.), 28029 Madrid (ES); Fundacion Instituto De Investigacion Sanitaria Fundacion Jimenez Diaz, 28040 Madrid (ES); Universidad Autónoma De Madrid, 28049 Madrid (ES)
(72) Inventor: NÚÑEZ SÁNCHEZ, Estefanía, 28029 Madrid (ES); VÁZQUEZ COBOS, Jesús, 28029 Madrid (ES); FUSTER, Valentín, 28029 Madrid (ES); MARTINEZ LOPEZ, Diego, 28040 Madrid (ES); MARTIN VENTURA, José Luis, 28049 Madrid (ES); BONZON KULICHENKO, Elena, 28029 Madrid (ES); CALVO ALCOCER, Enrique, 28029 Madrid (ES)
(74) Representative: Elsy, David
(86) International application number: PCT/EP2019/079623
(87) International publication number: WO 2020/089282

(56) References cited:
- EP-A1- 2 851 688
- WO-A2-2007/140188
- US-A1- 2010 017 182
- ANTONIO FERNÁNDEZ-ORTIZ ET AL: "The Progression and Early detection of Subclinical Atherosclerosis (PESA) study: Rationale and design", AMERICAN HEART JOURNAL, vol. 166, no. 6, 1 December 2013 (2013-12-01), pages 990-998, XP055566758, AMSTERDAM, NL ISSN: 0002-8703, DOI: 10.1016/j.ahj.2013.08.024
- FERNÁNDEZ-ALVIRA JUAN MIGUEL ET AL: "Predicting Subclinical Atherosclerosis in Low-Risk Individuals Ideal Cardiovascular Health Score and Fuster-BEWAT Score", JOURNAL OF THE AMERICAN COLLEGE OF CARDIOLOGY, vol. 70, no. 20, November 2017 (2017-11), pages 2463-2473, XP085260977, ISSN: 0735-1097, DOI: 10.1016/J.JACC.2017.09.032

## Description

### FIELD OF THE INVENTION

The present invention pertains to the field of diagnostics. In particular, it relates to protein biomarkers for the screening, diagnosis and/or monitoring of subclinical atherosclerosis and methods and kits using thereof.

### BACKGROUND OF THE INVENTION

One of the challenges associated to the clinical management of atherosclerotic disease is that it is often diagnosed too late, usually when the condition is very advanced and lesions are already irreversible, or when it has caused clinical signs or events in organs or territories vascularized by the diseased arteries.

Primary prevention is currently based on the evaluation of modifiable risk factors according to standardized recommendations. However, it has been found that at every level of risk factor exposure, there is substantial variation in the amount of atherosclerosis (Fernandez-Ortiz A et al., Am Heart J 2013; 166:990-8).

Non-invasive measurement by imaging methods of the subclinical atherosclerotic burden in middle-aged people has the potential to improve assessment of cardiovascular risk and might contribute to a more effective prevention of cardiovascular events. Nevertheless, the most recent guidelines (Perk J. et al., Eur Heart J 2012; 33:1635-701) only recommend these imaging tests in asymptomatic adults considered at moderate risk (Fernandez-Ortiz A et al. 2013).

The Progression and Early detection of Subclinical Atherosclerosis (PESA) study is a longitudinal cohort study aimed at characterizing the prevalence of subclinical atherosclerosis and the determinants associated with the presence and progression of the disease in a middle-aged asymptomatic population.

Interestingly, a substantial proportion of asymptomatic individuals classified as low risk on the basis of traditional cardiovascular risk scores (i.e., FHS-10 year) were shown in the PESA study to have extensive atherosclerosis (Fernandez-Friera L et al., Circulation. 2015; 131:2104-2113).

The authors performed an analysis aiming to identify differences between participants with subclinical atherosclerosis in a single territory (focal disease) and those with multiple territories (intermediated or generalized disease). The results of this analysis evidenced a statistically significant association between extent of atherosclerosis and cardiovascular risk (CVR) scores and most CVR factors. Fernandez-Friera L et al. 2015 further discloses that most individuals classified as high risk by the traditional risk factor scores scales had subclinical atherosclerosis, with a high proportion having intermediate or generalized disease.

However, subclinical atherosclerosis was also present in 60% of the individuals classified as low risk, suggesting an association of atherosclerosis with characteristics not considered in standard risk scales. This was a striking observation since individuals presenting multi-territorial subclinical atherosclerosis, despite being classified as low risk, will be more likely to develop atherosclerotic cardiovascular diseases (ASCVD) events.

Fernandez-Friera L et al. 2017 (J Am Coll Cardiol. 2017; 70(24):2979-2991) also relates to the PESA study and reports that many middle-aged individuals without CVR factors present atherosclerosis. This study has found that LDL-cholesterol (LDL-C) levels, even at levels currently considered normal, is independently associated with the presence and extent of subclinical atherosclerosis. Thus, pointing to a reduction of LDL-C as a preventive measure to be adopted even in individuals considered to be at low risk.

Identification of biomarkers of atherosclerosis in the subclinical setting is technically more challenging than in a context of well-developed CVD or acute CV events, where the disease is expected to have a clear impact on plasma protein levels. Very few studies have assessed potential plasma protein biomarkers of subclinical atherosclerosis. Immunoassays have been used to quantify alterations in the specific plasma proteins adiponectin (Saarikoski LA et al., 2010) and TIMP4 (Oikonen M et al., 2012), showing that these proteins are decreased in individuals with asymptomatic subclinical carotid atherosclerosis in the Cardiovascular Risk in Young Finns cohort. A discovery proteomics analysis of the Cardiovascular Risk in Young Finns cohort found an association between FBLN1C plasma levels and plaque presence in middle-aged individuals(Bhosale SD et al., 2018), and the result was confirmed by targeted proteomics in the same population. However, in our study FBLN1C did not pass the stringent filtering criteria used.

Yin X et al., 2014 (Arteriosclerosis, thrombosis, and vascular biology 2014, 34:939-945) is concerned with the identification of new plasma protein biomarkers that individually or in aggregate predict risk of ASCVD. This document revealed an association between PIGR and myocardial infarction in single marker analysis.

Ngo D et al. 2016 (Circulation 2016, 134:270-285) aims to detect associations between plasma protein concentrations and Framingham risk score components in the Framingham Heart Study (FHS). In particular, it mentions an association between levels of PIGR in plasma and smoking. Smoking is a well stablished cardiac risk factor and is one of the variables in the FHS risk assessment algorithm. PIGR is further reported to be associated with the coronary heart disease FHS score.

Accordingly, there is a need to find new biomarkers for the screening, diagnosis and/or monitoring of individuals presenting subclinical atherosclerosis. Moreover, it is particularly desirable to identify markers which are independent of traditional CVR factors and scores, in order to be able to detect those subjects presenting subclinical atherosclerosis from those classified as having low cardiovascular risk.

### SUMMARY OF THE INVENTION

The present application provides the results of what is, to our knowledge, the deepest and largest mass spectrometry-based plasma proteomics analysis to date in the search for atherosclerosis-related biomarkers. The inventors used a proteomics platform capable of quantifying more than 1000 proteins from undepleted plasma samples in a cohort of 444 individuals from the PESA study. This analysis was possible by combining the quantitative accuracy and robustness provided by multiplexed isobaric labeling with well-validated and automated statistical workflows (Navarro P and Vazquez J, 2014; Garcia-Marques F et al, 2016; Trevisan-Herraz M et al ., 2018). A second analysis of plasma samples obtained from the same individuals at 3-year follow-up validated the results and identified a set of putative biomarker proteins whose association with atherosclerosis remained stable over time. This validation in the second visit was essential to reduce error sources in the discovery phase, to discard proteins with marked biological variability, and to concentrate the efforts on a robust set of biomarkers useful for the detection of subclinical atherosclerosis.

The levels of PIGR (Polymeric immunoglobulin receptor), APOA (Apolipoprotein(a)), HPT (haptoglobin), HEP2 (heparin cofactor 2), C5 (complement component 5), ITIH1 (Inter-alpha-trypsin inhibitor 1) and IGHA2 (immunoglobulin heavy constant alpha 2) in plasma as determined by liquid chromatography tandem mass spectrometry (LC-MS/MS) analysis have been found by the inventors to be associated to subclinical atherosclerotic disease independently of each other, and of established cardiovascular risk factors (e.g., age, smoking) and cardiovascular risk scales (e.g. FHS risk score).

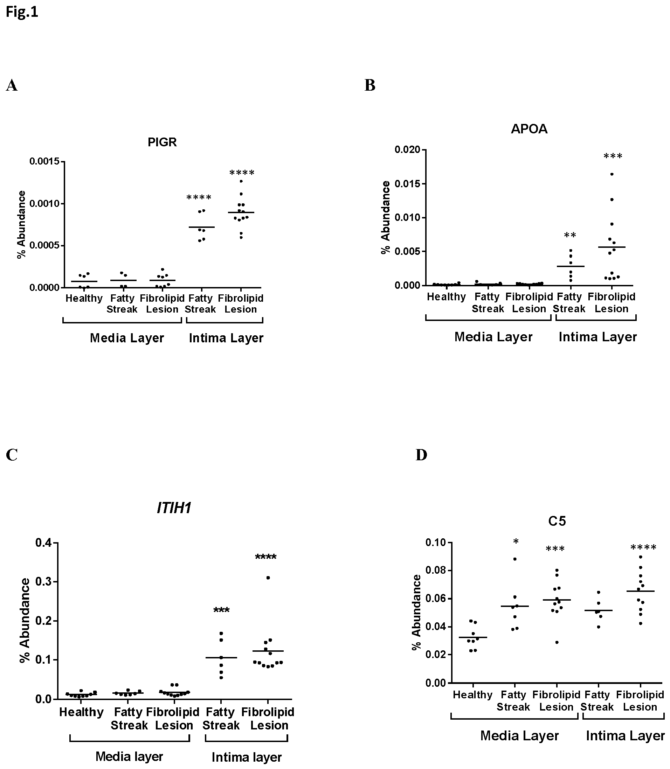

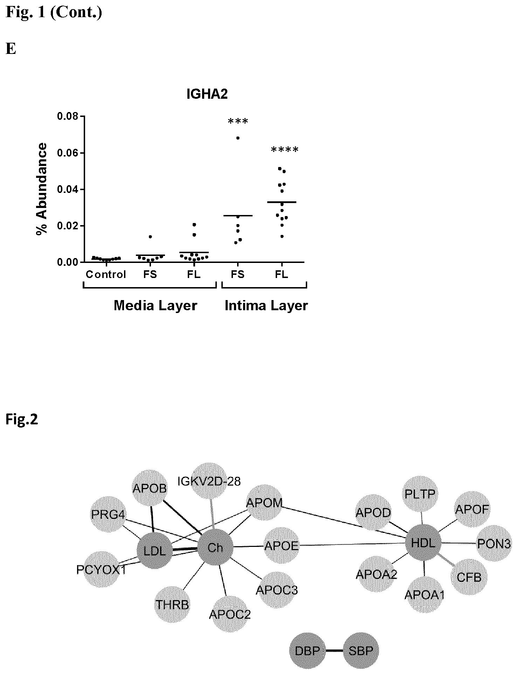

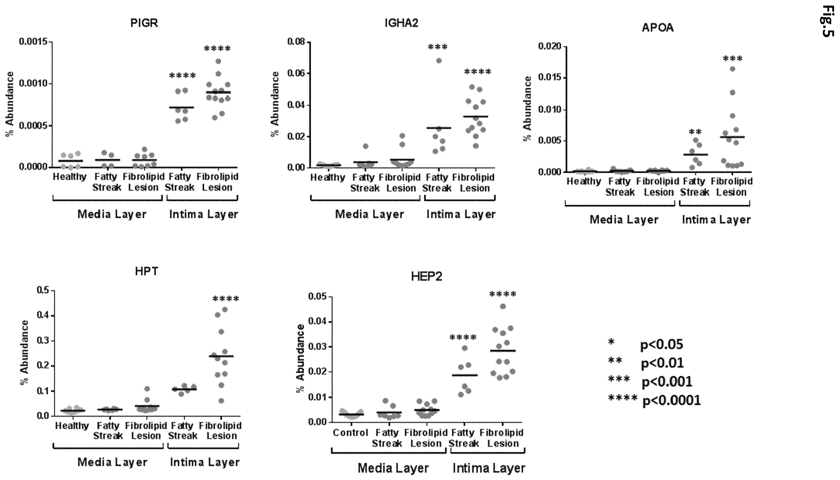

Moreover, the accumulation of these proteins in the media layer and mainly in the intima layer (plaque) has been shown to be higher as the plaque evolves from the fatty streak-type to the fibrolipid lesion-type (see Fig.1 and Fig.5). Thus, the levels of these proteins have also been associated by the inventors to the degree of advancement of atherosclerosis.

On this basis, these biomarkers, and preferably combinations thereof, are proposed by the inventors for use in a method for the screening, diagnosis and/or monitoring of subclinical atherosclerosis as described herein.

Accordingly, the first aspect of the invention relates to a method for the screening, diagnosis and/or monitoring of subclinical atherosclerosis in a subject, which comprises determining the levels of one or more protein markers as defined in claim 1.

In a second aspect not part of the invention here is provided a method for treating a subject having subclinical atherosclerosis, wherein said method comprises:
a. identifying a subject having subclinical atherosclerosis by the method for screening, diagnosis and/or monitoring as defined herein;
b. administering to said patient a therapeutically effective amount of a suitable treatment for preventing/reducing atherosclerotic plaques.

In a further aspect, the present invention refers to the use of a kit for determining the levels of one or more of the protein markers as described herein in a biological sample isolated from a subject. The kit may also contain instructions indicating how the materials within the kit may be used.

In still an additional aspect, the invention relates to the use of a kit of the preceding aspect in a method for the screening, diagnosis and/or monitoring of subclinical atherosclerosis as described herein.

### BRIEF DESCRIPTION OF THE DRAWINGS

**Figure 1****.** Quantitative proteomics analysis of the levels of PIGR (Fig. 1A), APOA (Fig. 1B), ITIH1 (Fig. 1C), C5 (Fig. 1D) and IGHA2 (Fig. 1E) in media and intima layers of samples from human aortas (controls; with atherosclerotic plaques with fatty streaks (FS) and with plaques with fibrolipidic lesions (FL)). Statistical significant results have been highlighted with asterisks (^{∗}), wherein ^{∗} means a p-value of 0.05, ^{∗∗} means a p-value of 0.01, ^{∗∗∗} means a p-value of 0.001 and ^{∗∗∗∗} means a p-value of 0.0001, and the reference value is the healthy group.
**Figure 2****.** Association of Plasma Protein levels with Traditional CV Risk Factors
   The network was constructed by analyzing the correlation of each one of the continuous risk factors with the plasma levels of each one of the proteins quantified by proteomics. Statistically significant correlations were selected by a Bonferroni-corrected p-value < 0.05 in both PESA-V1 and PESA-V2, and Pearson's correlation coefficients were used as weights to construct a correlation network using Cytoscape. The thickness of the lines is proportional to the weights (dark grey: positive correlation; light grey: negative correlation). Correlations between the risk factors are also shown. DBP: diastolic blood pressure; SBP: systolic blood pressure; LDL: low density lipoprotein; Ch: cholesterol; HDL: high density lipoprotein; APOB: apolipoprotein B-100; PRG4: proteoglycan 4; PCYOX1: prenylcysteine oxidase 1; P06309: Immunoglobulin kappa variable 2D-28 (GeneName IGKV2D-28); APOM: apolipoprotein M; APOE: apolipoprotein E; APOC3: apolipoprotein C-III; APOC2: apolipoprotein C-II; THRB: prothrombin; PLTP: phospholipid transfer protein; APOF: apolipoprotein F; PON3: Serum paraoxonase/lactonase 3; CFB: complement factor B; APOA1: apolipoprotein A-I; APOA2: apolipoprotein A-II.

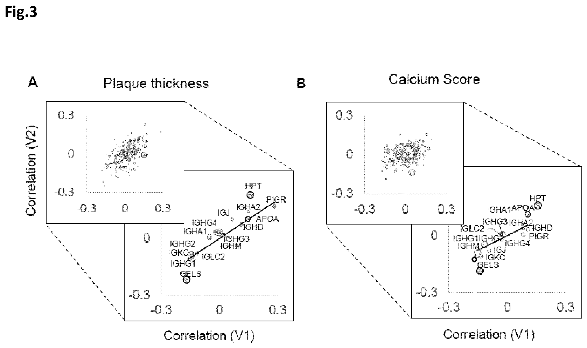

**Figure 3** Correlation of plasma proteins with plaque thickness and CACS in PESA-V1 and PESA-V2 cohorts.
   (A) Correlation of relative plasma protein levels with plaque thickness (Pearson's correlation coefficients) obtained in PESA-V1 are compared with those obtained in PESA-V2. Dot sizes are indicative of protein abundances in plasma (in number of quantified peptides per protein). Inset shows the behavior of proteins related to humoral immune response, and of other proteins yielding a significant correlation.
   (B) Correlation of relative plasma levels with CACS in PESA-V1 and in PESA-V2. Data are shown as in (A).

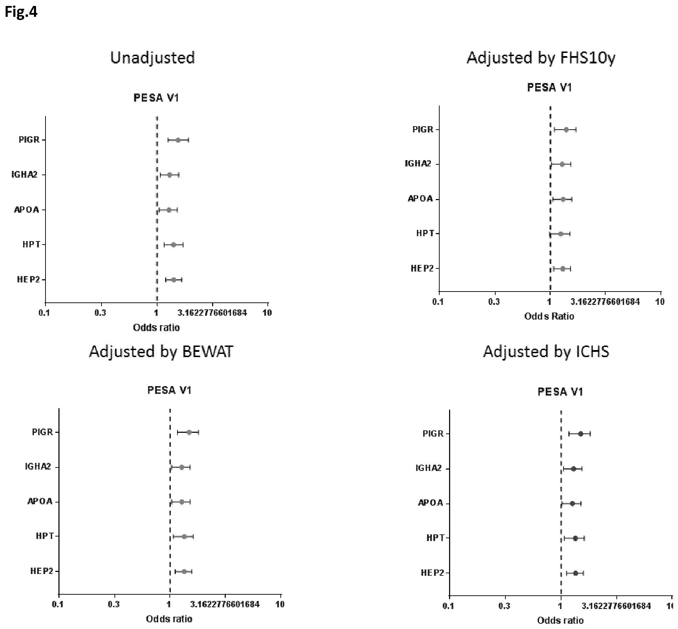

**Figure 4** Forest plots showing odds ratios of subclinical atherosclerosis (cases vs controls) in PESA-V1 for selected proteins.
   Odds ratios refer to relative protein values determined by proteomics and expressed in units of standard deviation, using univariate logistic regression models (Unadjusted), or multivariate models adjusted by common Risk Scores (FHS 10-year, BEWAT or ICHS). Error bars indicate 95% confidence intervals.
**Figure 5** Absolute protein abundance levels of the five selected proteins in human atherosclerotic tissue samples.
   The five proteins were subjected to absolute quantitation by proteomics and their levels expressed relative to the total protein amount of each sample. The levels were measured in samples from the media layer (obtained from healthy aortas, or from aortas showing early plaques (with fibrolipidic lessions or with fatty streaks)), or from the intima layer (with fibrolipidic lessions or with fatty streaks). Indicated statistical significances of protein abundance changes with respect to those of healthy samples is calculated using Student's t-test.
**Figure 6** Forest plots showing odds ratios of subclinical atherosclerosis (cases vs controls) in AWHS for selected proteins.
   Odds ratios refer to protein values as determined by turbidimetry and expressed in units of standard deviation, using univariate logistic regression models (Unadjusted), or multivariate models adjusted by common Risk Scores (FHS 10-year, BEWAT or ICHS). Error bars indicate 95% confidence intervals.
**Figure 7** Forest plots showing Areas Under the Curve after ROC analysis of protein biomarker panels for improved prediction of subclinical atherosclerosis in PESA-V1 and in AWHS
   Error bars indicate 95% confidence intervals of AUC values. Asterisks indicate statistical significane that AUC is significantly better than the one obtained using the risk score alone.
**Figure 8** Forest plots showing Areas Under the Curve after ROC analysis of protein biomarker panels for improved prediction of subclinical atherosclerosis in individuals with low risk (FHS 10-year < 0.1) in PESA-V1 and in AWHS. Error bars indicate 95% confidence intervals of AUC values. Asterisks indicate statistical significane that AUC is significantly better than 0.5.

### DETAILED DESCRIPTION OF THE INVENTION

### Definitions

The terms "subject", or "individual‴ are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "diagnosis", as used herein, refers both to the process of attempting to determine and/or identify a possible disease in a subject, i.e. the diagnostic procedure, and to the opinion reached by this process, i.e. the diagnostic opinion.

The term "screening" is understood herein as the examination or testing of a group of asymptomatic individuals pertaining to the general population, or of a group of individuals having one or more risk factors (i.e., a subject suspected of developing or at risk of developing a disease), with the objective of discriminating healthy individuals from those who have or are suspected of having a disease. A method of screening is generally used for the "early detection" of a disease. The expression "early detection" refers to detection before the presence of clinical signs.

The term "monitoring" as used herein refers to determining the evolution of the disease and/or the efficacy of a therapy, for example determining whether there is a remission of the disease; or on the contrary whether there is disease progression or a relapse.

The term "biomarker" as used herein refers to markers of disease which are typically substances found in a bodily sample that can be easily measured. The measured amount can correlate to underlying disease pathophysiology, such as presence or absence of subclinical atherosclerosis, or with its prognosis (i.e., likelihood of overcoming the underlying disease). In patients receiving treatment for their condition the measured amount may also correlate with responsiveness to therapy.

The term "therapeutically effective amount" as used herein refers to an amount that is effective, upon single or multiple dose administration to a subject (such as a human patient) in the prophylactic or therapeutic treatment of a disease, disorder or pathological condition.

The term "substantially identical" sequence as used herein refers to a sequence which is at least about 95%, preferably at least about 96%, 97%, 98%, or 99% identical to a reference sequence. Identity percentage between the two sequences can be determined by any means known in the art, for example the Needleman and Wunsch global alignment algorithm.

The term "affinity reagent" may refer to a ligand (e.g., antibody, peptide, protein, nucleic acid or small molecule) that selectively captures (binds to) a target molecule through specific molecular recognition, typically with a binding affinity in the nanomolar to sub-nanomolar range. For example, the affinity reagent may be an aptamer, antibody or antibody-mimetic.

The term "affinity" as used herein may refer to the equilibrium constant for the dissociation of an antigen with an antigen-binding molecule (KD), and is considered a measure for the binding strength between an antigenic determinant and an antigen-binding site on the antigen -binding molecule: the lesser the value of the KD, the stronger the binding strength between an antigenic determinant and the antigen-binding molecule (alternatively, the affinity can also be expressed as the association constant (KA), which is 1/KD). It will be clear to the skilled person that the dissociation constant may be the actual or apparent dissociation constant.

The term "aptamer" or "nucleic acid aptamer" as used herein may refer to an isolated or purified single-stranded nucleic acid (RNA or DNA) that binds with high specificity and affinity to a target through interactions other than Watson-Crick base pairing. An aptamer has a three dimensional structure that provides chemical contacts to specifically bind to a target. Unlike traditional nucleic acid binding, aptamer binding is not dependent upon a conserved linear base sequence, but rather a particular secondary or tertiary structure. That is, the nucleic acid sequences of aptamers are non-coding sequences. Any coding potential that an aptamer may possess is entirely fortuitous and plays no role whatsoever in the binding of an aptamer to a target. A typical minimized aptamer is 5-15 kDa in size (15-45 nucleotides), binds to a target with nanomolar to sub-nanomolar affinity, and discriminates against closely related targets (e.g., aptamers will typically not bind to other proteins from the same gene or functional family).

The term "antibody" as used herein may refer to an immunoglobulin or an antigen-binding fragment thereof. Unless otherwise specified, the term includes, but is not limited to, polyclonal, monoclonal, monospecific, multispecific, humanized, human, chimeric, synthetic, recombinant, hybrid, mutated, grafted, and *in vitro* generated antibodies. The antibody can include a constant region, or a portion thereof, such as the kappa, lambda, alpha, gamma, delta, epsilon and mu constant region genes. For example, heavy chain constant regions of the various isotypes can be used, including: IgG₁, IgG₂, IgG₃, IgG₄, IgM, IgA₁, IgA₂, IgD, and IgE. By way of example, the light chain constant region can be kappa or lambda. In certain embodiments, the term "antibody" may also refer to antibody derivatives, such as antibody-based fusion proteins (e.g. including a region equivalent to the Fc region of an immunoglobulin) or antibodies further modified to contain additional non-proteinaceous moieties, such as water soluble polymers, e.g. polyethylene glycol (PEG).

The terms "antigen-binding domain" and "antigen-binding fragment" refer to a part of an antibody molecule that comprises amino acids responsible for the specific binding between antibody and antigen. For certain antigens, the antigen-binding domain or antigen-binding fragment may only bind to a part of the antigen. The part of the antigen that is specifically recognized and bound by the antibody is referred to as the "epitope" or "antigenic determinant." Antigen-binding domains and antigen-binding fragments include Fab; a F(ab')₂ fragment (a bivalent fragment having two Fab fragments linked by a disulfide bridge at the hinge region); a Fv fragment; a single chain Fv fragment (scFv); a Fd fragment (having the two V_{H} and C_{H}1 domains); single domain antibodies (sdAbs; consisting of a single V_{H} domain), and other antibody fragments that retain antigen-binding function. The Fab fragment has V_{H}-C_{H}1 and V_{L}-C_{L} domains covalently linked by a disulfide bond between the constant regions. The Fᵥ fragment is smaller and has V_{H} and V_{L} domains non-covalently linked. The scFᵥ contains a flexible polypeptide that links (1) the C-terminus of V_{H} to the N-terminus of V_{L}, or (2) the C-terminus of V_{L} to the N-terminus of V_{H}. The sdAbs include heavy chain antibodies naturally devoid of light chains and single-domain antibodies derived from conventional four chain antibodies. These antigen-binding domains and fragments are obtained using conventional techniques known to those with skill in the art, and are evaluated for function in the same manner as are intact immunoglobulins.

The term "recombinant antibody" as used herein refers to an antibody produced or expressed using a recombinant expression vector, where the expression vector comprises a nucleic acid encoding the recombinant antibody, such that introduction of the expression vector into an appropriate host cell results in the production or expression of the recombinant antibody. Recombinant antibodies may be chimeric or humanized antibodies, mono- or multi-specific antibodies.

The term "antibody mimetic" as used herein may refer to protein-based scaffolds which have been engineered to bind therapeutic targets with affinity and specificity that match that of natural antibodies. Antibody mimetics have been developed utilizing an immunoglobulin-like fold, for example, fibronectin type III, NCAM and CTLA-4. Other mimetics scaffolds bearing no similarity to immunoglobulin folds have also been obtained. Non-limiting examples of said scaffolds are DARPins, anticalins, affibodies, adnectins, fynomers, etc. (see for instance, Weidle et al., 2013, Cancer Genomics & Proteomics, 10, 1-18; Lofblom, J. et al., 2011, Curr. Opin. Biotechnol., 22, 843-848; Banta, S. et al., 2010, Annu. Rev. Biomed. Eng., 15, 93-113).

### Methods of the Invention

In a first aspect, the present invention provides a method for the screening, diagnosis and/or monitoring of subclinical atherosclerosis in a subject, which comprises determining the levels of one or more protein markers as defined in claim 1.

Moreover, the accumulation of PIGR, IGHA2, APOA, HPT, HEP2, ITIH1 and C5 in plaque has been associated by the inventors to the degree of advancement of atherosclerosis (see Fig.1 and Fig. 5). Accordingly, the determination of the plasma levels of these proteins in the method of screening, diagnosing and/or monitoring of the invention may also provide information on or enable predicting the degree of advancement of subclinical atherosclerosis.

The term "subclinical atherosclerosis" as used herein refers to atherosclerosis in asymptomatic individuals. The expression "asymptomatic individuals" refers to those subjects which have not previously experienced clinical signs of cardiovascular disease, including for instance myocardial infarction, angina pectoris or stroke.

Preferably, "subclinical atherosclerosis" refers to the presence of atherosclerotic plaques in the carotid, aortic, or iliofemoral territories or CACS ≥1 in asymptomatic individuals. The multiterritorial "extent" of subclinical atherosclerosis is defined according to the number of vascular sites affected (right carotid, left carotid, abdominal aorta, right iliofemoral, left iliofemoral, and coronary arteries). For instance, as detailed in the Examples, in the PESA study participants were classified in four distinct categories according to the results of the imaging assays ("PESA score"), namely as disease free (0 vascular sites affected) or as having focal (1 site), intermediate (2-3 sites), or generalized (4-6 sites) atherosclerosis.

The "presence of atherosclerotic plaques" may be assessed by cross-sectional sweep of carotids, infrarenal abdominal aorta, and iliofemoral arteries. The term "plaque" typically refers to a focal protrusion into the arterial lumen of thickness >0.5 mm or >50% of the surrounding intima-media thickness or a diffuse thickness >1.5 mm measured between the media-adventitia and intima-lumen interfaces.

**Step (a)** of the method under the first aspect of the invention comprises determining in said biological sample the expression levels of one or more protein markers as defined above.

PIGR (Polymeric Immunoglobulin Receptor) binds polymeric IgA and IgM molecules at the basolateral surface of epithelial cells; the complex is then transported across the cell to be secreted at the apical surface. During this process a cleavage occurs that separates the extracellular (known as the secretory component) from the transmembrane segment. The canonical sequence of human PIGR is referred as SEQ ID NO:1 (UniProtKB Accession Number P01833-1; this is version 4 of the sequence of June 26, 2007):

MLLFVLTCLLAVFPAISTKSPIFGPEEVNSVEGNSVSITCYYPPTSVNRHTRKYWCRQGA
RGGCITLISSEGYVSSKYAGRANLTNFPENGTFVVNIAQLSQDDSGRYKCGLGINSRGLS
FDVSLEVSQGPGLLNDTKVYTVDLGRTVTINCPFKTENAQKRKSLYKQIGLYPVLVIDSS
GYVNPNYTGRIRLDIQGTGQLLFSVVINQLRLSDAGQYLCQAGDDSNSNKKNADLQVLKP
EPELVYEDLRGSVTFHCALGPEVANVAKFLCRQSSGENCDVVVNTLGKRAPAFEGRILLN
PQDKDGSFSVVITGLRKEDAGRYLCGAHSDGQLQEGSPIQAWQLFVNEESTIPRSPTVVK
GVAGGSVAVLCPYNRKESKSIKYWCLWEGAQNGRCPLLVDSEGWVKAQYEGRLSLLEEPG
NGTFTVILNQLTSRDAGFYWCLTNGDTLWRTTVEIKIIEGEPNLKVPGNVTAVLGETLKV
PCHFPCKFSSYEKYWCKWNNTGCQALPSQDEGPSKAFVNCDENSRLVSLTLNLVTRADEG
WYWCGVKQGHFYGETAAVYVAVEERKAAGSRDVSLAKADAAPDEKVLDSGFREIENKAIQ
DPRLFAEEKAVADTRDQADGSRASVDSGSSEEQGGSSRALVSTLVPLGLVLAVGAVAVGV
ARARHRKNVDRVSIRSYRTDISMSDFENSREFGANDNMGASSITQETSLGGKEEFVATTE
STTETKEPKKAKRSSKEEAEMAYKDFLLQSSTVAAEAQDGPQEA

The term "PIGR" as used herein refers to human PIGR protein with SEQ ID NO:1 and to sequences substantially identical thereto. Preferably, said sequence is SEQ ID NO:1.

The canonical sequence of human Immunoglobulin heavy constant alpha 2 (IGHA2) is referred as SEQ ID NO:2 (UniProtKB Accession Number P01877-1; this is version 4 of the sequence of March 15, 2017):

ASPTSPKVFPLSLDSTPQDGNVVVACLVQGFFPQEPLSVTWSESGQNVTARNFPPSQDAS
GDLYTTSSQLTLPATQCPDGKSVTCHVKHYTNSSQDVTVPCRVPPPPPCCHPRLSLHRPA
LEDLLLGSEANLTCTLTGLRDASGATFTWTPSSGKSAVQGPPERDLCGCYSVSSVLPGCA
QPWNHGETFTCTAAHPELKTPLTANITKSGNTFRPEVHLLPPPSEELALNELVTLTCLAR
GFSPKDVLVRWLQGSQELPREKYLTWASRQEPSQGTTTYAVTSILRVAAEDWKKGETFSC
MVGHEALPLAFTQKTIDRMAGKPTHINVSVVMAEADGTCY

The term "IGHA2" as used herein refers to human IGHA2 protein with SEQ ID NO:2 and to sequences substantially identical thereto. Preferably, said sequence is SEQ ID NO:2.

The presence of PIGR and IGHA2 in early atherosclerotic lesions is a new finding. PIGR and IGHA2 are functionally related and are known to be involved in the humoral immune response. IgA immunoglobulins are thought to be the first line of antigen-specific immune protection at mucosal surfaces, while PIGR is a receptor that binds circulating polymeric IgA and IgM at the basolateral surface of intestinal epithelial cells and transports them across the cell to be secreted at the apical surface into intestinal lumen (Kaetzel CS, 2005; Wines BD and Hogarth PM, 2006). While atherosclerosis is today considered a chronic immune-inflammatory disease with a strong autoimmune component and a clear contribution from both innate and adaptive immunity (Hansson GK and Hermansson A, 2011), there is little information about the role of IgA antibodies in CV diseases (Tsiantoulas D et al., 2014). PIGR levels have been reported to increase in sputum and blood of smokers and chronic obstructive pulmonary disease patients (Ohlmeier S et al., 2012); moreover, PIGR was linked to smoking in a cohort from the Framingham Offspring Study, although PIGR levels were not associated with CV disease in that cohort (Ngo D et al., 2016). The only link we are aware of between PIGR and atherosclerosis is the elevated level of this protein in extracellular vesicles from individuals with acute coronary syndrome, although inclusion of this parameter did not improve disease detection over conventional risk factors or troponin I (de Hoog VC et al., 2013). No association between the IgA isoform IGHA2 and atherosclerosis has been reported before; however, elevated total serum IgA (which includes IGHA1, the most abundant isoform, and IGHA2) has been reported in relation to advanced atherosclerosis or end-stage CV events; for example, in patients with severe atherosclerosis (Muscari A et al., 1988) or with previous myocardial infarction or other major ischemic events (Muscari A et al., 1993). Levels of IgA, together with those of IgE and IgG, but not IgM, also correlate with myocardial infarction and cardiac death in dyslipidemic men (Kovanen PT et al., 1998).

Apolipoprotein(a) (APOA) is the main constituent of lipoprotein(a) (LPA). APOA is known to be proteolytically cleaved and fragments accumulate in atherosclerotic lesions. APOA is well-known to accumulate into the intima; this protein contains lysine-binding sites that allows it to bind tightly to exposed surfaces on denuded endothelium, enter, and accumulate into subintimal spaces (Tsimikas S, 2017). There is abundant evidence linking APOA to atherosclerosis. While the physiological role of Lp(a) in human is still not fully elucidated (Orso E and Schmitz G, 2017), the particle has been identified as independent predictor of coronary artery calcification (Greif M et al., 2013), and epidemiology supports a strong association between elevated Lp(a) and atherosclerotic CV disease outcomes (Ellis KL et al., 2017), suggesting that Lp(a) plays a causal role in the disease (Ellis KL and Watts GF, 2018). There are no treatments that specifically lower Lp(a), and hence the potential effect of lowering Lp(a) on CVD risk has not been demonstrated; nevertheless, some authorities recommend testing Lp(a) levels in certain clinical situations, for example in individuals with a personal or family history of premature CAD or at intermediate/high risk of CAD (Ellis KL and Watts GF, 2018).

Our results provide the first demonstration of the potential of APOA protein levels as an independent predictor of atherosclerosis in the subclinical phase, complementing the information provided not only by known risk factors but also by IGAH2 and HPT.

The canonical sequence of human APOA is referred as SEQ ID NO:3 (UniProtKB Accession Number P08519; this is version 1 of the sequence of August 1, 1988):

MEHKEVVLLLLLFLKSAAPEQSHVVQDCYHGDGQSYRGTYSTTVTGRTCQAWSSMTPHQH
NRTTENYPNAGLIMNYCRNPDAVAAPYCYTRDPGVRWEYCNLTQCSDAEGTAVAPPTVTP
VPSLEAPSEQAPTEQRPGVQECYHGNGQSYRGTYSTTVTGRTCQAWSSMTPHSHSRTPEY
YPNAGLIMNYCRNPDAVAAPYCYTRDPGVRWEYCNLTQCSDAEGTAVAPPTVTPVPSLEA
PSEQAPTEQRPGVQECYHGNGQSYRGTYSTTVTGRTCQAWSSMTPHSHSRTPEYYPNAGL
IMNYCRNPDAVAAPYCYTRDPGVRWEYCNLTQCSDAEGTAVAPPTVTPVPSLEAPSEQAP
TEQRPGVQECYHGNGQSYRGTYSTTVTGRTCQAWSSMTPHSHSRTPEYYPNAGLIMNYCR
NPDAVAAPYCYTRDPGVRWEYCNLTQCSDAEGTAVAPPTVTPVPSLEAPSEQAPTEQRPG
VQECYHGNGQSYRGTYSTTVTGRTCQAWSSMTPHSHSRTPEYYPNAGLIMNYCRNPDAVA
APYCYTRDPGVRWEYCNLTQCSDAEGTAVAPPTVTPVPSLEAPSEQAPTEQRPGVQECYH
GNGQSYRGTYSTTVTGRTCQAWSSMTPHSHSRTPEYYPNAGLIMNYCRNPDAVAAPYCYT
RDPGVRWEYCNLTQCSDAEGTAVAPPTVTPVPSLEAPSEQAPTEQRPGVQECYHGNGQSY
RGTYSTTVTGRTCQAWSSMTPHSHSRTPEYYPNAGLIMNYCRNPDAVAAPYCYTRDPGVR
WEYCNLTQCSDAEGTAVAPPTVTPVPSLEAPSEQAPTEQRPGVQECYHGNGQSYRGTYST
TVTGRTCQAWSSMTPHSHSRTPEYYPNAGLIMNYCRNPDAVAAPYCYTRDPGVRWEYCNL
TQCSDAEGTAVAPPTVTPVPSLEAPSEQAPTEQRPGVQECYHGNGQSYRGTYSTTVTGRT
CQAWSSMTPHSHSRTPEYYPNAGLIMNYCRNPDAVAAPYCYTRDPGVRWEYCNLTQCSDA
EGTAVAPPTVTPVPSLEAPSEQAPTEQRPGVQECYHGNGQSYRGTYSTTVTGRTCQAWSS
MTPHSHSRTPEYYPNAGLIMNYCRNPDAVAAPYCYTRDPGVRWEYCNLTQCSDAEGTAVA
PPTVTPVPSLEAPSEQAPTEQRPGVQECYHGNGQSYRGTYSTTVTGRTCQAWSSMTPHSH
SRTPEYYPNAGLIMNYCRNPDAVAAPYCYTRDPGVRWEYCNLTQCSDAEGTAVAPPTVTP
VPSLEAPSEQAPTEQRPGVQECYHGNGQSYRGTYSTTVTGRTCQAWSSMTPHSHSRTPEY

YPNAGLIMNYCRNPDAVAAPYCYTRDPGVRWEYCNLTQCSDAEGTAVAPPTVTPVPSLEA
PSEQAPTEQRPGVQECYHGNGQSYRGTYSTTVTGRTCQAWSSMTPHSHSRTPEYYPNAGL
IMNYCRNPDAVAAPYCYTRDPGVRWEYCNLTQCSDAEGTAVAPPTVTPVPSLEAPSEQAP
TEQRPGVQECYHGNGQSYRGTYSTTVTGRTCQAWSSMTPHSHSRTPEYYPNAGLIMNYCR
NPDAVAAPYCYTRDPGVRWEYCNLTQCSDAEGTAVAPPTVTPVPSLEAPSEQAPTEQRPG
VQECYHGNGQSYRGTYSTTVTGRTCQAWSSMTPHSHSRTPEYYPNAGLIMNYCRNPDAVA
APYCYTRDPGVRWEYCNLTQCSDAEGTAVAPPTVTPVPSLEAPSEQAPTEQRPGVQECYH
GNGQSYRGTYSTTVTGRTCQAWSSMTPHSHSRTPEYYPNAGLIMNYCRNPDAVAAPYCYT
RDPGVRWEYCNLTQCSDAEGTAVAPPTVTPVPSLEAPSEQAPTEQRPGVQECYHGNGQSY
RGTYSTTVTGRTCQAWSSMTPHSHSRTPEYYPNAGLIMNYCRNPDAVAAPYCYTRDPGVR
WEYCNLTQCSDAEGTAVAPPTVTPVPSLEAPSEQAPTEQRPGVQECYHGNGQSYRGTYST
TVTGRTCQAWSSMTPHSHSRTPEYYPNAGLIMNYCRNPDAVAAPYCYTRDPGVRWEYCNL
TQCSDAEGTAVAPPTVTPVPSLEAPSEQAPTEQRPGVQECYHGNGQSYRGTYSTTVTGRT
CQAWSSMTPHSHSRTPEYYPNAGLIMNYCRNPDAVAAPYCYTRDPGVRWEYCNLTQCSDA
EGTAVAPPTVTPVPSLEAPSEQAPTEQRPGVQECYHGNGQSYRGTYSTTVTGRTCQAWSS
MTPHSHSRTPEYYPNAGLIMNYCRNPDAVAAPYCYTRDPGVRWEYCNLTQCSDAEGTAVA
PPTVTPVPSLEAPSEQAPTEQRPGVQECYHGNGQSYRGTYSTTVTGRTCQAWSSMTPHSH
SRTPEYYPNAGLIMNYCRNPDAVAAPYCYTRDPGVRWEYCNLTQCSDAEGTAVAPPTVTP
VPSLEAPSEQAPTEQRPGVQECYHGNGQSYRGTYSTTVTGRTCQAWSSMTPHSHSRTPEY
YPNAGLIMNYCRNPDAVAAPYCYTRDPGVRWEYCNLTQCSDAEGTAVAPPTVTPVPSLEA
PSEQAPTEQRPGVQECYHGNGQSYRGTYSTTVTGRTCQAWSSMTPHSHSRTPEYYPNAGL
IMNYCRNPDAVAAPYCYTRDPGVRWEYCNLTQCSDAEGTAVAPPTVTPVPSLEAPSEQAP
TEQRPGVQECYHGNGQSYRGTYSTTVTGRTCQAWSSMTPHSHSRTPEYYPNAGLIMNYCR
NPDAVAAPYCYTRDPGVRWEYCNLTQCSDAEGTAVAPPTVTPVPSLEAPSEQAPTEQRPG
VQECYHGNGQSYRGTYSTTVTGRTCQAWSSMTPHSHSRTPEYYPNAGLIMNYCRNPDAVA
APYCYTRDPGVRWEYCNLTQCSDAEGTAVAPPTVTPVPSLEAPSEQAPTEQRPGVQECYH
GNGQSYRGTYSTTVTGRTCQAWSSMTPHSHSRTPEYYPNAGLIMNYCRNPDAVAAPYCYT
RDPGVRWEYCNLTQCSDAEGTAVAPPTVTPVPSLEAPSEQAPTEQRPGVQECYHGNGQSY
RGTYSTTVTGRTCQAWSSMTPHSHSRTPEYYPNAGLIMNYCRNPDAVAAPYCYTRDPGVR
WEYCNLTQCSDAEGTAVAPPTVTPVPSLEAPSEQAPTEQRPGVQECYHGNGQSYRGTYST
TVTGRTCQAWSSMTPHSHSRTPEYYPNAGLIMNYCRNPDAVAAPYCYTRDPGVRWEYCNL
TQCSDAEGTAVAPPTVTPVPSLEAPSEQAPTEQRPGVQECYHGNGQSYRGTYSTTVTGRT
CQAWSSMTPHSHSRTPEYYPNAGLIMNYCRNPDAVAAPYCYTRDPGVRWEYCNLTQCSDA
EGTAVAPPTVTPVPSLEAPSEQAPTEQRPGVQECYHGNGQSYRGTYSTTVTGRTCQAWSS
MTPHSHSRTPEYYPNAGLIMNYCRNPDPVAAPYCYTRDPSVRWEYCNLTQCSDAEGTAVA
PPTITPIPSLEAPSEQAPTEQRPGVQECYHGNGQSYQGTYFITVTGRTCQAWSSMTPHSH
SRTPAYYPNAGLIKNYCRNPDPVAAPWCYTTDPSVRWEYCNLTRCSDAEWTAFVPPNVIL
APSLEAFFEQALTEETPGVQDCYYHYGQSYRGTYSTTVTGRTCQAWSSMTPHQHSRTPEN
YPNAGLTRNYCRNPDAEIRPWCYTMDPSVRWEYCNLTQCLVTESSVLATLTVVPDPSTEA
SSEEAPTEQSPGVQDCYHGDGQSYRGSFSTTVTGRTCQSWSSMTPHWHQRTTEYYPNGGL
TRNYCRNPDAEISPWCYTMDPNVRWEYCNLTQCPVTESSVLATSTAVSEQAPTEQSPTVQ
DCYHGDGQSYRGSFSTTVTGRTCQSWSSMTPHWHQRTTEYYPNGGLTRNYCRNPDAEIRP
WCYTMDPSVRWEYCNLTQCPVMESTLLTTPTVVPVPSTELPSEEAPTENSTGVQDCYRGD
GQSYRGTLSTTITGRTCQSWSSMTPHWHRRIPLYYPNAGLTRNYCRNPDAEIRPWCYTMD
PSVRWEYCNLTRCPVTESSVLTTPTVAPVPSTEAPSEQAPPEKSPVVQDCYHGDGRSYRG
ISSTTVTGRTCQSWSSMIPHWHQRTPENYPNAGLTENYCRNPDSGKQPWCYTTDPCVRWE
YCNLTQCSETESGVLETPTVVPVPSMEAHSEAAPTEQTPVVRQCYHGNGQSYRGTFSTTV
TGRTCQSWSSMTPHRHQRTPENYPNDGLTMNYCRNPDADTGPWCFTMDPSIRWEYCNLTR
CSDTEGTVVAPPTVIQVPSLGPPSEQDCMFGNGKGYRGKKATTVTGTPCQEWAAQEPHRH
STFIPGTNKWAGLEKNYCRNPDGDINGPWCYTMNPRKLFDYCDIPLCASSSFDCGKPQVE
PKKCPGSIVGGCVAHPHSWPWQVSLRTRFGKHFCGGTLISPEWVLTAAHCLKKSSRPSSY
KVILGAHQEVNLESHVQEIEVSRLFLEPTQADIALLKLSRPAVITDKVMPACLPSPDYMV
TARTECYITGWGETQGTFGTGLLKEAQLLVIENEVCNHYKYICAEHLARGTDSCQGDSGG
PLVCFEKDKYILQGVTSWGLGCARPNKPGVYARVSRFVTWIEGMMRNN

The term "APOA" as used herein refers to human APOA protein with SEQ ID NO:3 and to sequences substantially identical thereto. Preferably, said sequence is SEQ ID NO:3.

Inter-alpha-trypsin inhibitor heavy chain H1 (ITIH1) may act as a carrier of hyaluronan in serum or as a binding protein between hyaluronan and other matrix protein, including those on cell surfaces in tissues to regulate the localization, synthesis and degradation of hyaluronan which are essential to cells undergoing biological processes. The canonical sequence of human ITIH1 is referred as SEQ ID NO:4 (UniProtKB Accession Number P19827; this is version 3 of the sequence of July 15, 1998):

MDGAMGPRGLLLCMYLVSLLILQAMPALGSATGRSKSSEKRQAVDTAVDGVFIRSLKVNC
KVTSRFAHYVVTSQVVNTANEAREVAFDLEIPKTAFISDFAVTADGNAFIGDIKDKVTAW
KQYRKAAISGENAGLVRASGRTMEQFTIHLTVNPQSKVTFQLTYEEVLKRNHMQYEIVIK
VKPKQLVHHFEIDVDIFEPQGISKLDAQASFLPKELAAQTIKKSFSGKKGHVLFRPTVSQ
QQSCPTCSTSLLNGHFKVTYDVSRDKICDLLVANNHFAHFFAPQNLTNMNKNVVFVIDIS
GSMRGQKVKQTKEALLKILGDMQPGDYFDLVLFGTRVQSWKGSLVQASEANLQAAQDFVR
GFSLDEATNLNGGLLRGIEILNQVQESLPELSNHASILIMLTDGDPTEGVTDRSQILKNV
RNAIRGRFPLYNLGFGHNVDFNFLEVMSMENNGRAQRIYEDHDATQQLQGFYSQVAKPLL
VDVDLQYPQDAVLALTQNHHKQYYEGSEIVVAGRIADNKQSSFKADVQAHGEGQEFSITC
LVDEEEMKKLLRERGHMLENHVERLWAYLTIQELLAKRMKVDREERANLSSQALQMSLDY
GFVTPLTSMSIRGMADQDGLKPTIDKPSEDSPPLEMLGPRRTFVLSALQPSPTHSSSNTQ
RLPDRVTGVDTDPHFIIHVPQKEDTLCFNINEEPGVILSLVQDPNTGFSVNGQLIGNKAR
SPGQHDGTYFGRLGIANPATDFQLEVTPQNITLNPGFGGPVFSWRDQAVLRQDGVVVTIN
KKRNLVVSVDDGGTFEVVLHRVWKGSSVHQDFLGFYVLDSHRMSARTHGLLGQFFHPIGF
EVSDIHPGSDPTKPDATMVVRNRRLTVTRGLQKDYSKDPWHGAEVSCWFIHNNGAGLIDG
AYTDYIVPDIF

The term "ITIH1" as used herein refers to human ITIH1 protein with SEQ ID NO:4 and to sequences substantially identical thereto. Preferably, said sequence is SEQ ID NO:4.

Human complement C5 activation by a C5 convertase initiates the spontaneous assembly of the late complement components, C5-C9, into the membrane attack complex. The canonical sequence of human C5 is referred as SEQ ID NO:5 (UniProtKB Accession Number P01031-1; this is version 4 of the sequence of February 5, 2008):

MGLLGILCFLIFLGKTWGQEQTYVISAPKIFRVGASENIVIQVYGYTEAFDATISIKSYP
DKKFSYSSGHVHLSSENKFQNSAILTIQPKQLPGGQNPVSYVYLEVVSKHFSKSKRMPIT
YDNGFLFIHTDKPVYTPDQSVKVRVYSLNDDLKPAKRETVLTFIDPEGSEVDMVEEIDHI
GIISFPDFKIPSNPRYGMWTIKAKYKEDFSTTGTAYFEVKEYVLPHFSVSIEPEYNFIGY
KNFKNFEITIKARYFYNKVVTEADVYITFGIREDLKDDQKEMMQTAMQNTMLINGIAQVT

FDSETAVKELSYYSLEDLNNKYLYIAVTVIESTGGFSEEAEIPGIKYVLSPYKLNLVATP
LFLKPGIPYPIKVQVKDSLDQLVGGVPVTLNAQTIDVNQETSDLDPSKSVTRVDDGVASF
VLNLPSGVTVLEFNVKTDAPDLPEENQAREGYRAIAYSSLSQSYLYIDWTDNHKALLVGE
HLNIIVTPKSPYIDKITHYNYLILSKGKIIHFGTREKFSDASYQSINIPVTQNMVPSSRL
LVYYIVTGEQTAELVSDSVWLNIEEKCGNQLQVHLSPDADAYSPGQTVSLNMATGMDSWV
ALAAVDSAVYGVQRGAKKPLERVFQFLEKSDLGCGAGGGLNNANVFHLAGLTFLTNANAD
DSQENDEPCKEILRPRRTLQKKIEEIAAKYKHSVVKKCCYDGACVNNDETCEQRAARISL
GPRCIKAFTECCVVASQLRANISHKDMQLGRLHMKTLLPVSKPEIRSYFPESWLWEVHLV
PRRKQLQFALPDSLTTWEIQGVGISNTGICVADTVKAKVFKDVFLEMNIPYSVVRGEQIQ
LKGTVYNYRTSGMQFCVKMSAVEGICTSESPVIDHQGTKSSKCVRQKVEGSSSHLVTFTV
LPLEIGLHNINFSLETWFGKEILVKTLRVVPEGVKRESYSGVTLDPRGIYGTISRRKEFP
YRIPLDLVPKTEIKRILSVKGLLVGEILSAVLSQEGINILTHLPKGSAEAELMSVVPVFY
VFHYLETGNHWNIFHSDPLIEKQKLKKKLKEGMLSIMSYRNADYSYSVWKGGSASTWLTA
FALRVLGQVNKYVEQNQNSICNSLLWLVENYQLDNGSFKENSQYQPIKLQGTLPVEAREN
SLYLTAFTVIGIRKAFDICPLVKIDTALIKADNFLLENTLPAQSTFTLAISAYALSLGDK
THPQFRSIVSALKREALVKGNPPIYRFWKDNLQHKDSSVPNTGTARMVETTAYALLTSLN
LKDINYVNPVIKWLSEEQRYGGGFYSTQDTINAIEGLTEYSLLVKQLRLSMDIDVSYKHK
GALHNYKMTDKNFLGRPVEVLLNDDLIVSTGFGSGLATVHVTTVVHKTSTSEEVCSFYLK
IDTQDIEASHYRGYGNSDYKRIVACASYKPSREESSSGSSHAVMDISLPTGISANEEDLK
ALVEGVDQLFTDYQIKDGHVILQLNSIPSSDFLCVRFRIFELFEVGFLSPATFTVYEYHR
PDKQCTMFYSTSNIKIQKVCEGAACKCVEADCGQMQEELDLTISAETRKQTACKPEIAYA
YKVSITSITVENVFVKYKATLLDIYKTGEAVAEKDSEITFIKKVTCTNAELVKGRQYLIM
GKEALQIKYNFSFRYIYPLDSLTWIEYWPRDTTCSSCQAFLANLDEFAEDIFLNGC

The term "C5" as used herein refers to human C5 protein with SEQ ID NO:5 and to sequences substantially identical thereto. Preferably, said sequence is SEQ ID NO:5.

The presence and accumulation of HPT in the intimal layer is consistent with its antioxidant role in promoting clearance of free hemoglobin released from red blood cells (RBC); however, the accumulation of HPT in the earliest phases of plaque formation has not been documented before. In this regard, the presence of redox-active iron, hemoglobin and glycophorin A in early atherosclerotic plaques has been recently revealed, implicating intimal RBC infiltration as one of the initial triggers for intimal oxidation (Delbosc S et al., 2017).

Several human studies have indicated that the HPT 2-2 phenotype may be associated with CVD in type 2 diabetes mellitus (Levy AP et al., 2002; Levy AP et al., 2004). Increased HPT levels have been previously observed in CAD patients (Lee CW et al., 2013) and were predictive of CV events(Holme I et al., 2009). HPT is considered an acute phase protein, and, like other proteins of this class such as CRP, fibrinogen, and serum-amyloid protein, has been used as a marker of inflammation. These markers, alone or in combination, improve the prediction of major CV events such as acute myocardial infarction and ischemic stroke (Holme I et al., 2009; Engstrom G et al., 2002; Holme I et al., 2010; Brea D et al., 2009; Sabatine MS et al., 2007). However, these proteins also detect other inflammation-associated diseases; for instance, similar increases in HPT and fibrinogen occur in patients with renal dysfunction or advanced CV disease, reflecting shared inflammatory events is these conditions (Luczak M et al., 2011). Moreover, a recent study of associations between acute phase proteins and CV outcome in 581 patients found no association between HPT and coronary plaque characteristics or clinical events (Battes LC et al., 2014). In a discovery proteomics study, the inflammatory markers SAA-1 and CRP were found to be increased in patients with stable atherosclerosis or acute coronary syndrome, but no changes were detected in HPT levels (Kristensen LP et al., 2014). Our present results showed no association of these acute phase proteins with plaque thickness or CACS. These results suggest that HPT plasma levels do not strictly track the behavior of inflammatory markers.

The canonical sequence of human haptoglobin is referred as SEQ ID NO:9 (UniProtKB Accession Number P00738; this is version 1 of the sequence of July 21, 1986):

MSALGAVIALLLWGQLFAVDSGNDVTDIADDGCPKPPEIAHGYVEHSVRYQCKNYYKLRT
EGDGVYTLNDKKQWINKAVGDKLPECEADDGCPKPPEIAHGYVEHSVRYQCKNYYKLRTE
GDGVYTLNNEKQWINKAVGDKLPECEAVCGKPKNPANPVQRILGGHLDAKGSFPWQAKMV
SHHNLTTGATLINEQWLLTTAKNLFLNHSENATAKDIAPTLTLYVGKKQLVEIEKVVLHP
NYSQVDIGLIKLKQKVSVNERVMPICLPSKDYAEVGRVGYVSGWGRNANFKFTDHLKYVM
LPVADQDQCIRHYEGSTVPEKKTPKSPVGVQPILNEHTFCAGMSKYQEDTCYGDAGSAFA
VHDLEEDTWYATGILSFDKSCAVAEYGVYVKVTSIQDWVQKTIAEN

The term "HPT" as used herein refers to human haptoglobin protein with SEQ ID NO:9 and to sequences substantially identical thereto. Preferably, said sequence is SEQ ID NO:9.

HEP2 has been previously found in the lipid-rich core of atheromas (Rau JC et al., 2009), but has not been described in early plaques. Reduced expression of HEP2 has been associated with aggravated atherosclerosis (Kanagawa Y et al., 2001; Takamori N et al., 2004), due presumably to its role as inhibitor of thrombin. Similarly, plasma HEP2 activity has been inversely associated to the prevalence of arterial disease (Aihara K et al., 2009), individuals with high levels of HEP2 have been shown to have less atherosclerosis (Aihara K et al., 2004) and HEP2 is thought to play a protective role against vascular and cardiac remodeling (Aihara K et al., 2009; Ikeda Y et al., 2012). Hence, our finding that HEP2 is increased in subclinical atherosclerosis was not expected from previous studies.

The canonical sequence of human heparin cofactor 2 (HEP2) is referred as SEQ ID NO:10 (UniProtKB Accession Number P05546; this is version 3 of the sequence of November 1, 1991):

MKHSLNALLIFLIITSAWGGSKGPLDQLEKGGETAQSADPQWEQLNNKNLSMPLLPADFH
KENTVTNDWIPEGEEDDDYLDLEKIFSEDDDYIDIVDSLSVSPTDSDVSAGNILQLFHGK
SRIQRLNILNAKFAFNLYRVLKDQVNTFDNIFIAPVGISTAMGMISLGLKGETHEQVHSI
LHFKDFVNASSKYEITTIHNLFRKLTHRLFRRNFGYTLRSVNDLYIQKQFPILLDFKTKV
REYYFAEAQIADFSDPAFISKTNNHIMKLTKGLIKDALENIDPATQMMILNCIYFKGSWV
NKFPVEMTHNHNFRLNEREVVKVSMMQTKGNFLAANDQELDCDILQLEYVGGISMLIVVP
HKMSGMKTLEAQLTPRVVERWQKSMTNRTREVLLPKFKLEKNYNLVESLKLMGIRMLFDK
NGNMAGISDQRIAIDLFKHQGTITVNEEGTQATTVTTVGFMPLSTQVRFTVDRPFLFLIY
EHRTSCLLFMGRVANPSRS

The term "HEP2" as used herein refers to human HEP2 protein with SEQ ID NO:10 and to sequences substantially identical thereto. Preferably, said sequence is SEQ ID NO: 10.

In a particular embodiment, the method for the screening, diagnosis and/or monitoring of the invention comprises determining in step a) the protein expression levels of one or more biomarkers comprising or consisting of:
i. PIGR and/or IGHA2; and
ii. optionally, one, two, three, four or five biomarkers selected from APOA, HPT, HEP2, ITIH1 and C5.

Preferably, step a) comprises determining the expression levels of PIGR and/or IGHA2 and one, two or three biomarkers selected from APOA, HPT, HEP2, ITIH1 and C5. Accordingly, in a preferred embodiment, optionally in combination with one or more of the features or embodiments described herein, said one or more biomarkers in step a) is a plurality of biomarkers comprising or consisting of:
a. PIGR and/or IGHA2, and APOA;
b. PIGR and/or IGHA2, and HPT;
c. PIGR and/or IGHA2, and HEP2;
d. PIGR and/or IGHA2, and C5;
e. PIGR and/or IGHA2, and ITIH1;
f. PIGR and/or IGHA2, APOA and HPT;
g. PIGR and/or IGHA2, APOA and HEP2;
h. PIGR and/or IGHA2, APOA and C5;
i. PIGR and/or IGHA2, APOA and ITIH1;
j. PIGR and/or IGHA2, HPT and HEP2;
k. PIGR and/or IGHA2, HPT and C5;
l. PIGR and/or IGHA2, HPT and ITIH1;
m. PIGR and/or IGHA2, HEP2 and C5;
n. PIGR and/or IGHA2, HEP2 and ITIH1;
o. PIGR and/or IGHA2, APOA, HPT and C5;
p. PIGR and/or IGHA2, APOA, HPT and ITIH1;
q. PIGR and/or IGHA2, APOA, HEP2 and C5;
r. PIGR and/or IGHA2, APOA, HEP2 and ITIH1;
s. PIGR and/or IGHA2, HPT, HEP2 and C5;
t. PIGR and/or IGHA2, HPT, HEP2 and ITIH1; and
u. PIGR and/or IGHA2, APOA, HPT and HEP2.

In a particular embodiment, optionally in combination with one or more of the features or embodiments described herein, said one or more biomarkers in step a) is a plurality of biomarkers comprising or consisting of:
- PIGR, APOA and ITIH1; or
- PIGR, APOA and C5; or
- IGHA2, APOA and ITIH1; or
- IGHA2, APOA and C5; or
- PIGR, ITIH1 and C5; or
- IGHA2, ITIH1 and C5.

More preferably, said plurality of biomarkers in a) comprises PIGR and/or IGHA2; and further comprises two biomarkers which are APOA; and at least one of HPT, HEP2, ITIH1 or C5. Accordingly, in another embodiment, optionally in combination with any of the embodiments or features described herein, said plurality of biomarkers comprises or consists of:
- IGHA2, APOA and at least one of HPT, HEP2, ITIH1 or C5; or
- PIGR, APOA and at least one of HPT, HEP2, ITIH1 or C5.

Even more preferably, said plurality of biomarkers in a) comprises or consists of:
- IGHA2, APOA and HPT; or
- PIGR, APOA and HPT.

In a further embodiment, said plurality of biomarkers in a) comprises or consists of PIGR, IGHA2, APOA, and at least one of HPT, HEP2, ITIH1 or C5. Preferably, said plurality of biomarkers in a) comprises or consists of a plurality of biomarkers selected from the group consisting of:
(i) PIGR, IGHA2, APOA, ITIH1 and C5;
(ii) PIGR, IGHA2, APOA, HPT and HEP2;
(iii) PIGR, IGHA2, APOA, HPT and ITIH1;
(iv) PIGR, IGHA2, APOA, HPT and C5;
(v) PIGR, IGHA2, APOA, HEP2 and ITIH1; and
(vi) PIGR, IGHA2, APOA, HEP2 and C5.

Alternatives embodiments not part of the invention comprise determining in step a) the expression levels of one or more biomarkers comprising or consisting of:
i. at least ITIH1; and
ii. optionally, one, two, three, four, five or six biomarkers, selected from APOA, HPT, HEP2, C5, PIGR and IGHA2.

Preferably, said one or more biomarkers in a) is a plurality of biomarkers comprising ITIH1 and one or two biomarkers selected from APOA, HPT, HEP2, C5, PIGR and IGHA2. Accordingly, in a preferred embodiment, optionally in combination with one or more of the features or embodiments described herein, said plurality of biomarkers comprises or consists of:
- ITIH1 and APOA; or
- ITIH1 and PIGR; or
- ITIH1 and IGHA2; or
- ITIH1 and C5; or
- ITIH1 and HPT; or
- ITIH1 and HEP2; or
- ITIH1, APOA and PIGR; or
- ITIH1, APOA and IGHA2; or
- ITIH1, APOA and C5; or
- ITIH1, APOA and HPT; or
- ITIH1, APOA and HEP2; or
- ITIH1, PIGR and IGHA2; or
- ITIH1, PIGR and C5; or
- ITIH1, PIGR and HPT; orITIH1, PIGR and HEP2; or
- ITIH1, IGHA2 and C5; or
- ITIH1, IGHA2 and HPT; or
- ITIH1, IGHA2 and HEP2; or
- ITIH1, C5 and HPT; or
- ITIH1, C5 and HEP2; or
- ITIH1, HPT and HEP2.

More preferably, said plurality of biomarkers in a) comprises ITIH1 and two biomarkers consisting of (i) APOA; and (ii) at least one of PIGR, IGHA2, HPT, HEP2 or C5.

Additional alternatives embodiments not part of the invention comprise determining in step a) the expression levels of one or more biomarkers comprising or consisting of:
i. at least HPT; and
ii. optionally, one, two, three, four, five or six biomarkers, selected from APOA, HEP2, C5, ITIH1, PIGR and IGHA2.

Preferably, said plurality of biomarkers in a) comprises HPT and one or two biomarkers selected from APOA, HEP2, C5, ITIH1, PIGR and IGHA2. Accordingly, in a preferred embodiment, optionally in combination with one or more of the features or embodiments described herein, said plurality of biomarkers comprises or consists of:
- HPT and APOA; or
- HPT and PIGR; or
- HPT and IGHA2; or
- HPT and C5; or
- HPT and ITIH1; or
- HPT and HEP2;
- HPT, APOA and PIGR; or
- HPT, APOA and IGHA2; or
- HPT, APOA and C5; or
- HPT, APOA and ITIH1; or
- HPT, APOA and HEP2; or
- HPT, PIGR and IGHA2; or
- HPT, PIGR and C5; or
- HPT, PIGR and ITIH1; or
- HPT, PIGR and HEP2; or
- HPT, IGHA2 and C5; or
- HPT, IGHA2 and ITIH1; or
- HPT, IGHA2 and HEP2; or
- HPT, C5 and ITIH1; or
- HPT, C5 and HEP2; or
- HPT, ITIH1 and HEP2.

More preferably, said plurality of biomarkers in a) comprises HPT and two biomarkers consisting of (i) APOA; and (ii) at least one of PIGR, HEP2, IGHA2, C5 or ITIH1.

Still additional alternative embodiments not part of the invention comprise determining in step a) the expression levels of one or more biomarkers comprising or consisting of:
i. at least APOA; and
ii. optionally, one, two, three, four, five or six biomarkers, selected from ITIH1, HPT, HEP2, C5, PIGR and IGHA2.

Preferably, said one or more biomarkers in a) is a plurality of biomarkers comprising APOA and one or two biomarkers selected from ITIH1, HPT, HEP2, C5, PIGR and IGHA2. Accordingly, in a preferred embodiment, optionally in combination with one or more of the features or embodiments described herein, said plurality of biomarkers comprises or consists of:
- APOA and ITIH1; or
- APOA and PIGR; or
- APOA and IGHA2; or
- APOA and C5; or
- APOA and HPT; or
- APOA and HEP2; or
- APOA, ITIH1 and PIGR; or
- APOA, ITIH1 and IGHA2; or
- APOA, ITIH1 and C5; or
- APOA, ITIH1 and HPT; or
- APOA, ITIH1 and HEP2; or
- APOA, PIGR and IGHA2; or
- APOA, PIGR and C5; or
- APOA, PIGR and HPT; or
- APOA, PIGR and HEP2; or
- APOA, IGHA2 and C5; or
- APOA, IGHA2 and HPT; or
- APOA, IGHA2 and HEP2; or
- APOA, C5 and HPT; or
- APOA, C5 and HEP2; or
- APOA, HPT and HEP2.

More preferably, said plurality of biomarkers in a) comprises APOA and two biomarkers consisting of (i) HPT; and (ii) at least one of PIGR, IGHA2, HEP2, ITIH1 or C5.

Still further alternatives embodiments not part of the invention comprise determining in step a) the expression levels of one or more biomarkers comprising or consisting of:
i. at least HEP2; and
ii. optionally, one, two, three, four, five or six biomarkers, selected from APOA, HPT, C5, ITIH1, PIGR and IGHA2.

Preferably, said plurality of biomarkers in a) comprises HEP2 and one or two biomarkers selected from APOA, HPT, C5, ITIH1, PIGR and IGHA2. Accordingly, in a preferred embodiment, optionally in combination with one or more of the features or embodiments described herein, said plurality of biomarkers comprises or consists of:
- HEP2 and APOA; or
- HEP2 and PIGR; or
- HEP2 and IGHA2; or
- HEP2 and C5; or
- HEP2 and ITIH1; or
- HEP2 and HPT;
- HEP2, APOA and PIGR; or
- HEP2, APOA and IGHA2; or
- HEP2, APOA and C5; or
- HEP2, APOA and ITIH1; or
- HEP2, APOA and HPT; or
- HEP2, PIGR and IGHA2; or
- HEP2, PIGR and C5; or
- HEP2, PIGR and ITIH1; or
- HEP2, PIGR and HPT; or
- HEP2, IGHA2 and C5; or
- HEP2, IGHA2 and ITIH1; or
- HEP2, IGHA2 and HPT; or
- HEP2, C5 and ITIH1; or
- HEP2, C5 and HPT; or
- HEP2, ITIH1 and HPT.

More preferably, said plurality of biomarkers in a) comprises HEP2 and two biomarkers consisting of (i) APOA; and (ii) at least one of PIGR, HPT, IGHA2, C5 or ITIH1.

The term "sample" or "biological sample", as used herein, refers to biological material isolated from a subject. The biological sample may contain any biological material suitable for detecting the desired biomarker and may comprise cellular and/or non-cellular material from the subject. The sample can be isolated from any suitable biological tissue or fluid such as, for example, blood, blood plasma, serum, cerebral spinal fluid (CSF), urine, amniotic fluid, lymph fluids, external secretions of the respiratory, intestinal, genitourinary tracts, tears, saliva, white blood cells. Preferably, the samples used for the determination of the level(s) of the protein markers in the methods of the invention are samples which can be obtained using minimally invasive procedures. In a preferred embodiment, the samples are blood, plasma or serum samples. Preferably, this biological sample is a blood plasma.

These types of samples are routinely used in the clinical practice and a person skilled in the art will know how to identify the most appropriate means for their obtaining and preservation. Once a sample has been obtained, it may be used fresh, it may be frozen, lyophilized or preserved using appropriate means.

The expression "determining the levels of the marker", as used herein, refers to ascertaining the absolute or relative amount or concentration of the biomarker in the sample. Techniques to assay levels of individual biomarkers from test samples are well known to the skilled technician, and the invention is not limited by the means by which the components are assessed.

Methods for quantifying protein expression are well known in the art. Suitable methods for determining the levels of a given protein include, without limitation, those described herein below. Preferred methods for determining the protein expression levels in the methods of the present invention are immunoassays. Various types of immunoassays are known to one skilled in the art for the quantitation of proteins of interest. These methods are based on the use of affinity reagents, which may be any antibody or other ligand specifically binding to the target protein or to a fragment thereof, wherein said affinity reagent is preferably labeled. For instance, the affinity reagent may be enzymatically labelled, or labeled with a radioactive isotope or with a fluorescent agent.

Affinity reagents may be any antibody or ligand specifically binding to the target protein or to a fragment thereof. Affinity ligands may include proteins, peptides, nucleic acid or peptide aptamers, and other target specific protein scaffolds, like antibody-mimetics. Specific antibodies against the protein markers used in the methods of the invention may be produced for example by immunizing a host with a protein of the present invention or a fragment thereof. Likewise, peptides specific against the protein markers used in the methods of the invention may be produced by screening synthetic peptide libraries.

Western blot or immunoblotting techniques allow comparison of relative abundance of proteins separated by an electrophoretic gel (e.g., native proteins by 3-D structure or denatured proteins by the length of the polypeptide). Immunoblotting techniques use antibodies (or other specific ligands in related techniques) to identify target proteins among a number of unrelated protein species. They involve identification of protein target via antigen-antibody (or protein-ligand) specific reactions. Proteins are typically separated by electrophoresis and transferred onto a sheet of polymeric material (generally nitrocellulose, nylon, or polyvinylidene difluoride). Dot and slot blots are simplified procedures in which protein samples are not separated by electrophoresis but immobilized directly onto a membrane.

Traditionally, quantification of proteins in solution has been carried out by immunoassays on a solid support. Said immunoassay may be for example an enzyme-linked immunosorbent assay (ELISA), a fluorescent immunosorbent assay (FIA), a chemiluminescence immunoassay (CIA), or a radioimmunoassay (RIA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay. Multiplex and any next generation versions of any of the above, are specifically encompassed. In a particular embodiment, said immunoassay is an ELISA assay or any multiplex version thereof.

Other methods that can be used for quantification of proteins in solution are techniques based on mass spectrometry (MS), also called mass spectroscopy. The term "mass spectrometry (MS)-based methods" as used herein refers to mass spectrometry alone or coupled to other detection or separation methods, including gas chromatography combined with mass spectroscopy, liquid chromatography combined with mass spectroscopy, supercritical fluid chromatography combined with mass spectroscopy, ultra-performance liquid chromatography combined with mass spectrometry, MALDI combined with mass spectroscopy, ion spray spectroscopy combined with mass spectroscopy, capillary electrophoresis combined with mass spectrometry, NMR combined with mass spectrometry and IR combined with mass spectrometry. These MS-based methods may include single MS or tandem MS. In another particular embodiment, protein expression levels are determined by liquid chromatography coupled to tandem mass spectrometry (LC-MS/MS) analysis.

Mass spectrometers operate by converting the analyte molecules to a charged (ionized) state, with subsequent analysis of the ions and any fragment ions that are produced during the ionization process, on the basis of their mass to charge ratio (m/z). Several different technologies are available for both ionization and ion analysis, resulting in many different types of mass spectrometers with different combinations of these two processes. On the one hand, examples of ion sources include electrospray ionization source, atmospheric pressure chemical ionization source, atmospheric pressure photo-ionization or matrix-assisted laser desorption ionization (MALDI). On the other hand, mass spectrometers analyzers may be, but are not limited to, quadrupole analyzers, time-of-flight (TOF) analyzers, ion trap analyzers, orbitrap analyzers or hybrid analyzers, such as hybrid quadrupole time-of-flight (QTOF) analyzers, hybrid quadrupole-orbitrap analyzers, hybrid ion trap-orbitrap analyzers, hybrid triple quadrupole linear ion trap analyzers or trihybrid quadrupole-ion trap-orbitrap analyzers. In a preferred embodiment, the levels of the protein markers are determined by using a hybrid quadrupole-orbitrap analyzer.

Internal standards may be used in the MS analysis as it enables to correct for any losses or inefficiencies in the sample preparation process or for alterations in ionization efficiency, for instance those due to ion suppression. Stable or isobaric isotope versions of the analyte are ideal internal standards as they have almost identical chemical properties but are easily distinguished during MS. In a preferred embodiment, optionally in combination with one or more of the embodiments described above or below, the determination of the levels of the protein marker is conducted by an MS-based method using isotope/isobaric-labeled versions of the protein marker as internal standards. In addition, or alternatively, the extraction solvent may be spiked with compounds not detected in unspiked biological samples.

As shown in the Examples (see Tables 5 and 6), IGHG1 and IGHG2 are also independent predictors of subclinical atherosclerosis. In particular, these have been shown to be decreased in the presence of the disease. Accordingly, the method of the invention may further comprise:
a) determining in said biological sample the protein expression levels of IGHG1 and/or IGHG2,
b) comparing the levels of the biomarkers with a reference value;
c) wherein when the levels in the subject's sample of IGHG1 and/or IGHG2 are decreased with respect to the corresponding reference value is indicative of subclinical atherosclerosis.

In addition, the IGHA2 levels may be expressed relative to the levels of any of immunoglobulin heavy constant alpha 1 (IGHA1), immunoglobulin heavy constant gamma 1 (IGHG1), immunoglobulin heavy constant gamma 2 (IGHG2) or a combination thereof. Preferably, IGHA2 levels are expressed relative to the levels of IGHA1 or the average levels of IGHG1 and IGHG2.

The canonical sequence of human immunoglobulin heavy constant alpha 1 (IGHA1) is referred as SEQ ID NO:6 (UniProtKB Accession Number P01876-1; this is version 2 of the sequence of February 1, 1991):

ASPTSPKVFPLSLCSTQPDGNVVIACLVQGFFPQEPLSVTWSESGQGVTARNFPPSQDAS
GDLYTTSSQLTLPATQCLAGKSVTCHVKHYTNPSQDVTVPCPVPSTPPTPSPSTPPTPSP
SCCHPRLSLHRPALEDLLLGSEANLTCTLTGLRDASGVTFTWTPSSGKSAVQGPPERDLC
GCYSVSSVLPGCAEPWNHGKTFTCTAAYPESKTPLTATLSKSGNTFRPEVHLLPPPSEEL
ALNELVTLTCLARGFSPKDVLVRWLQGSQELPREKYLTWASRQEPSQGTTTFAVTSILRV
AAEDWKKGDTFSCMVGHEALPLAFTQKTIDRLAGKPTHVNVSVVMAEVDGTCY

The term "IGHA1" as used herein refers to human IGHA1 protein with SEQ ID NO:6 and to sequences substantially identical thereto. Preferably, said sequence is SEQ ID NO:6.

The canonical sequence of human immunoglobulin heavy constant gamma 1 (IGHG1) is referred as SEQ ID NO:7 (UniProtKB Accession Number P01857-1; this is version 1 of the sequence of July 21, 1986):

The term "IGHG1" as used herein refers to human IGHG1 protein with SEQ ID NO:7 and to sequences substantially identical thereto. Preferably, said sequence is SEQ ID NO:7.

The canonical sequence of human immunoglobulin heavy constant gamma 2 (IGHG2) is referred as SEQ ID NO:8 (UniProtKB Accession Number P01859-1; this is version 2 of the sequence of December 16, 2008):

ASTKGPSVFPLAPCSRSTSESTAALGCLVKDYFPEPVTVSWNSGALTSGVHTFPAVLQSS
GLYSLSSVVTVPSSNFGTQTYTCNVDHKPSNTKVDKTVERKCCVECPPCPAPPVAGPSVF
LFPPKPKDTLMISRTPEVTCVVVDVSHEDPEVQFNWYVDGVEVHNAKTKPREEQFNSTFR
VVSVLTVVHQDWLNGKEYKCKVSNKGLPAPIEKTISKTKGQPREPQVYTLPPSREEMTKN
QVSLTCLVKGFYPSDISVEWESNGQPENNYKTTPPMLDSDGSFFLYSKLTVDKSRWQQGN
VFSCSVMHEALHNHYTQKSLSLSPGK

The term "IGHG2" as used herein refers to human IGHG2 protein with SEQ ID NO:8 and to sequences substantially identical thereto. Preferably, said sequence is SEQ ID NO:8.

The inventors also found that IGHG1, IGKC and IGLC2 decreased with plaque thickness in PESA-V1, whereas IGHA1, IGHG3, IGHG4 and IGHM were unaffected, remaining these changes detectable in PESA-V2 (Fig. 3A). Hence, the presented data supports a role for the humoral immune response in the early phases of atherosclerosis (Tsiantoulas D et al., 2014) and also suggest the existence of Ig class switching. A role in IgA class switching has been proposed for BAFF (B-cell activating factor) and APRIL (proliferation-inducing ligand), two factors implicated in B-cell development in autoimmune diseases (Kaneko T et al., 2014), with evidence suggesting a role for BAFF in directing IgA1 switching and APRIL in directing IgA2 switching (Litinskiy MB et al., 2002). It is therefore conceivable that an IgA class switch is triggered by the differential activation of specific B-cell subsets proposed to take place during atherosclerosis (Tsiantoulas D et al., 2014).

In **step (b)**, the screening and/or diagnostic method of the invention comprises comparing the level(s) of the protein marker(s) with a reference value.

The term "reference value", as used herein, relates to a predetermined criteria used as a reference for evaluating the values or data obtained from the samples collected from a subject. This "reference value" may also be referred as "cut-off value" or "threshold value".

The reference value or reference level can be an absolute value, a relative value, a value that has an upper or a lower limit, a range of values, an average value, a median value, a mean value, or a value as compared to a particular control or baseline value. A reference value can be based on an individual sample value or can be based on a large number of samples, such as from population of subjects of the chronological age matched group, or based on a pool of samples including or excluding the sample to be tested.

The expression levels of the protein markers can be used for calculating a combined score to be compared with a reference value. The combined score is a value obtained according to a given mathematical formula or algorithm wherein the expression values of each of the protein markers used in the methods of the invention are variables of said mathematical algorithm. In a particular embodiment, when calculating the combined score, this is proportional to the expression levels of one or more of PIGR, IGHA2, APOA, HPT, HEP2, ITIH1 and C5, wherein the higher the score, the higher the likelihood of subclinical atherosclerosis.

Furthermore, **step (c)** the method under the first aspect of the invention comprises classifying the subject as having or presenting a high likelihood of having subclinical atherosclerosis according to the comparison with the reference value, wherein when the levels in the subject's sample of PIGR, IGHA2, APOA, HPT, HEP2, ITIH1 and/or C5 are increased in the subject sample with respect to the corresponding reference value is indicative of subclinical atherosclerosis.

The biomarkers levels are considered "increased" when its value is higher than a reference value. Preferably, the biomarker levels (or the combined score) are considered to be higher than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than the reference value.

Likewise, the biomarker levels are considered "decreased" when its value is lower than the reference value. Preferably, the biomarker levels (or the combined score) are considered to be lower than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than the reference value.

Alternatively or in addition, subjects having more than about 1.1,1.2, 1.3, 1.4, 1.5, 2, 3, 4, 5, 6, 7, 8, 9, 10, 15 or 20 fold levels deviation with respect to the reference value as described herein.

The method of the invention, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical significance measures obtained by statistical tests; illustrative, non-limiting examples of said statistical significance measures include determining confidence intervals, determining the p-value, etc. Preferred confidence intervals are at least 90%, at least 95%, at least 97%, at least 98%, at least 99%. The p-values are, preferably less than 0.1, less than 0.05, less than 0.01, less than 0.005 or less than 0.0001. The teachings of the present invention preferably allow correctly classifying at least 60%, at least 70%, at least 80%, or at least 90% of the subjects of a determining group or population analyzed.

It is further noted that the accuracy of the method of the invention can be further increased by additionally considering biochemical parameters and/or clinical characteristics of the patients (e.g. age, sex, tobacco and/or other cardiovascular risk factors), such as included in traditional cardiovascular risk scores.

In a particular embodiment, optionally in combination with one or more of the embodiments or features as described herein, the method according to the first aspect of the invention further comprises conducting a traditional cardiovascular risk score. Typically, risk scores comprise biochemical and physiological determinations, as well as other clinical and/or lifestyle characteristics of the subject. Preferably said traditional cardiovascular risk score is selected from the group consisting of 10-y FHS or 30-y FHS (Kannel et al., 1979; Splansky et al., 2007), ICHS (Fernández-Alvira et al., 2017) and the BEWAT (Fernández-Alvira et al., 2017) scores, more preferably wherein said cardiovascular risk score is 10-y FHS.

Said risk score may be combined with the protein measures of PIGR, IGHA2, APOA, HPT, HEP2, ITIH1 and/or C5 (including any combination thereof as described herein above) using appropriate mathematical combinations, preferably using multivariate logistic regression models. In a particular embodiment, optionally in combination with any of the embodiments or features as described herein, said method is preferably a method for the screening and/or diagnosis of subclinical atherosclerosis and the biomarker measurement is performed in a sample isolated from a subject classified as low risk when conducting a traditional cardiovascular risk score. For instance, coronary heart disease (CHD) risk at 10 years in percent can be calculated with the help of the Framingham Risk Score (FHS-10y). Individuals with low risk have 10% or less CHD risk at 10 years, with intermediate risk 10-20%, and with high risk 20% or more. However it should be remembered that these categorizations are arbitrary.

Moreover, the method under the first aspect of the invention can comprise additionally conducting *in vivo* imaging studies. This may include but are not limited to 2/3-dimensional vascular ultrasound analysis of presence and morphology of atherosclerosis plaques and computed tomography analysis of coronary artery calcification. Preferably, additional *in vivo* imaging studies are conducted in those subjects selected for presenting in step c) biomarker levels indicative of atherosclerosis.

The methods of the present invention or any of the steps thereof might be implemented by a computer. Therefore, a further aspect of the invention refers to a computer implemented method, wherein the method is any of the methods part of the invention disclosed herein or any combination thereof.

It is noted that any computer program capable of implementing any of the methods of the present invention or used to implement any of these methods or any combination thereof, also forms part of the present invention. This computer program is typically directly loadable into the internal memory of a digital computer, comprising software code portions for performing the steps of comparing the levels of the protein markers as described in the invention, from the one or more biological samples of a subject, with a reference value and determining the presence or likelihood of having subclinical atherosclerosis, when said product is run on a computer.

It is also noted that any device or apparatus comprising means for carrying out the steps of any of the methods of the present invention or any combination thereof, or carrying a computer program capable of, or for implementing any of the methods of the present invention or any combination thereof, is included as forming part of the present specification.

The methods of the invention may also comprise the storing of the method results in a data carrier, preferably wherein said data carrier is a computer readable medium. The present invention further relates to a computer-readable storage medium having stored thereon a computer program of the invention or the results of any of the methods of the invention. As used herein, "a computer readable medium" can be any apparatus that may include, store, communicate, propagate, or transport the results of the determination of the method of the invention. The medium can be an electronic, magnetic, optical, electromagnetic, infrared, or semiconductor system (or apparatus or device) or a propagation medium.

In a second aspect not part of the invention, provided herein is a method for treating a subject having subclinical atherosclerosis, wherein said method comprises:
a. identifying a subject having subclinical atherosclerosis by the method for screening diagnosis and/or monitoring as defined herein;
b. administering to said patient a therapeutically effective amount of a suitable treatment for preventing/reducing atherosclerotic plaques.

These may include treatments for reducing the levels of HDL cholesterol or total triglyceride levels; and/or increasing HDL cholesterol levels in plasma. Statins are currently among the most therapeutically effective drugs available for reducing the level of LDL in blood. Statins are also known to raise HDL cholesterol levels and decrease total triglyceride levels. It is believed that statins disrupt the biosynthesis of cholesterol and other sterols in the liver by competitively inhibiting the 3-hydroxy-3-methyl-glutaryl-coenzyme A reductase enzyme ("HMG-CoA reductase"). HMG-CoA reductase catalyzes the conversion of HMG-CoA to mevalonate, which is the rate determining step in the biosynthesis of cholesterol. Consequently, its inhibition leads to a reduction in the rate of formation of cholesterol in the liver. Statins are well known in the art, and these include for instance pravastatin, simvastatin, lovastatin, fluvastatin, atorvastatin and rosuvastatin.

### Kit and use of a kit in the methods of the invention

In a further aspect, here described but not part of the invention is a kit for determining the levels of one or more of the protein markers as described herein in a biological sample isolated from a subject. The kit may also contain instructions indicating how the materials within the kit may be used.

The term "kit" or "testing kit" denotes combinations of reagents and adjuvants required for an analysis. Although a test kit consists in most cases of several units, one-piece analysis elements are also available, which must likewise be regarded as testing kits.

In a particular embodiment, the kit comprises reagents adequate for the determination of the protein expression levels of one or more of the markers selected from the group consisting of PIGR, IGHA2, APOA, HPT, HEP2, ITIH1 and C5.

These reagents may be useful for determining the expression levels of the target protein marker(s) using any suitable method as described herein above. For instance, the determination of the levels of said protein marker(s) may be carried out by using an affinity reagent, (e.g., by an immunoassay) as described under the first aspect of the invention.

In a particular embodiment, optionally in combination with one or more of the features or embodiments as described herein, said kit comprises:
a. a reagent for determining the protein expression levels of (e.g., an affinity reagent for) at least one of PIGR or IGHA2 (preferably PIGR and IGHA2);
b. optionally, a reagent for determining the protein expression levels of (e.g., an affinity reagent for) one, two, three, four or five of APOA, HPT, HEP2, ITIH1 and C5;
c. optionally, further comprising instructions for the use of said reagents in determining said protein expression levels in a biological sample isolated from a subject.

In a preferred embodiment, optionally in combination with one or more of the features or embodiments as described herein, said kit comprises:
a. a reagent for determining the protein expression levels of (e.g., an affinity reagent for) at least one of PIGR or IGHA2 (preferably, a reagent for determining the protein expression levels of PIGR and a reagent for determining the protein expression levels of IGHA2);
b. a reagent for determining the protein expression levels of (e.g., an affinity reagent for) APOA; and
c. a reagent for determining the protein expression levels of (e.g., an affinity reagent for) at least one of HPT, HEP2, ITIH1 or C5;
d. optionally, further comprising instructions for the use of said reagents in determining said protein expression levels in a biological sample isolated from a subject.

In an alternative embodiment, optionally in combination with one or more of the features or embodiments as described herein, said kit comprises:
a. a reagent for determining the protein expression levels of (e.g., an affinity reagent for) ITIH1 ;
b. optionally, a reagent for determining the protein expression levels of (e.g., an affinity reagent for) one, two, three, four, five or six biomarkers selected from APOA, HPT, HEP2, C5, PIGR and IGHA2;
c. optionally, further comprising instructions for the use of said reagents in determining said protein expression levels in a biological sample isolated from a subject.

In a preferred embodiment, optionally in combination with one or more of the features or embodiments as described herein, said kit comprises:
a. a reagent for determining the protein expression levels of (e.g., an affinity reagent for) ITIH1 ;
b. a reagent for determining the protein expression levels of (e.g., an affinity reagent for) APOA; and
c. a reagent for determining the protein expression levels of (e.g., an affinity reagent for) at least one of PIGR, HPT, HEP2, IGHA2 or C5;
d. optionally, further comprising instructions for the use of said reagents in determining said protein expression levels in a biological sample isolated from a subject.

In a further alternative embodiment, optionally in combination with one or more of the features or embodiments as described herein, said kit comprises:
a. a reagent for determining the protein expression levels of (e.g., an affinity reagent for) HPT;
b. optionally, a reagent for determining the protein expression levels of (e.g., an affinity reagent for) one, two, three, four, five or six biomarkers selected from APOA, ITIH1, HEP2, C5, PIGR and IGHA2;
c. optionally, further comprising instructions for the use of said reagents in determining said protein expression levels in a biological sample isolated from a subject.

In a preferred embodiment, optionally in combination with one or more of the features or embodiments as described herein, said kit comprises:
a. a reagent for determining the protein expression levels of (e.g., an affinity reagent for) HPT;
b. a reagent for determining the protein expression levels of (e.g., an affinity reagent for) APOA; and
c. a reagent for determining the protein expression levels of (e.g., an affinity reagent for) at least one of PIGR, ITIH1, HEP2, IGHA2 or C5;
d. optionally, further comprising instructions for the use of said reagents in determining said protein expression levels in a biological sample isolated from a subject.

In still additional alternative embodiments, optionally in combination with one or more of the features or embodiments as described herein, said kit comprises:
a. a reagent for determining the protein expression levels of (e.g., an affinity reagent for) APOA;
b. optionally, a reagent for determining the protein expression levels of (e.g., an affinity reagent for) one, two, three, four, five or six biomarkers selected from ITIH1, HPT, HEP2, C5, PIGR and IGHA2;
c. optionally, further comprising instructions for the use of said reagents in determining said protein expression levels in a biological sample isolated from a subject.

In a preferred embodiment, optionally in combination with one or more of the features or embodiments as described herein, said kit comprises:
a. a reagent for determining the protein expression levels of (e.g., an affinity reagent for) APOA;
b. a reagent for determining the protein expression levels of (e.g., an affinity reagent for) HPT; and
c. a reagent for determining the protein expression levels of (e.g., an affinity reagent for) at least one of PIGR, ITIH1, HEP2, IGHA2 or C5;
d. optionally, further comprising instructions for the use of said reagents in determining said protein expression levels in a biological sample isolated from a subject.

In still further alternative embodiments, optionally in combination with one or more of the features or embodiments as described herein, said kit comprises:
a. a reagent for determining the protein expression levels of (e.g., an affinity reagent for) HEP2;
b. optionally, a reagent for determining the protein expression levels of (e.g., an affinity reagent for) one, two, three, four, five or six biomarkers selected from ITIH1, HPT, APOA, C5, PIGR and IGHA2;
c. optionally, further comprising instructions for the use of said reagents in determining said protein expression levels in a biological sample isolated from a subject.

In a preferred embodiment, optionally in combination with one or more of the features or embodiments as described herein, said kit comprises:
a. a reagent for determining the protein expression levels of (e.g., an affinity reagent for) HEP2;
b. a reagent for determining the protein expression levels of (e.g., an affinity reagent for) APOA; and
c. a reagent for determining the protein expression levels of (e.g., an affinity reagent for) at least one of PIGR, ITIH1, HPT, IGHA2 or C5;
d. optionally, further comprising instructions for the use of said reagents in determining said protein expression levels in a biological sample isolated from a subject.

Preferred combinations of biomarkers to be measured and corresponding reagents (e.g. affinity reagents) are as described for the first aspect of the invention herein above.

Moreover, any of these embodiments, may further comprise one or more of the following:
- a reagent for determining the protein expression levels of (e.g., an affinity reagent for) IGHA1;
- a reagent for determining the protein expression levels of (e.g., an affinity reagent for) IGHG1; or
- a reagent for determining the protein expression levels of (e.g., an affinity reagent for) IGHG2.

The term "an affinity reagent for" as used herein refers to an affinity reagent capable of specifically binding to the recited target protein. The various affinity reagents may be labelled with the same or different tags. Preferably, these will be labelled with different tags for multiplex analysis.

In a particular embodiment, optionally in combination with one or more of the embodiments or features described herein, said affinity reagent is an antibody, preferably a monoclonal antibody. The affinity reagent may bind to any linear or conformational region (e.g. epitope) specific for the target protein.

Illustrative/non-liming examples of commercially available antibodies specifically binding to the proteins as described herein, include the following:
- Rat monoclonal PIGR (7C1, ABCAM) Catalog number ab170321,
- Mouse Anti-Human IgA2 (A9604D2, Southern biotech) Catalog number 9140-01,
- Complement C5 Monoclonal Antibody (12F3, thermo scientific) Catalog number HYB 029-02-02,
- Rabbit monoclonal Anti-Lipoprotein (a) antibody [EPR6474, ABCAM) Catalog number ab125014,
- ITIH1 Monoclonal Antibody (40B10 , thermo scientific) Catalog number LF-MA014,
- IGHA1: mouse antihuman iga1 (souther biotech,9130-01)
- IGHG1: Mouse IgG1, Kappa Monoclonal (ABCAM, ab81032)
- IGHG2: Mouse antihuman-igG2 monoclonal (SIGMA, 15635)

Possible immunoassays and affinity reagents have been described herein. In a particular embodiment, said immunoassay is an ELISA assay.

In some embodiments, the kit includes the above-mentioned affinity reagents for the target protein(s) and one or more ancillary reagents. A primary affinity reagent (e.g. a primary antibody) may be used for capturing purposes and a secondary affinity reagent (e.g. secondary antibody) for detection purposes.

The primary or capturing affinity reagent is typically a monoclonal antibody specific for the target protein. Secondary or detection affinity reagents can be monoclonal or polyclonal antibodies. Also, these can be derived from any mammalian organism, including mice, rats, hamsters, goats, camels, chicken, rabbit, and others.

The detection affinity reagent may be labeled. A tag or label can be any composition which is detectable. Any analytical means known in the art can be used for determining or detecting the secondary affinity reagent. These means include the use of spectroscopy, chemistry, photochemistry, biochemistry, immunochemistry, or optics. The label can be, for example, an enzyme (e.g., horseradish peroxidase (HRP), alkaline phosphatase, beta-galactosidase, and others commonly used in an ELISA), a radiolabel (e.g., ³H, ¹²⁵I, ³⁵S, ¹⁴C, or ³²P), a chemiluminescent compound (e.g. luciferin, and 2,3-dihydrophthalazinediones, luminol, etc.), a fluorescent dye (e.g., fluorescein isothiocyanate, Texas red, rhodamine, etc.), or any other dye known in the art. The label may be coupled directly or indirectly (e.g., via binding pairs such as biotin and avidin) to the detection affinity reagent according to methods well known in the art. As indicated above, a wide variety of labels may be used. The choice of label may depend on sensitivity required, ease of conjugation with the compound, stability requirements, available instrumentation, or disposal provisions.

Preferably, said kit comprises a solid support or surface which is coated with the primary affinity reagent. The solid support can include any support known in the art on which a protein of this disclosure can be immobilized. In some embodiments, said solid supports are microtiter well plates, slides (e.g., glass slides), chips (e.g., protein chips, biosensor chips, such as Biacore chips), microfluidic cartridges, cuvettes, beads (e.g., magnetic beads, xMAP^{®} beads) or resins. Ancillary reagents typically used in an immunoassay, such as a solid support immunoassay, can include, e.g., an immobilization buffer, an immobilization reagent, a dilution buffer, a detection reagent, a blocking buffer, a washing buffer, a detection buffer, a stop solution, a system rinse buffer, and a system cleaning solution which are well known by a person skilled in the art.

In addition, the determination of the levels of said protein marker(s) may be carried out by a mass spectrometry (MS)-based method, and said kit may comprise said marker unlabelled and/or said marker stably labelled for detection by a mass spectrometry (MS)-based method, preferably wherein the marker is labelled with a tag which comprises one or more stable isotope. Isotopic atoms which may be incorporated into the tag are heavy atoms for example ¹³C, ¹⁵N, ¹⁸O and/or ³⁴S, which can be distinguished by MS.

In still an additional aspect, the invention relates to the use of a kit of the preceding aspect in a method for the screening, diagnosis and/or monitoring of subclinical atherosclerosis as described herein.

Preferred features and embodiments are as defined above for other aspects of the invention.

It is contemplated that any features described herein can optionally be combined with any of the embodiments of any method, kit, use of a kit, or computer program of the invention; and any embodiment discussed in this specification can be implemented with respect to any of these. It will be understood that particular embodiments described herein are shown by way of illustration and not as limitations of the invention. The principal features of this invention can be employed in various embodiments without departing from the scope of the invention.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

The following examples serve to illustrate the present invention and should not be construed as limiting the scope thereof.

### EXAMPLES

### EXAMPLE 1.- Identifying protein biomarkers of subclinical atherosclerosis by Quantitative proteomics - PESA cohort visit 1 (PESA-v1)

### 1.1 Material and Methods

PESA subcohort: the proteomics analysis are performed on plasma from 476 individuals. The samples are paired according to sex, age and clinical history. All PESA individuals have been subjected to extensive imaging and biochemical analysis as described previously (Fernandez-Friera et al., 2015; Fernandez-Ortiz et al., 2013). According to the extent of subclinical atherosclerosis, individuals were classified into four groups using the PESA score (Fernandez-Friera et al., 2015): no disease, focalized, intermediate and generalized disease.

Quantitative proteomics by multiplexed isobaric labeling and quantitative analysis of the data are performed following detailed protocols set up in our laboratory and already described (Burillo et al., 2016; Garcia-Marques et al., 2016; Gomez-Serrano et al., 2016; Latorre-Pellicer et al., 2016; Martin-Alonso et al., 2015). Identification, quantification and statistical and systems biology analysis are done using models developed in our laboratory and fully described before (Bonzon-Kulichenko et al., 2015; Garcia-Marques et al., 2016; Jorge et al., 2009; Navarro et al., 2014; Navarro and Vazquez, 2009; Trevisan-Herraz et al., 2018, in the press). Mass spectrometry analysis are performed in a hybrid quadrupole-orbitrap machine (HF Orbitrap, ThermoFisher).

The epidemiological analysis are performed as follows. The selection of a panel of potential biomarkers in the PESA subcohort is performed by analyzing quantitative protein values, determined as described above, using the SPSS package by constructing multivariate linear or logistic regression models to predict disease (in terms of plaque number, thickness, number of affected territories or PESA score), after adjustment by all other known risk factors (e.g., cholesterol, blood pressure, gender, age, LDL and HDL levels). ROC analysis is then performed to measure the increase in predictive power of the biomarker panel over existing methods to predict cardiovascular risk (for instance FHS 10 year, BEWAT or ICHS scores).

### 1. 2.- Analysis of the association of PIGR, APOA and C5 levels in plasma with the extent of atherosclerotic lesions in asymptomatic individuals

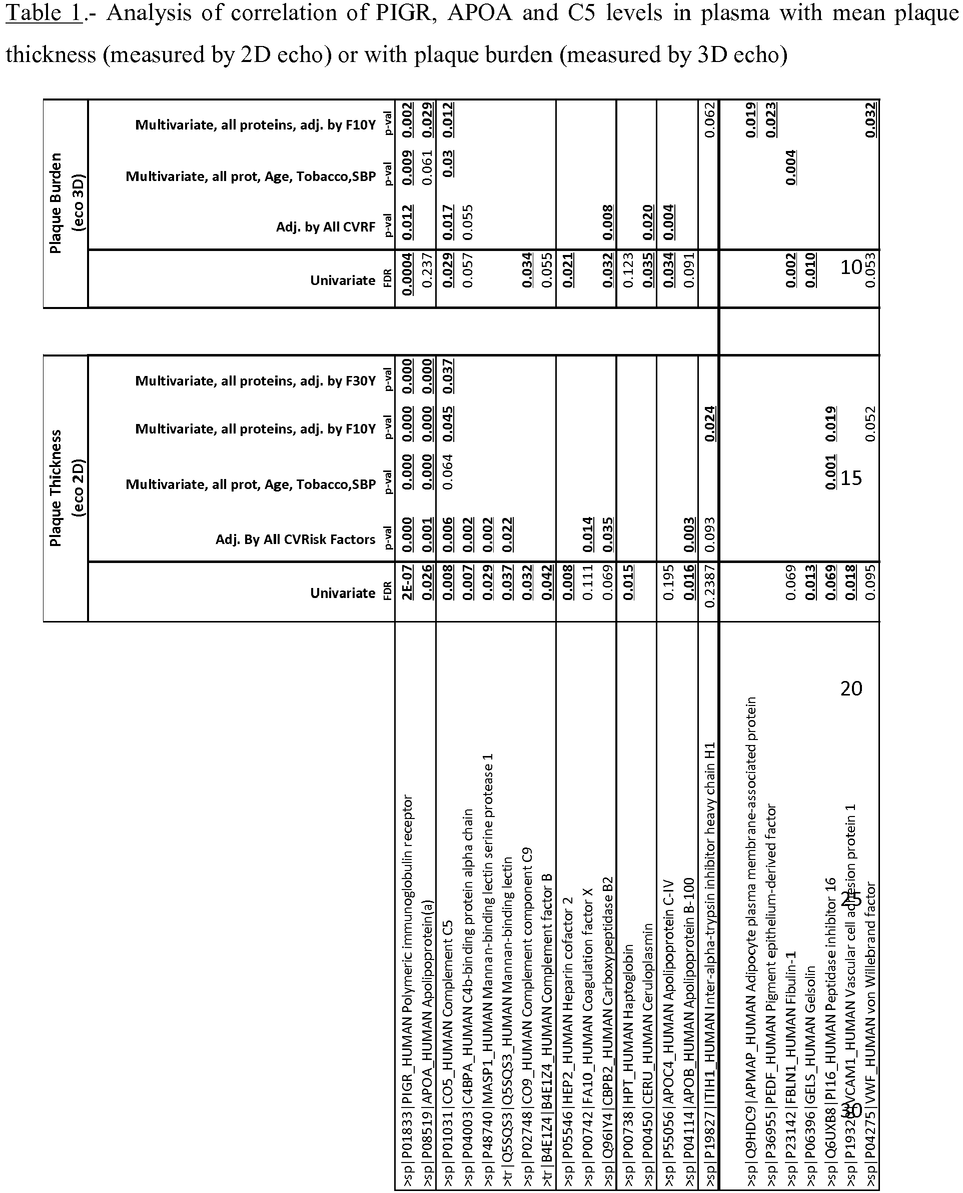

The table shows how the plasma levels of PIGR, APOA and C5 have a strong correlation with either of two independent measurements of the extent of atherosclerotic lesions even after adjustment by conventional risk factors. Besides, the three proteins remain significantly associated to plaque thickness when the three are taken together in a multivariate model including risk factors. This result means that the three proteins are associated with plaque independently from each other.

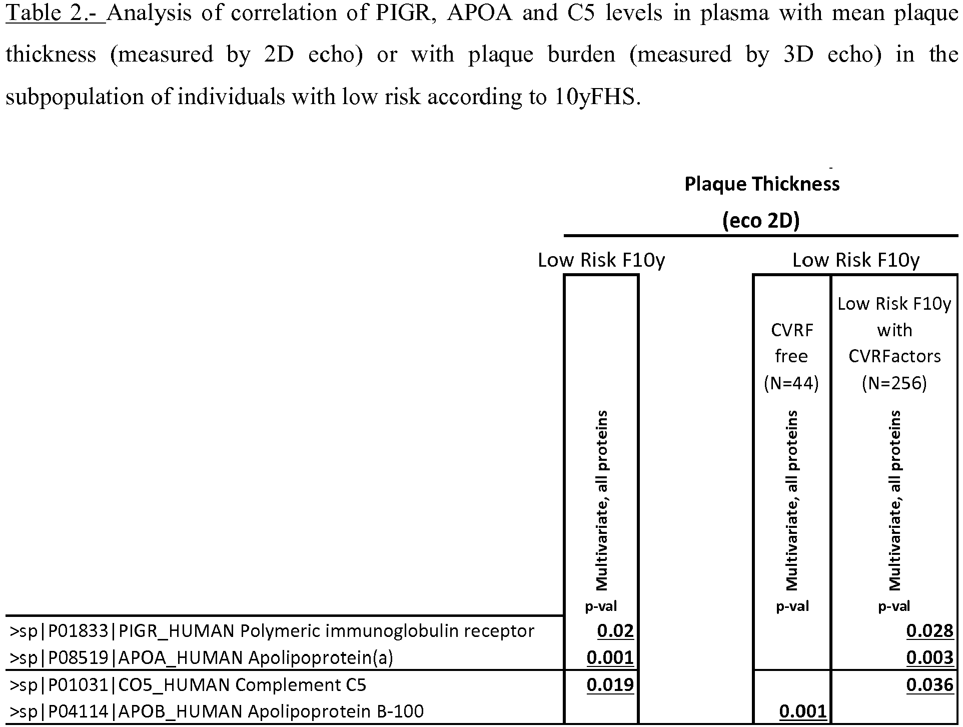

The table shows how the levels of PIGR, APOA and C5, taken together in a multivariate model, maintain their correlation with the extent of atherosclerotic lesions even in the subpopulation of individuals with low-risk according to FHS10y. These results indicate that these proteins may independently and effectively trace the presence of atherosclerosis lesions in cases where there is no evidence of cardiovascular risk according to classical risk factors.

### 1. 3.- Analysis of the protein levels of PIGR, APOA, ITIH1, C5 and IGHA2 in media and intima layers from human aorta without or with atherosclerotic lesions

The data shown in Fig.1 indicates that all the protein selected in plasma as potential markers of subclinical atherosclerosis, namely PIGR, APOA, ITIH1, C5 and IGHA2, accumulate in the media layer and mainly in the intima layer (plaque), and that the accumulation is higher as the plaque evolves from the fatty streak-type to the fibrolipid lesion-type. Such accumulation may explain (or be a consequence of) the increased plasma levels in individuals with subclinical atherosclerosis.

### 1. 4.- Analysis of the performance of PIGR, APOA and ITIH1 (C5) plasma levels as independent predictors of generalized subclinical atherosclerosis

**Table 3.- Logistic regression analysis of PIGR, APOA, ITIH1 and C5 levels in plasma as predictors of generalized disease in the PESA population (No disease vs Generalized in PESA score).**

| PESA score (Normal vs Generalized) **logistic regression** | | | | | | | | | | | | | | | | | | | | | | | | |
|---|---|---|---|---|---|---|---|---|---|---|---|---|---|---|---|---|---|---|---|---|---|---|---|---|
| | **Adj. by All CVRF** | | | | **Multivariate, all proteins, adj. by Age, Tobacco** | | | | **Multivariate, all proteins, adj. by F10Y** | | | | **Multivariate, all proteins, adj. by F30Y** | | | | **Multivariate, all proteins, adj. by BEWAT** | | | | **Multivariate, all proteins, adj. by ICHS** | | | |
| | **p-val** | **OR** | **95% CI** | | **p-val** | **OR** | **95% CI** | | | | | | **p-val** | **OR** | **95% CI** | | **p-val** | **OR** | **95% CI** | | **p-val** | **OR** | **95% CI** | |
| PIGR | **0** | 1.44 | 1.19 | 1.75 | **0** | 1.48 | 1.22 | 1.8 | **0** | 1.441 | | 1.2 1.73 | **0** | 1.42 | 1.19 | 1.7 | **0** | 1.53 | 1.28 | 1.83 | **0** | 1.54 | 1.29 | 1.84 |
| APOA | **0.019** | 1.12 | 1.02 | 1.22 | **0** | 1.15 | 1.05 | 1.27 | **0** | 1.123 | 1.03 | 1.23 | **0.02** | 1.11 | 1.02 | 1.22 | **0.01** | 1.12 | 1.03 | 1.21 | **0** | 1.11 | 1.02 | 1.21 |
| C5 | **0.03** | 1.37 | 1.03 | 1.83 | | | | | | | | | | | | | | | | | | | | |
| ITIH1 | **0.024** | 1.6 | 1.07 | 2.41 | **0** | 1.84 | 1.22 | 2.76 | **0** | 1.927 | 1.32 | 2.82 | **0** | 1.8 | 1.23 | 2.64 | **0** | 1.84 | 1.27 | 2.67 | **0** | 1.84 | 1.27 | 2.66 |

These data show that each one of PIGR, APOA, ITIH1 and C5 levels in plasma is an independent predictor of generalized subclinical atherosclerosis even when known risk factors are included in the logistic model. In addition, PIGR, APOA and ITIH1, together, are also independent predictors in the presence of known risk scores such as 10y-FHS, 30y-FHS, BEWAT or ICHS. Without willing to be bound by theory, in the regression analysis we observed that C5 and ITIH1 have strong covariance, so that they appear to be dependent. The observed covariance would suggest that C5 and ITIH1 can be interchanged (if ITIH1 is not considered in the multivariate model, then C5 behaves as independent predictor together with PIGR and APOA and any of the known risk factors)

**Table 4.- Logistic regression analysis of PIGR, APOA and ITIH1 levels in plasma as predictors of generalized disease in the subpopulation of individuals with low risk according to 10yFHS or 30yFHS.**

| | PESA score (Normal vs Generalized) | | | | | | | |
|---|---|---|---|---|---|---|---|---|
| | **F10Y Low Risk Population** | | | | **F30Y Low Risk Population** | | | |
| | **Multivariate (All)** | | | | **Multivariate (All)** | | | |
| | **p-val** | **OR** | **95% CI** | | **p-val** | **OR** | **95% CI** | |
| >sp\|P01833\|PIGR_HUMAN Polymeric immunoglobulin receptor | **0.009** | 1.33 | 1.08 | 1.651 | **0.025** | 1.46 | 1.05 | 2.032 |
| >sp\|P08519\|APOA_HUMAN Apolipoprotein(a) | **0.003** | 1.17 | 1.05 | 1.293 | | | | |
| >tr\|B4E1Z4\|B4E1Z4_HUMAN Complement factor B | **0.028** | 1.59 | 1.05 | 2.393 | | | | |
| >sp\|P19827\|ITIH1_HUMAN Inter-alpha-trypsin inhibitor heavy cha | **0.001** | 2.38 | 1.42 | 3.991 | **0.03** | 2.53 | 1.1 | 5.85 |

Table 4 shows that PIGR, APOA and ITIH1 maintain their ability of being independent predictors of generalized disease even in the subpopulation of individuals with low-risk according to 10yFHS. The table also shows that PIGR and ITIH1 maintain their ability of being independent predictors of generalized disease even in the subpopulation of individuals with low-risk according to 30yFHS. These results indicate that these proteins may effectively predict the presence of atherosclerosis lesions in cases where there is no evidence of cardiovascular risk according to classical risk factors.

### 1. 5.- Analysis of the association with atherosclerosis and of the power to predict generalized atherosclerosis of IGHA2 levels in plasma

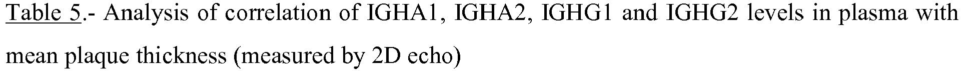

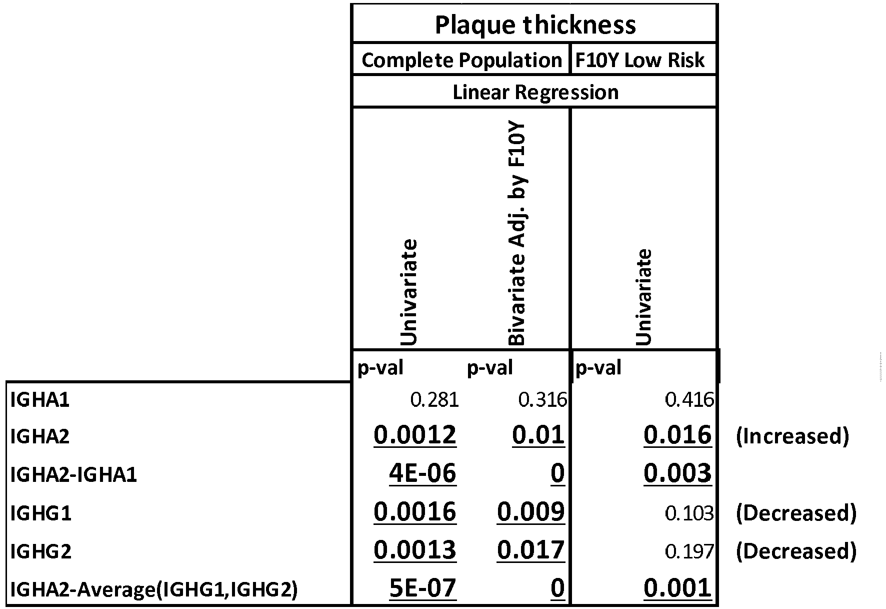

The table shows that plaque thickness correlates with increased abundance of IGHA2 (but not IGHA1) and with decreased abundance of IGHG1 or IGHG2, independently of 10yFHS. The correlation with IGHA2 is maintained even in the subpopulation of individuals with low-risk according to FHS10y. The ratio IGHA2/IGHG1 have an even stronger correlation than IGHA2 alone. The ratio IGHA2/average(IGHG1,IGHG2) is even better. These results suggest that plaque thickness correlates with Ig isotype switching.

**Table 6.- Logistic regression analysis of IGHA1, IGHA2, IGHG1 and IGHG2 levels in plasma as predictors of generalized disease in the PESA population (No disease vs Generalized in PESA score).**

| | | **PESA Score** | | | | | | | | | | | |
|---|---|---|---|---|---|---|---|---|---|---|---|---|---|
| | | **All Population (Extremes)** | | | | | | | | **F10Y Low Risk Population** | | | |
| | | **Logistic Regresion** | | | | | | | | | | | |
| | **t-test** | **Univariate** | | | | **Bivariate (Adj. F10Y)** | | | | **Univariate** | | | |
| | **p-val** | **p-val** | **OR** | **95%CI** | | **p-val** | **OR** | **95%CI** | | **p-val** | **OR** | **95%CI** | |
| **IGHA1** | 0.897601 | 0.887 | 0.993 | 0.895 | 1.101 | 0.852 | 1.011 | 0.902 | 1.132 | 0.627 | 0.968 | 0.847 | 1.105 |
| **IGHA2** | **5E-05** | **0** | 1.27 | 1.127 | 1.431 | **0** | 1.272 | 1.115 | 1.451 | **0.002** | 1.28 | 1.098 | 1.492 |
| **IGHA2-IGHA1** | **1E-05** | **0** | 1.327 | 1.166 | 1.51 | **0.001** | 1.261 | 1.102 | 1.444 | **0.001** | 1.308 | 1.111 | 1.539 |
| **IGHG1** | **0.01** | **0.013** | 0.809 | 0.684 | 0.956 | **0.04** | 0.83 | 0.695 | 0.992 | 0.091 | 0.84 | 0.687 | 1.028 |
| **IGHG2** | **0.031** | **0.034** | 0.876 | 0.775 | 0.99 | 0.261 | 0.927 | 0.813 | 1.058 | 0.552 | 0.956 | 0.824 | 1.109 |
| **IGHA2-Average(IGHG1,IGHG2)** | **8E-08** | **0** | 1.333 | 1.191 | 1.492 | **0** | 1.308 | 1.157 | 1.48 | **0** | 1.306 | 1.131 | 1.508 |

The table shows that the IGHA2 level in plasma (corrected or not by other Igs), but not those of IGHA1, is an independent predictor of generalized subclinical atherosclerosis even when the risk score 10yFHS is included in the logistic model. This effect is maintained in the population with low risk.

### 1. 6.- Analysis of the performance of biomarker panels as independent predictors of generalized subclinical atherosclerosis

Table 7.- Receiver-operating curve (ROC) analysis of several risk scores and risk factors as predictors of generalized disease in the PESA population (No disease vs Generalized disease).

| | **ROC Curves** | | | |
|---|---|---|---|---|
| | **AUC** | **95% CI** | | **p value** |
| **FHS10y** | 0.713 | 0.658 | 0.768 | **0** |
| **BEWAT** | 0.613 | 0.552 | 0.674 | **0** |
| **ICHS** | 0.611 | 0.55 | 0.672 | **0.001** |
| **Tobacco** | 0.689 | 0.631 | 0.748 | **0** |
| **Age** | 0.713 | 0.658 | 0.769 | **0** |

The table shows how the three risk scores, as well as individual risk factors, are able to produce a statistically significant discrimination between no diseased individuals and those with generalized disease.

**Table 8.- Receiver-operating curve (ROC) analysis of PIGR, APOA, ITIH1, C5 and IGHA2 as predictors of generalized disease in the PESA population (No disease vs Generalized disease).**

| | **ROC Curves** | | | |
|---|---|---|---|---|
| | **AUC** | **95% CI** | | **p value** |
| **PIGR** | 0.66 | 0.6 | 0.719 | **0** |
| **APOA** | 0.573 | 0.51 | 0.636 | **0.024** |
| **ITIH1** | 0.579 | 0.517 | 0.641 | **0.014** |
| **C5** | 0.61 | 0.549 | 0.671 | **0.001** |
| **IGHA2** | 0.634 | 0.573 | 0.694 | **0** |

The table shows that each one of the individual proteins are able to produce a statistically significant discrimination between no diseased individuals and those with generalized disease.

**Table 9.- Receiver-operating curve (ROC) analysis of several panels containing three proteins from the group PIGR, APOA, ITIH1, C5 and IGHA2, as predictors of generalized disease in the PESA population (No disease vs Generalized disease).**

| | **ROC Curves** | | | |
|---|---|---|---|---|
| | **AUC** | **95% CI** | | **p value** |
| **PIGR+APOA+C5** | 0.707 | 0.651 | 0.764 | **0** |
| **IGHA2+APOA+ITIH1** | 0.686 | 0.627 | 0.745 | **0** |

When compared with the data in Table 7, this table shows that it is possible to use combinations of three proteins that are able to discriminate between no diseased individuals and those with generalized disease with a performance similar or better than that of widely-used risk scores.

**Table 10.- Receiver-operating curve (ROC) analysis of 10yFHS alone or in combination with different protein panels, as predictors of generalized disease in the PESA population (No disease vs Generalized disease).**

| | **ROC Curves** | | |
|---|---|---|---|
| | **AUC** | **95% Cl** | |
| **FHS10y** | 0.713 | 0.658 | 0.768 |
| **FHS10y+PIGR+APOA+C5** | 0.767 | 0.715 | 0.819 |
| **FHS10y+PIGR+APOA+ITIH1** | 0.78 | 0.729 | 0.83 |
| **FHS10y+IGHA2+APOA+C5** | 0.784 | 0.734 | 0.834 |
| **FHS10y+IGHA2+APOA+ITIH1** | 0.779 | 0.728 | 0.83 |
| **FHS10y+PIGR+IGHA2+APOA+C5+ITIH1** | 0.812 | 0.764 | 0.86 |

The table shows that the addition to 10yFHS of panels containing several proteins from the group PIGR, APOA, ITIH1, C5 and IGHA2 produces a discriminatory performance significantly higher than that of 10yFHS alone (as deduced from the 95% confidence intervals of AUC values). These results indicate that the protein panel may serve to significantly improve the prediction of subclinical atherosclerosis in comparison with current standards.

**Table 11.- Receiver-operating curve (ROC) analysis of ICHS alone or in combination with different protein panels, as predictors of generalized disease in the PESA population (No disease vs Generalized disease).**

| | **ROC Curves** | | |
|---|---|---|---|
| | **AUC** | **95% Cl** | |
| **ICHS** | 0.611 | 0.55 | 0.672 |
| **ICHS+PIGR+APOA+C5** | 0.708 | 0.651 | 0.764 |
| **ICHS+PIGR+APOA+ITIH1** | 0.718 | 0.662 | 0.774 |
| **ICHS+IGHA2+APOA+C5** | 0.721 | 0.664 | 0.778 |
| **ICHS+IGHA2+APOA+ITIH1** | 0.699 | 0.64 | 0.758 |
| **ICHS+PIGR+IGHA2+APOA+C5+ITIH1** | 0.763 | 0.711 | 0.816 |
| **ICHS+IGHA2** | 0.664 | 0.605 | 0.724 |

The table shows that the addition to ICHS of panels containing several proteins from the group PIGR, APOA, ITIH1, C5 and IGHA2 produces a discriminatory performance significantly higher than that of ICHS alone (as deduced from the 95% confidence intervals of AUC values). These results indicate that the protein panel may serve to significantly improve the prediction of subclinical atherosclerosis in comparison with current standards.

**Table 12.- Receiver-operating curve (ROC) analysis of BEWAT score alone or in combination with different protein panels, as predictors of generalized disease in the PESA population (No disease vs Generalized disease).**

| | **ROC Curves** | | |
|---|---|---|---|
| | **AUC** | **95% Cl** | |
| **BEWAT** | 0.613 | 0.552 | 0.674 |
| **BEWAT+PIGR+APOA+C5** | 0.711 | 0.654 | 0.768 |
| **BEWAT+PIGR+APOA+ITIH1** | 0.725 | 0.669 | 0.781 |
| **BEWAT+IGHA2+APOA+C5** | 0.727 | 0.67 | 0.784 |
| **BEWAT+IGHA2+APOA+ITIH1** | 0.712 | 0.654 | 0.77 |
| **BEWAT+PIGR+IGHA2+APOA+C5+ITIH1** | 0.767 | 0.715 | 0.819 |
| **BEWAT+IGHA2** | 0.665 | 0.606 | 0.724 |

The table shows that the addition to BEWAT score of panels containing several proteins from the group PIGR, APOA, ITIH1, C5 and IGHA2 produces a discriminatory performance significantly higher than that of BEWAT alone (as deduced from the 95% confidence intervals of AUC values). These results indicate that the protein panel may serve to significantly improve the prediction of subclinical atherosclerosis in comparison with current standards.

### EXAMPLE 2.- Confirming stability of the protein abundance changes over time - PESA cohort visit 2(PESA-v2) & validation the obtained biomarker panel by immunoturbidimetric analysis

### 2.1 Material & Methods

**Plasma samples.** Plasma samples were collected from PESA study cohort (Fernandez-Friera L et al., 2015) and AWHS cohort. (Casasnovas JA et al., 2012) For the discovery phase, a nested case-control study within the prospective PESA cohort was designed (PESA-V1). To account for potential cofounding and to increase efficiency, the study were restricted to men, and controls were matched for CV risk factors. Firstly, 222 case subjects were selected among participants with extensive subclinical atherosclerosis, defined as subjects with 3 or more vascular territories affected. Control subjects were selected in a 1:1 fashion, among participants with non-extensive subclinical atherosclerosis, defined as subjects with none or 1 vascular territory affected. The control subjects were matched with case subjects based on age (caliper: 3 years), hypertension, dyslipemia and diabetes. Plasma samples from the same individuals were also collected three years later (PESA-V2), except two cases and their matched controls that did not renew their consent for the 'omics' analysis. The validation set was designed within the AWHS cohort following the same methodology. A nested case-control, restricted to men and matched by age, hypertension, dyslipemia and diabetes was performed. Two hundred and twenty case subjects were selected among participants with extensive subclinical atherosclerosis, defined as subjects with 3 or more vascular territories affected. The same number of control subjects were selected from control participants with non-extensive subclinical atherosclerosis, defined as subjects with 2 or less vascular territories affected.

**Assessment of CV risk factors and subclinical atherosclerosis.** In the PESA study, CV risk factors were prospectively collected through questionnaires (smoking, family history) or objective quantification (hypertension, diabetes, dyslipidemia) as previously described. (Fernandez-Friera L et al., 2015) Two-dimensional vascular ultrasound and noncontrast cardiac computed tomography were performed in all participants as previously described. (Fernandez-Friera L et al., 2015; Fernandez-Friera L et al., 2017) The presence of atherosclerotic plaques by ultrasound was assessed by cross-sectional sweep of carotids, infrarenal abdominal aorta, and iliofemoral arteries. The identification of plaques in both carotid and femoral arteries and the clinical characteristics in the AWHS study were determined as described (Laclaustra M et al., 2016).

**Tissue samples.** Aortic tissue samples from the media and intima layers were obtained from dead organ donors with the authorization of the French Biomedicine Agency (PFS 09-007, BRIF BB-0033-00029; AoS BBMRI-EU /infrastructure BIOBANQUE ; No. Access : 2, Last : April 15, 2014. [BIORESOURCE]). Some of these samples were macroscopically normal (AoS) and devoid of early atheromatous lesions and were used as healthy controls (9 individuals) for comparison with those with samples with fatty streaks (FS) (7 individuals for media layer and 6 in the case of the intima layer) or fibrolipidic (FL) plaques (11 individuals in the case of media layer and 12 in the case of intima layer). Tissue (100 mg of FL, FS or AoS) was homogenized at low temperature, and lysates were resuspended in buffer (50 mM iodoacetamide (Sigma), 1% SDS, 1mM EDTA, 100 mM Tris-HCL, pH 8.5) for protein extraction or in TRIZOL for mRNA isolation. Proteins were extracted by vortexing samples 4 times with 15min intervals on ice. Proteins in the supernatant were measured by the BCA method and stored at -80°C until proteomics analysis.

**Proteomic analysis.** Plasma and tissue protein samples were subjected to filter-aided digestion with trypsin according to manufacter's instructions (Nanosep Centrifugal Devices with Omega Membrane-10K, PALL), and the resulting peptides to multiplexed isobaric labeling with TMT reagents (Thermo Fisher Scintific).(Baldan-Martin M et al., 2018; Bagwan N et al., 2018) Two of the 10 channels were reserved for internal reference standard samples created by pooling the samples. Plasma peptides were separated into five fractions using a high pH reversed-phase peptide fractionation kit (Thermo Fisher Scientific), and tissue peptides were separated into eight fractions using OASIS MCX cartridges. Each plasma fraction was analyzed by LC-MS/MS using an Ultimate 3000 HPLC system (Thermo Fisher Scientific) coupled via a nanoelectrospray ion source (Thermo Fisher Scientific, Bremen, Germany) to a Q Exactive HF mass spectrometer (Thermo Fisher Scientific, Bremen, Germany).(Bagwan N et al., 2018) Tissue fractions were analyzed on an Easy nLC 1000 liquid chromatography system coupled to an Orbitrap Fusion mass spectrometer (Themo Scientific). (Baldan-Martin M et al., 2018)

**Immunoturbidimetric analysis**. Plasma levels of IGHA2, HPT and APOA were measured by immunoturbidimetric assays (LK088.OPT, NK058.OPT and LK098.OPT, respectively) using the Binding Site Optilite analyzer. The analysis was performed in a blinded manner by a technician from The Binding Site company.

**Statistical analysis.** Peptide identification, quantification and statistical and systems biology analysis were performed using the models developed in our laboratory (Navarro P et al., 2014; Navarro P and Vazquez J, 2008; Jorge I et al., 2009; Garcia-Marques F et al., 2016; Bonzon-Kulichenko E et al., 2016; Martinez-Bartolome S et al., 2008). Quantitative information was extracted from the MS/MS spectra of TMT-labeled peptides. Peptide quantification was analyzed using the WSPP model, which uses raw quantifications as input data and computes the protein log2-fold changes for each individual with respect to the mean value of the two reference internal standard samples. In this model, protein log2-ratios are expressed as standardized variables in units of standard deviation according to their estimated variances (Zq values).

Adjusted linear and logistic regression models were used to analyze the association of proteins with the presence and extent of atherosclerosis using SPSS software (IBM, Armonk, New York). A multivariate analysis was conducted to explore candidate variables with a clinical association: glucose, LDL, HDL, systolic and diastolic blood pressure, smoking, and age. Associations were expressed as odds ratios (ORs) with 95% confidence intervals (CI). Differences were considered significant at a p-value<0.05 was considered statistically significant. The C-statistic or area under the receiver operating characteristic (ROC) curve (AUC) were calculated for each model as a measure of the discriminatory power of each biomarker protein panel. Comparison of the C-index for models including and not including information provided by the biomarker panels was performed according to the method of DeLong(DeLong ER et al., 1988). To evaluate whether the biomarker panels helped to correctly classify individuals according to presence and extent of subclinical atherosclerosis, we calculated the categorical net reclassification index using four discrete risk categories (NRI^{0.25}), and the category-free net reclassification index (NRI^{>0}), also called continuous net reclassification index(Kerr KF et al., 2014, Pepe MS et al., 2015; Leening MJ et al., 2014), which does not depend on the arbitrary choice of categories, but deems any change in predicted risk in the correct direction as appropriate. In the low-risk subpopulation we evaluated whether the biomarker panels helped to classify individuals according to the presence of subclinical atherosclerosis using two risk categories (risk/no risk) (NRI^{0.5}).

### Study design

The current report examines a subcohort of the PESA study.(Fernandez-Ortiz A et al., 2013) The first cohort, used for the discovery phase (PESA-V1), included 444 men with a mean age of 48.5 years, organized as 222 pairs of individuals with no clinical history of atherosclerosis. Complete clinical characteristics of the PESA-V1 cohort are detailed in Table 13. Plasma samples from PESA-V1 were analyzed by deep quantitative proteomics.

**Table 13.- Characteristics of the PESA-V1 Population used for the Discovery Phase**

| | **No atherosclerosis (n=222)** | **Atherosclerosis (n=222)** |
|---|---|---|
| Age (years) | 48 ±4 | 49 ± 4 |
| SBP (mmHg) | 120 ± 12 | 124 ± 12 |
| DBP (mmHg) | 75 ± 9 | 77 ± 9 |
| Fasting glucose (mg/dl) | 94 ± 11 | 96 ± 15 |
| Total cholesterol (mg/dl) | 201 ± 33 | 210 ± 36 |
| LDL-C (mg/dl) | 136 ± 30 | 143 ± 33 |
| HDL-C (mg/dl) | 44 ± 10 | 43 ± 10 |
| Triglycerides (mg/dl) | 106 ± 58 | 121 ± 65 |
| Current smoking | 25% | 41% |

The stability of the detected protein abundance changes over time was confirmed by repeating the proteomics analysis with plasma collected from the same individuals three years later (PESA-V2) (except for the four individuals that did not renew their consent for the 'omics' analysis); the clinical characteristics of the PESA-V2 individuals are depicted in Supplementary Table 1. The proteomics quantified a mean of 1093 proteins per individual, and 454 proteins could be quantified in more than 80% of the individuals. The analysis of the 884 plasma samples required a total of 560 LC-MS runs.

**Table 14.- Characteristics of the PESA-V2 Population**

| | **No atherosclerosis (n=220)** | **Atherosclerosis (n=220)** |
|---|---|---|
| Age (years) | 48 ± 4 | 49 ± 4 |
| SBP (mmHg) | 120 ± 12 | 124 ± 12 |
| DBP (mmHg) | 75 ± 10 | 77 ± 9 |
| Fasting glucose (mg/dl) | 92 ± 10 | 97 ± 21 |
| Total cholesterol (mg/dl) | 203 ± 33 | 209 ± 37 |
| LDL-C (mg/dl) | 137 ± 29 | 141 ± 34 |
| HDL-C (mg/dl) | 46 ± 11 | 44 ± 10 |
| Triglycerides (mg/dl) | 102 ± 50 | 121 ± 65 |
| Current smoking | 21% | 34% |

| | | |
|---|---|---|
| Values are mean ± SD or % | | |

For the validation phase, we examined a third cohort of 350 men from the Aragon Workers Health Study. This cohort had a mean age of 50.3 years and was organized as 175 pairs of individuals with no history of clinical CV disease and with clinical characteristics similar to those of the PESA subcohort (Table 15).(Casasnovas JA et al., 2012; Laclaustra M et al., 2016) Plasma samples from the AWHS cohort were analyzed by turbidimetry using commercially available antibodies against selected proteins.

**Table 15.- Characteristics of the AWHS Population used for the Validation Phase**

| | **No atherosclerosis (n=175)** | **Atherosclerosis (n=175)** |
|---|---|---|
| Age (years) | 49.6 ± 4.1 | 51.0 ± 3.6 |
| SBP (mmHg) | 122.1 ± 12.0 | 125.8 ± 13.9 |
| DBP (mmHg) | 81.4 ± 8.7 | 82.5 ± 8.8 |
| Fasting glucose (mg/dl) | 98.2 ± 17.9 | 98.3 ± 17.2 |
| Total cholesterol (mg/dl) | 215.4 ± 35.5 | 221.6 ± 37.3 |
| HDL-C (mg/dl) | 54.0 ± 11.1 | 50.4 ± 10.4 |
| Current smoking | 17% | 44% |

| | | |
|---|---|---|
| Values are mean ± SD or % | | |

### 2.1 Association of plasma proteins with traditional risk factors

A correlation network was constructed including the proteins whose levels were significantly correlated with traditional continuous CV risk factors in both the PESA-V1 and the PESA-V2 sample sets; the CV risk factors considered were glucose, age, systolic and diastolic blood pressure, cholesterol, LDL and HDL (Fig.2). Most correlations were of apolipoproteins and other proteins implicated in lipid transport, which showed a positive correlation with LDL, HDL and cholesterol. Apart from these expected associations, there were no clear association patterns with other factors, suggesting that in the subclinical phase traditional CV risk factors have a limited impact on the plasma proteome.

### 2.2 Association of plasma proteins with plague thickness and calcium score

Association between plasma protein levels and plaque thickness was assessed by univariate linear regression analysis corrected by multiple hypothesis testing. This analysis revealed a list of plasma proteins showing significant correlation (FDR < 5%) with plaque thickness in PESA-V1 (Table 16). Most proteins maintained the correlation with plaque thickness with a FDR below 15% at 3-year follow-up (PESA-V2) (Table 16). Most of these proteins were related to the humoral immune response, including polymeric immunoglobulin receptor (PIGR) and IGHA2, which were increased, and immunoglobulin heavy constant gamma 2 (IGHG2), immunoglobulin kappa constant (IGKC), immunoglobulin heavy constant gamma 1 (IGHG1) and immunoglobulin lambda constant 2 (IGLC2), which were decreased. HPT, C4-binding protein (C4BP), heparin cofactor 2 (HEP2), APOA, complement component 9 (CO9) and gelsolin (GELS) also maintained their correlation with plaque thickness three years later (Table 16). We also noticed that vascular adhesion protein 1 (VCAM1) and von Willebrand factor (VWF), two proteins considered to be biomarkers of endothelial dysfunction, decreased their levels with plaque thickness in PESA-V1 (Table 16). Although this tendency was contrary to the expected, this observation was not reproduced in PESA-V2.

**Table 16.- Proteins showing significant correlation with plaque thickness. Linear regression analysis was performed to measure correlation between plaque thickness and plasma protein levels in PESA-V1 and PESA-V2. Statistical significance of the Pearson's correlation coefficient was expressed in terms of the false discovery rate (FDR). Listed are the proteins that have a significant correlation in V1 (FDR ≤ 5%) and in V2 (FDR ≤ 15%).**

| **Proteins** | **PESA-V1** | | **PESA-V2** | |
|---|---|---|---|---|
| **Increased** | **p-val** | **FDR** | **p-val** | **FDR** |
| Polymeric immunoglobulin receptor (PIGR) | <0.001 | <0.001 | 0.002 | 0.044 |
| C4b-binding protein alpha chain (C4BPA) | <0.001 | 0.009 | 0.022 | 0.120 |
| Heparin cofactor 2 (HEP2) | <0.001 | 0.012 | <0.001 | 0.027 |
| Haptoglobin (HPT) | <0.001 | 0.021 | <0.001 | <0.001 |
| Apolipoprotein(a) (APOA) | <0.001 | 0.037 | 0.021 | 0.115 |
| Ig alpha-2 chain C region (IGHA2) | 0.001 | 0.041 | 0.002 | 0.044 |
| Complement component C9 (CO9) | 0.002 | 0.045 | 0.004 | 0.061 |
| | | | | |

| **Decreased** | **p-val** | **FDR** | **p-val** | **FDR** |
|---|---|---|---|---|
| Gelsolin (GELS) | <0.001 | 0.018 | <0.001 | <0.001 |
| Ig kappa chain C region (IGKC) | <0.001 | 0.019 | 0.010 | 0.089 |
| Ig gamma-1 chain C region (IGHG1) | 0.002 | 0.041 | 0.010 | 0.085 |

In general the correlations with plaque thickness in PESA-V1 were reproduced in PESA-V2 (Fig. 3A). Interestingly, the proteins related to the humoral immune response were among those that better maintained in V2 the correlation observed in V1 (Fig. 3A). The opposite association with plaque thickness of IGHA2 and immunoglobulin heavy constant delta (IGHD) in comparison with that of IGLC2, IGKC, IGHG1 and IGHG2, together with the lack of correlation for the related isotypes immunoglobulin heavy constant alpha 1 (IGHA1) and immunoglobulin heavy constant gamma 3 and 4 (IGHG3 and IGHG34, respectively), suggested that plaque formation is associated with an isotype switch.

Among the proteins that correlated with plaque thickness, we judged that IGHA2, PIGR, HEP2, HPT, APOA, GELS, IGKC, IGLC2 and IGHG1 were the proteins that better maintained the correlation in both PESA-V1 and V2 after adjustment by individual risk factors using multivariate linear regression analysis (Table 17).

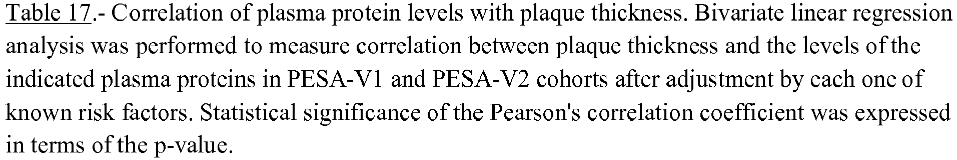

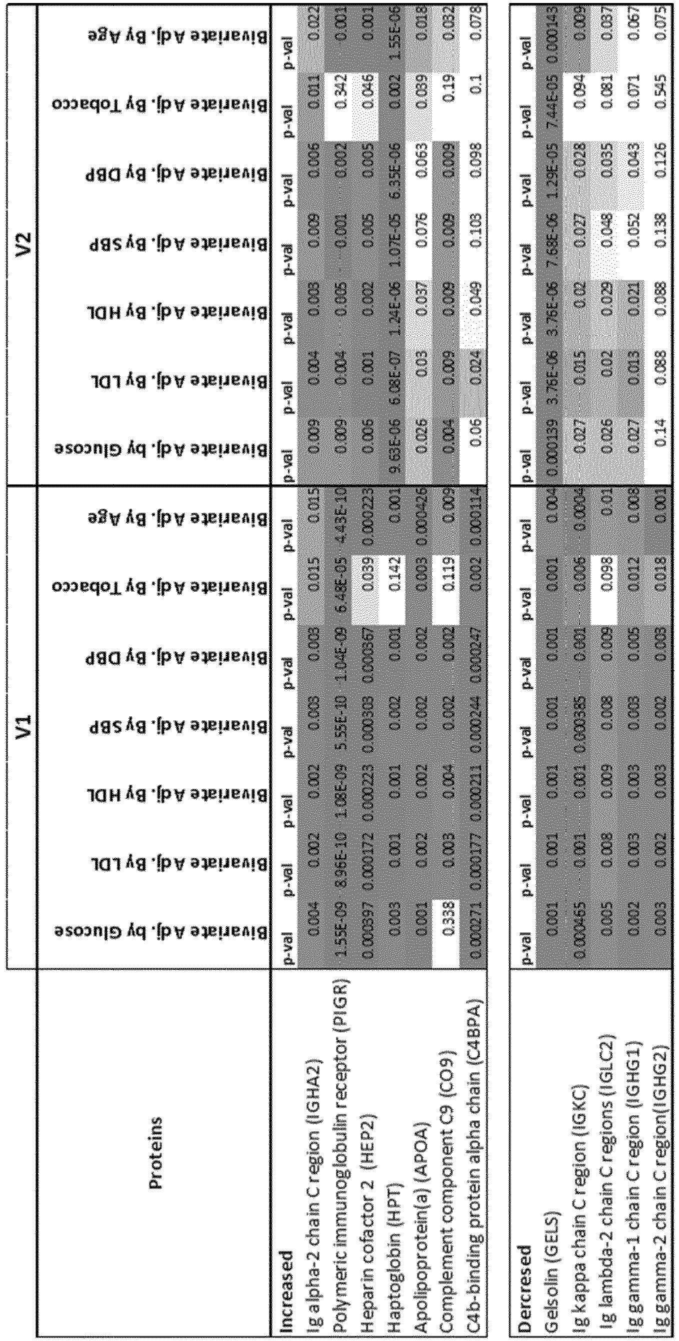

Since these results were obtained after the analysis of a large number of proteins by proteomics, we studied whether some of them could be reproduced using other approaches. Turbidimetry analysis of the plasma levels of two representative proteins (IGHA2 and APOA) for which there were commercially available antibodies confirmed their independent correlation with plaque thickness (Table 18).

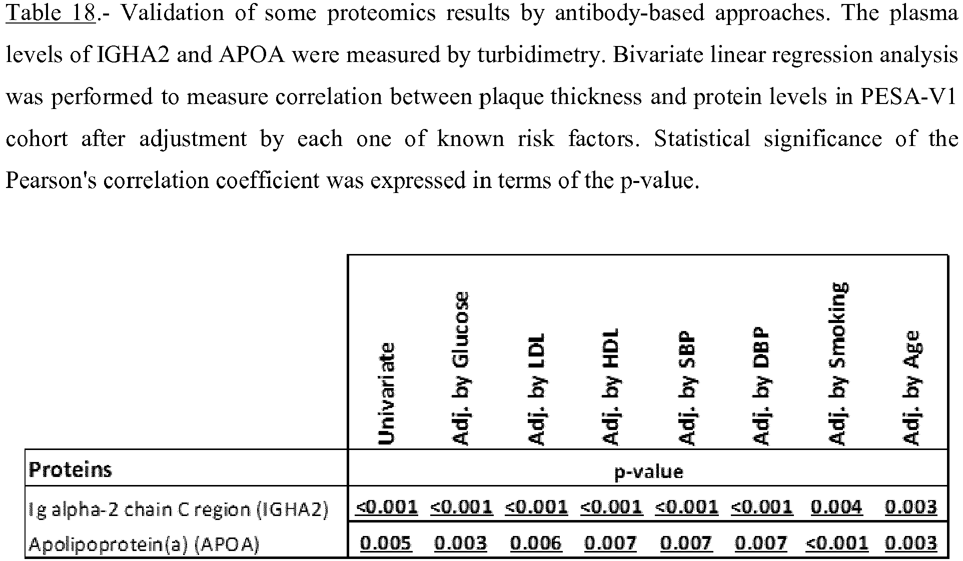

A similar analysis was performed with the proteins that correlated with coronary artery calcium score (CACS). Some of the proteins associated to plaque thickness, including HPT, APOA and GELS, also showed a significant correlation with CACS in PESA-V1 which was maintained in V2 (Table 19 and Fig. 3B).

**Table 19.- Proteins showing significant correlation with coronary artery calcium score. Linear regression analysis was performed to measure correlation between CACS and plasma protein levels in PESA-V1 and PESA-V2. Statistical significance of the Pearson's correlation coefficient was expressed in terms of the false discovery rate (FDR). Listed are the proteins that have a significant correlation in V1 (FDR ≤ 5%) and in V2 (FDR ≤ 15%).**

| **Proteins** | **PESA-V1** | | **PESA-V2** | |
|---|---|---|---|---|
| **Increased** | **p-val** | **FDR** | **p-val** | **FDR** |
| Haptoglobin | <0.001 | 0.041 | <0.001 | 0.045 |

| **Dercreased** | **p-val** | **FDR** | **p-val** | **FDR** |
|---|---|---|---|---|
| Gelsolin (GELS) | 0.001 | 0.040 | <0.001 | 0.013 |
| CD5 antigen-like (CD5L) | <0.001 | 0.022 | 0.004 | 0.150 |

However, among the proteins related to humoral immune response, only IGKC maintained its correlation with CACS (Table 19 and Fig. 3B). Complement factor B (CFB), immunoglobulin heavy constant mu (IGHM) and CD5 antigen-like (CD5L) were other proteins that correlated with CACS but not with plaque thickness. Among the proteins correlating with CACS, HPT, APOA, GELS, CD5L and IGKC were the ones that better maintained the correlation in both PESA-V1 and V2 after adjustment by risk factors (Table 20).

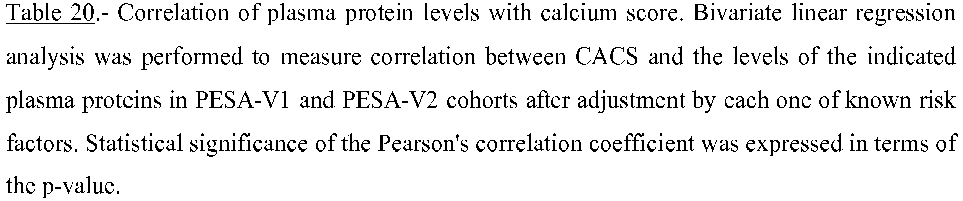

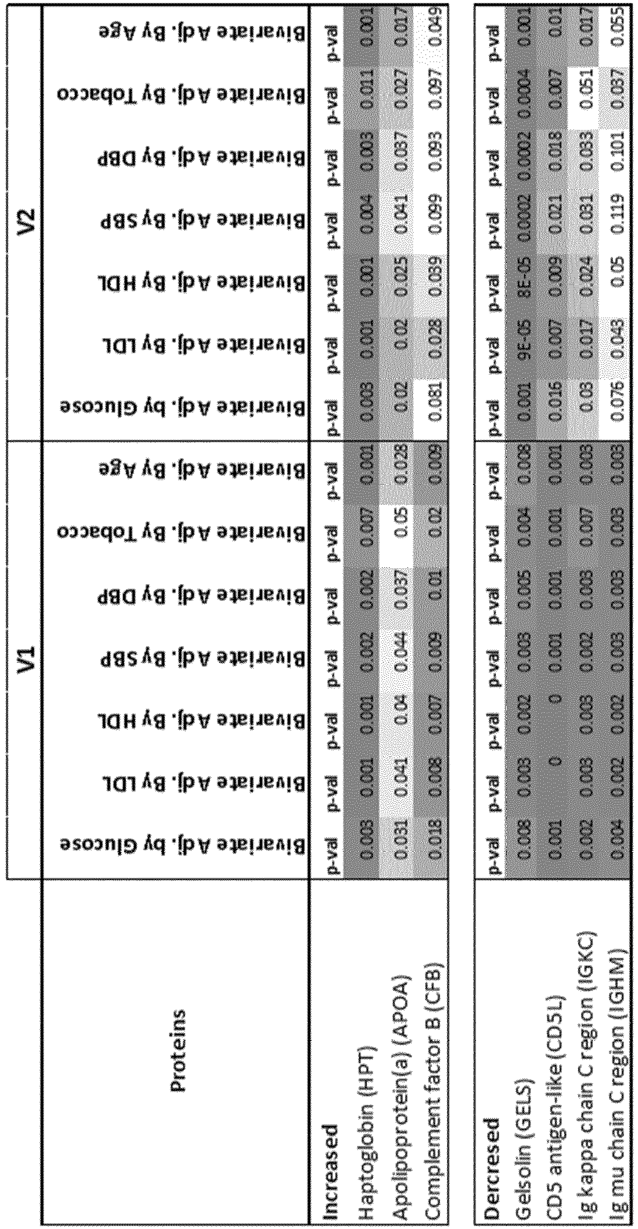

We also analyzed the behavior of representative acute-phase proteins (including C-reactive protein (CRP), serum amyloid A-1 (SAA1) and alpha-1-acid glycoprotein 1 (ORM1)) and of other proteins that have been previously described to associate with subclinical atherosclerosis (Table 21). While CRP correlated with plaque thickness in PESA-V2, none of the acute phase proteins maintained a stable correlation in the two PESA visits. Among proteins previously proposed to associate with subclinical atherosclerosis,(Saarikoski LA et al., 2010; Bhosale SD et al., 2018) cadherin-13 (CDH13) showed significant correlation with plaque thickness in PESA-V1, but the result was not confirmed in PESA-V2. No associations were detected with CACS in any of the two visits.

**Table 21.- Correlation with plaque thickness and Calcium score of representative acute-phase proteins and proteins described to associate with subclinical atherosclerosis**

| | **Plaque thickness** | | | | **Calcium Score** | | | | |
|---|---|---|---|---|---|---|---|---|---|
| | **V1** | | **V2** | | **V1** | | V2 | | |
| **Proteins** | **p-val** | FDR | **p-val** | **FDR** | **p-val** | **FDR** | **p-val** | **FDR** | **Refs.** |
| **Serum amyloId A-1 proteIn (SAA1)** | 0.300 | 0.409 | 0.372 | 0.480 | 0.021 | 0.125 | 0.211 | 0.467 | [1] |
| **C-reactive proteIn (CRP)** | 0.145 | 0.290 | 0.002 | **0.046 (up)** | 0.318 | 0.465 | 0.024 | 0.145 | [1], [2] |
| **Alpha-1-acid glycoprotein 1 (ORM1)** | 0.182 | 0.309 | 0.121 | 0.271 | 0.465 | 0.477 | 0.064 | 0.207 | [2] |
| **AdlponectIn (ADIPOQ)** | 0.083 | 0.234 | 0.002 | 0.101 | 0.208 | 0.381 | 0.007 | 0.357 | [3] |
| **FIbuiIn-1 (FBLN1)** | 0.003 | 0.017 | 0.032 | 0.133 | 0.013 | 0.053 | 0.163 | 0.336 | [4] |
| **Cadherin-13 (CDH13)** | 0.000 | **0.020 (down)** | 0.139 | 0.378 | 0.061 | 0.245 | 0.026 | 0.578 | [4] |
| **72 kDa type IV collagenase (MMP2)** | 0.308 | 0.409 | 0.391 | 0.473 | 0.393 | 0.477 | 0.280 | 0.467 | [4] |
| **ApollpoproteIn E (APOE)** | 0.325 | 0.388 | 0.103 | 0.238 | 0.243 | 0.321 | 0.237 | 0.337 | [4] |

### References:

1. Kristensen LP, et al. Plasma proteome profiling of atherosclerotic disease manifestations reveals elevated levels of the cytoskeletal protein vinculin. J Proteomics. 2014;101:141-53.
2. Yin, X., et al., Protein biomarkers of new-onset cardiovascular disease: prospective study from the systems approach to biomarker research in cardiovascular disease initiative. Arterioscler Thromb Vase Biol, 2014. 34(4): p. 939-45.
3. Saarikoski, L.A., et al., Adiponectin is related with carotid artery intima-media thickness and brachial flow-mediated dilatation in young adults--the Cardiovascular Risk in Young Finns Study. Ann Med, 2010. 42(8): p. 603-11.
4. Bhosale, S.D., et al., Serum Proteomic Profiling to Identify Biomarkers of Premature Carotid Atherosclerosis. Sci Rep, 2018. 8(1): p. 9209.

### 2.3 Association between plasma protein alterations and subclinical atherosclerosis are independent of risk scores

A logistic regression analysis was conducted to determine the effect of plasma protein levels on the likelihood that participants have subclinical atherosclerotic disease. Increased plasma levels of PIGR, IGHA2, APOA, HPT and HEP2 were significantly associated with increased likelihood of disease and the associations remained significant after adjustment by FHS 10-year score (Table 22 and Fig. 4). Decreased levels of IGHG1 were also associated with disease after adjustment by FHS 10-year score (Table 22). CD5L was not significantly associated, and GELS and IGKC lost their association with subclinical atherosclerosis after adjustment by FHS 10-year score (Table 22).

**Table 22.- Logistic regression analysis of association with the presence of subclinical atherosclerosis. Odds ratios refer to relative protein values determined by proteomics and expressed in units of standard deviation, using univariate logistic regression models (Univariate), or bivariate models adjusted by FHS 10-year score (Adj. F10Y).**

| **Proteins** | **Univariate** | | | | **Adj. by FHS 10-year** | | | |
|---|---|---|---|---|---|---|---|---|
| **Increased** | **p-val** | **OR** | **95% Cl** | | **p-val** | **OR** | **95% Cl** | |
| Polymeric immunoglobulin receptor (PIGR) | **<0.001** | 1.554 | 1.256 | 1.922 | **0.005** | 1.374 | 1.102 | 1.711 |
| Ig alpha-2 chain C region (IGHA2) | **0.008** | 1.295 | 1.069 | 1.567 | **0.024** | 1.259 | 1.031 | 1.538 |
| Apolipoprotein(a) (APOA) | **0.018** | 1.261 | 1.041 | 1.527 | **0.015** | 1.279 | 1.049 | 1.56 |
| Haptoglobin (HPT) | **<0.001** | 1.412 | 1.157 | 1.723 | **0.05** | 1.225 | 0.994 | 1.51 |
| Heparin cofactor 2 (HEP2) | **<0.001** | 1.414 | 1.195 | 1.673 | **0.005** | 1.285 | 1.078 | 1.531 |

| **Decreased** | **p-val** | **OR** | **95% Cl** | | **p-val** | **OR** | **95% Cl** | |
|---|---|---|---|---|---|---|---|---|
| Gelsolin (GELS) | **0.008** | 0.772 | 0.638 | 0.936 | 0.107 | 0.849 | 0.695 | 1.036 |
| Ig gamma-1 chain C region (IGHG1) | **0.007** | 0.819 | 0.709 | 0.946 | **0.017** | 0.834 | 0.719 | 0.968 |
| Ig kappa chain C region (IGKC) | **0.049** | 0.833 | 0.694 | 0.999 | 0.181 | 0.879 | 0.727 | 1.062 |
| CD5 antigen-like (CD5L) | 0.226 | 0.947 | 0.868 | 1.034 | 0.408 | 0.962 | 0.879 | 1.054 |

To select a biomarker panel, several protein combinations were tested using multivariate logistic regression models. IGHG1 did not reach statistical significance when other proteins were included in the same panel, while PIGR, IGHA2, APOA, HPT and HEP2 could be combined to form several three-protein panels, where PIGR could be replaced by IGHA2 and HEP2 by HPT (Table 23). The individual association of these five proteins with subclinical atherosclerosis remained significant after adjustment by BEWAT or ICHS risk scores (Fig. 4).

**Table 23.- Multivariate Logistic Regression Models for prediction of subclinical atherosclerosis in PESA-V1. Multivariate logistic regression models were used to determine association of protein values with the presence of subclinical atherosclerosis.**

| | | **p-val** | **OR** | **95% Cl** | |
|---|---|---|---|---|---|
| **Model 1** | Poly meric immunoglobulin receptor (PIGR) | **0.004** | 1.39 | 1.11 | 1.74 |
| | Ig alpha-2 chain C region(IGHA2) | **0.020** | 1.29 | 1.04 | 1.59 |
| | Apolipoprotein(a) (APOA) | **0.005** | 1.34 | 1.09 | 1.63 |
| | Haptoglobin (HPT) | 0.341 | 1.12 | 0.58 | 1.43 |
| | Heparin cofactor 2 (HEP2) | **0.032** | 1.28 | 1.02 | 1.61 |
| | Ig gamma-1 chain C region (IGHG1) | 0.425 | 0.93 | 0.79 | 1.10 |
| **Model 2** | Polymeric immunoglobulin receptor (PIGR) | **0.000** | 1.49 | 1.19 | 1.85 |
| | Apolipoprotein(a) (APOA) | 0.004 | 1.34 | 1.10 | 1.64 |
| | Heparin cofactor 2 (HEP2) | **0.001** | 1.34 | 1.12 | 1.60 |
| **Model 3** | Poly meric immunoglobulin receptor (PIGR) | **0.000** | 1.52 | 1.22 | 1.89 |
| | Apolipoprotein(a) (APOA) | **0.006** | 1.32 | 1.08 | 1.61 |
| | Haptoglobin (HPT) | **0.016** | 1.28 | 1.05 | 1.58 |
| **Model 4** | Ig alpha-2 chain C region(IGHA2) | **0.001** | 1.39 | 1.14 | 1.71 |
| | Apolipoprotein(a) (APOA) | **0.005** | 1.32 | 1.09 | 1.61 |
| | Heparin cofactor 2 (HEP2) | **0.000** | 1.49 | 1.25 | 1.77 |
| **Model 5** | Ig alpha-2 chain C region(IGHA2) | **0.009** | 1.30 | 1.07 | 1.58 |
| | Apolipoprotein(a) (APOA) | **0.015** | 1.27 | 1.05 | 1.55 |
| | Haptoglobin (HPT) | 0.001 | 1.39 | 1.14 | 1.70 |
| **Model 6** | FH510y | **0.000** | 1.74 | 1.38 | 2.21 |
| | Ig alpha-2 chain C region (IGHA2) | **0.022** | 1.27 | 1.03 | 1.55 |
| | Apolipoprotein(a) (APOA) | **0.012** | 1.29 | 1.06 | 1.58 |
| | Haptoglobin (HPT) | 0.071 | 1.21 | 0.98 | 1.49 |
| **Model 7** | BEWAT | **0.005** | 0.75 | 0.61 | 0.92 |
| | Ig alpha-2 chain C region (IGHA2) | **0.017** | 1.27 | 1.04 | 1.55 |
| | Apolipoprotein(a) (APOA) | **0.016** | 1.27 | 1.05 | 1.55 |
| | Haptoglobin (HPT) | **0.008** | 1.32 | 1.08 | 1.61 |
| **Model 8** | ICH5 | 0.021 | 0.79 | 0.64 | 0.96 |
| | Ig alpha-2 chain C region (IGHA2) | 0.012 | 1.29 | 1.06 | 1.57 |
| | Apolipoprotein(a) (APOA) | **0.018** | 1.27 | 1.04 | 1.54 |
| | Haptoglobin (HPT) | **0.008** | 1.32 | 1.08 | 1.62 |

### 2.4 The plasma proteins that associate with subclinical atherosclerosis accumulate in early atherosclerotic lesions

We determined the abundance of the five selected proteins in the media and intima layers of early human atherosclerosis lesions (fatty streak and fibrolipidic plaques). Quantitative proteomics analysis revealed markedly elevated absolute abundances of PIGR, IGHA2, APOA, HPT and HEP2 in the intimal relative to the medial layer (Fig. 5). Intimal accumulation of these five proteins increased further as lesions progressed from the fatty streak to the fibrolipidic stage (Fig. 5). Hence, the five proteins that associate with subclinical atherosclerosis in plasma also accumulate in atherosclerotic lesions in the initial stages of plaque formation.

### 2.5 Validation in a cohort from the Aragon Worker Health Study (AWHS)

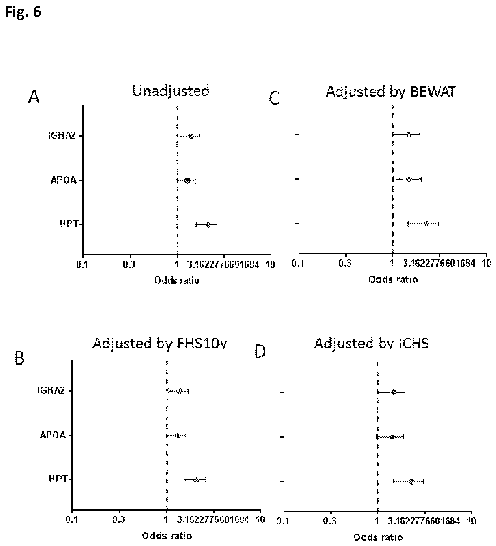

For the validation phase, we selected the panel composed by IGHA2, APOA and HPT, since these proteins could be measured using commercially available antibody-based kits used in the clinic. The validation study was conducted with a set of 350 plasma samples obtained from the AWHS cohort. Multivariate logistic regression analysis revealed that increased plasma concentration of each of the three proteins was significantly associated with an increased likelihood of subclinical atherosclerosis (Fig. 6A). This trend was maintained after adjusting by any of the three risk scores, indicating that the levels of these proteins were independently associated with subclinical atherosclerosis in the AWHS population (Fig. 6B-D).

### 2.6 Predictive value of the protein biomarker panel

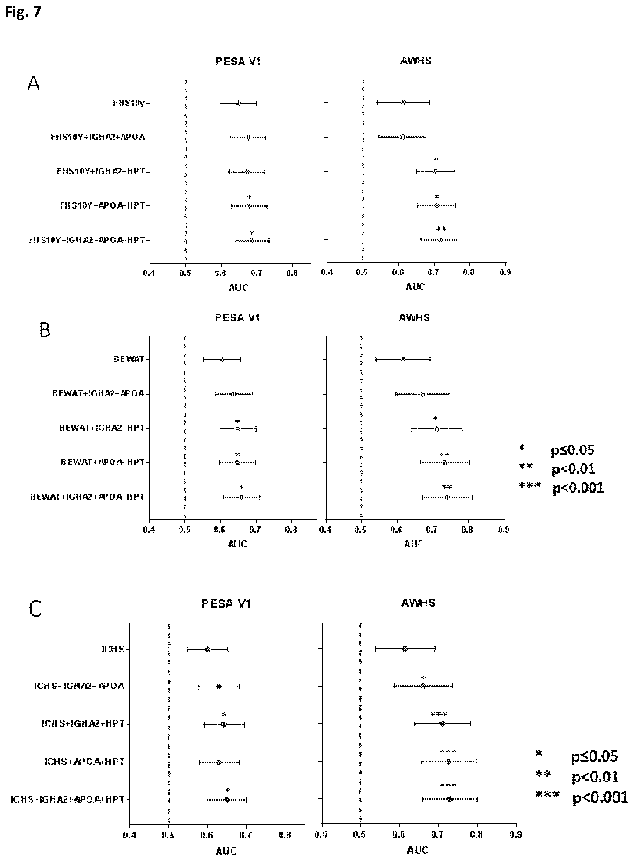

The ability of the three proteins, included as a biomarker panel, to improve subclinical atherosclerosis prediction by risk scores alone was assessed by receiver operating characteristic (ROC) analysis. In the PESA-V1 cohort, a panel composed by the three proteins and FHS 10-year score produced an AUC (0.76:0.71-0.81 95% CI) significantly better than the FHS 10-year score alone (0.71:0.66-0.77, p < 0.05, DeLong test) (Fig. 7A). Categorical and continuous NRI produced by the protein panel were NRI^{0.25} = 7% and NRI^{>0} = 40%, respectively (Table 24). The protein panel also improved disease prediction in the AWHS cohort (AUC = 0.72:0.66-0.77 vs 0.61:0.54-0.69, p < 0.01) (Fig. 7A), yielding NRI^{0.25} = 29% and NRI^{>0} = 51%. The biomarker panel also significantly improved the AUCs for the BEWAT score (Fig. 7B) and the ICHS score (Fig. 7C), with NRIs above 16% and reaching 64% in some cases (Table 24), in both populations. AUCs were also significantly improved by some combinations of just two proteins, for example, IGHA2 and HPT or APOA and HPT (Fig. 7 and Table 24).

**Table 24.- Net reclassification indexes of the protein biomarker panels. Listed are categorical (NRI^{0.25}) and continuous (NRI^{>0}) net reclassification indexes to evaluate improvement in performance to correctly classify individuals according to the presence and extent of subclinical atherosclerosis over that achieved by risk scores alone (FHS 10-year, BEWAT or ICHS). To calculate NRI^{0.25} the individuals were classified into four discrete risk categories.**

| | **PE5A** | | **AWH5** | |
|---|---|---|---|---|
| | **NRI ^{0.25}** | **NRI ^{>0}** | **NRI ^{0.25}** | **NRI^{>0}** |
| **FH510Y+IGHA2+APOA** | 5.0% | 31.5% | -4.8% | 2.0% |
| **FHS10Y+IGHA2+HPT** | 7.2% | 33.3% | 25.4% | 51.4% |
| **FH510Y+APOA+HPT** | 10.4% | 26.1% | 29.0% | 49.6% |
| **FH510Y+IGHA2+APOA+HPT** | 7.2% | 40.5% | 29.0% | 51.3% |
| **BEWAT+IGHA2+APOA** | 16.2% | 34.2% | 16.5% | 29.3% |
| **BEWAT+IGHA2+HPT** | 11.3% | 33.3% | 33.7% | 53.6% |
| **BEWAT+APOA+HPT** | 14.9% | 24.3% | 43.8% | 66.3% |
| **BEWAT+IGHA2+APOA+HPT** | 20.3% | 37.8% | 47.0% | 64.5% |
| **ICH5+IGHA2+APOA** | 8.6% | 32.4% | 3.7% | 30.4% |
| **ICH5+IGHA2+HPT** | 10.4% | 35.1% | 18.0% | 49.4% |
| **ICH5+APOA+HPT** | 8.6% | 32.4% | 17.2% | 42.8% |
| **ICHS+IGHA2+APOA+HPT** | 16.7% | 45.0% | 26.0% | 45.1% |

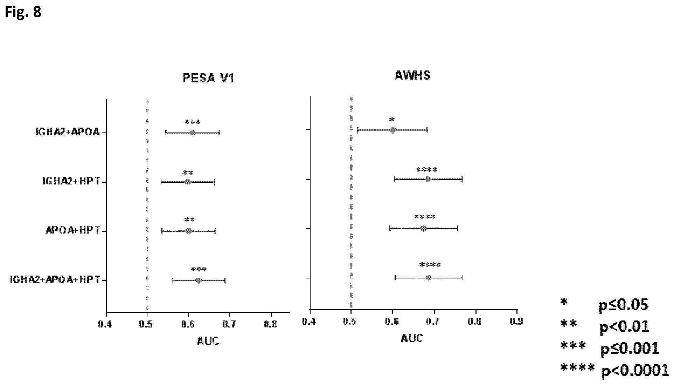

To assess whether these proteins provide useful information about individuals at low risk of CV events according to risk scores, we selected a subpopulation of participants with a FHS 10-year score < 0.1 (Fernandez-Friera L et al., 2015; Pencina MJ et al., 2009; Ford ES et al., 2004). In this subpopulation, the 3-protein biomarker panel efficiently predicted subclinical disease in PESA-V1 (AUC = 0.62:0.56-0.68, p < 0.001 vs AUC = 0.5) (Fig. 8 left), yielding NRIs of 5-8% (Table 25). The 3-protein panel also predicted disease in the AWHS subpopulation (AUC = 0.68:0.6-0.76, p < 0.0001 vs AUC = 0.5) (Fig. 8 right), with NRIs ranging from 10 to 36% (Table 25). Subclinical atherosclerosis in both populations was effectively predicted by some combinations of just 2 proteins (Fig. 8).

**Table 25.- Net reclassification indexes of the protein biomarker panels in the low-risk population. Listed are categorical (NRI^{0.5}) net reclassification indexes to evaluate improvement in performance to correctly classify individuals according to the presence and extent of subclinical aterosclerosis in the low risk subpopulations (FHS 10-year score < 0.1). Two discrete categories (risk/no risk) were used to calculate NRI^{0.5}.**

| | **PESA** | **AWHS** |
|---|---|---|
| | **NRI**^{0.5} | **NRI** ^{0.5} |
| **IGHA2+APOA** | 8.8% | 10.9% |
| **IGHA2+HPT** | 4.1% | 34.9% |
| **APOA+HPT** | 4.6% | 32.0% |
| **IGHA2+APOA+HPT** | 5.1% | 36.0% |

### List of references

1. Kannel WB and Wolf PA. Peripheral and cerebral atherothrombosis and cardiovascular events in different vascular territories: insights from the Framingham Study. Curr Atheroscler Rep. 2006;8:317-23.
2. Kannel WB. Risk factors for atherosclerotic cardiovascular outcomes in different arterial territories. J Cardiovasc Risk. 1994;1:333-9.
3. Khot UN, Khot MB, Bajzer CT, Sapp SK, Ohman EM, Brener SJ, Ellis SG, Lincoff AM and Topol EJ. Prevalence of conventional risk factors in patients with coronary heart disease. JAMA. 2003;290:898-904.
4. Silverman MG, Blaha MJ, Krumholz HM, Budoff MJ, Blankstein R, Sibley CT, Agatston A, Blumenthal RS and Nasir K. Impact of coronary artery calcium on coronary heart disease events in individuals at the extremes of traditional risk factor burden: the Multi-Ethnic Study of Atherosclerosis. Eur Heart J. 2014;35:2232-41.
5. Yusuf S, Rangarajan S, Teo K, Islam S, Li W, Liu L, Bo J, Lou Q, Lu F, Liu T, Yu L, Zhang S, Mony P, Swaminathan S, Mohan V, Gupta R, Kumar R, Vijayakumar K, Lear S, Anand S, Wielgosz A, Diaz R, Avezum A, Lopez-Jaramillo P, Lanas F, Yusoff K, Ismail N, Iqbal R, Rahman O, Rosengren A, Yusufali A, Kelishadi R, Kruger A, Puoane T, Szuba A, Chifamba J, Oguz A, McQueen M, McKee M, Dagenais G and Investigators P. Cardiovascular risk and events in 17 low-, middle-, and high-income countries. N Engl J Med. 2014;371:818-27.
6. Fernandez-Ortiz A, Jimenez-Borreguero LJ, Penalvo JL, Ordovas JM, Mocoroa A, Fernandez-Friera L, Laclaustra M, Garcia L, Molina J, Mendiguren JM, Lopez-Melgar B, de Vega VM, Alonso-Farto JC, Guallar E, Sillesen H, Rudd JH, Fayad ZA, Ibanez B, Sanz G and Fuster V. The Progression and Early detection of Subclinical Atherosclerosis (PESA) study: rationale and design. Am Heart J. 2013;166:990-8.
7. Peters SA, den Ruijter HM, Bots ML and Moons KG. Improvements in risk stratification for the occurrence of cardiovascular disease by imaging subclinical atherosclerosis: a systematic review. Heart. 2012;98:177-84.
8. Naqvi TZ, Mendoza F, Rafii F, Gransar H, Guerra M, Lepor N, Berman DS and Shah PK. High prevalence of ultrasound detected carotid atherosclerosis in subjects with low Framingham risk score: potential implications for screening for subclinical atherosclerosis. J Am Soc Echocardiogr. 2010;23:809-15.
9. Pen A, Yam Y, Chen L, Dennie C, McPherson R and Chow BJ. Discordance between Framingham Risk Score and atherosclerotic plaque burden. Eur Heart J. 2013;34:1075-82.
10. Fernandez-Friera L, Penalvo JL, Fernandez-Ortiz A, Ibanez B, Lopez-Melgar B, Laclaustra M, Oliva B, Mocoroa A, Mendiguren J, Martinez de Vega V, Garcia L, Molina J, Sanchez-Gonzalez J, Guzman G, Alonso-Farto JC, Guallar E, Civeira F, Sillesen H, Pocock S, Ordovas JM, Sanz G, Jimenez-Borreguero LJ and Fuster V. Prevalence, Vascular Distribution, and Multiterritorial Extent of Subclinical Atherosclerosis in a Middle-Aged Cohort: The PESA (Progression of Early Subclinical Atherosclerosis) Study. Circulation. 2015;131:2104-13.
11. Hoefer IE, Steffens S, Ala-Korpela M, Back M, Badimon L, Bochaton-Piallat ML, Boulanger CM, Caligiuri G, Dimmeler S, Egido J, Evans PC, Guzik T, Kwak BR, Landmesser U, Mayr M, Monaco C, Pasterkamp G, Tunon J, Weber C, Atherosclerosis ESCWG and Vascular B. Novel methodologies for biomarker discovery in atherosclerosis. European heart journal. 2015;36:2635-42.
12. Chaturvedi N, Coady E, Mayet J, Wright AR, Shore AC, Byrd S, Mc GTSA, Kooner JS, Schalkwijk CG and Hughes AD. Indian Asian men have less peripheral arterial disease than European men for equivalent levels of coronary disease. Atherosclerosis. 2007;193:204-12.
13. Koenig W and Khuseyinova N. Biomarkers of atherosclerotic plaque instability and rupture. Arterioscler Thromb Vasc Biol. 2007;27:15-26.
14. Avgerinos ED, Kadoglou NP, Moulakakis KG, Giannakopoulos TG and Liapis CD. Current role ofbiomarkers in carotid disease: a systematic review. Int J Stroke. 2011;6:337-45.
15. Donahue MP, Rose K, Hochstrasser D, Vonderscher J, Grass P, Chibout SD, Nelson CL, Sinnaeve P, Goldschmidt-Clermont PJ and Granger CB. Discovery of proteins related to coronary artery disease using industrial-scale proteomics analysis of pooled plasma. Am Heart J. 2006;152:478-85.
16. Basak T, Tanwar VS, Bhardwaj G, Bhardwaj N, Ahmad S, Garg G, V S, Karthikeyan G, Seth S and Sengupta S. Plasma proteomic analysis of stable coronary artery disease indicates impairment of reverse cholesterol pathway. Sci Rep. 2016;6:28042.
17. Wilhelm M, Schlegl J, Hahne H, Gholami AM, Lieberenz M, Savitski MM, Ziegler E, Butzmann L, Gessulat S, Marx H, Mathieson T, Lemeer S, Schnatbaum K, Reimer U, Wenschuh H, Mollenhauer M, Slotta-Huspenina J, Boese JH, Bantscheff M, Gerstmair A, Faerber F and Kuster B. Mass-spectrometry-based draft of the human proteome. Nature. 2014;509:582-7.
18. Kim MS, Pinto SM, Getnet D, Nirujogi RS, Manda SS, Chaerkady R, Madugundu AK, Kelkar DS, Isserlin R, Jain S, Thomas JK, Muthusamy B, Leal-Rojas P, Kumar P, Sahasrabuddhe NA, Balakrishnan L, Advani J, George B, Renuse S, Selvan LD, Patil AH, Nanjappa V, Radhakrishnan A, Prasad S, Subbannayya T, Raju R, Kumar M, Sreenivasamurthy SK, Marimuthu A, Sathe GJ, Chavan S, Datta KK, Subbannayya Y, Sahu A, Yelamanchi SD, Jayaram S, Rajagopalan P, Sharma J, Murthy KR, Syed N, Goel R, Khan AA, Ahmad S, Dey G, Mudgal K, Chatterjee A, Huang TC, Zhong J, Wu X, Shaw PG, Freed D, Zahari MS, Mukherjee KK, Shankar S, Mahadevan A, Lam H, Mitchell CJ, Shankar SK, Satishchandra P, Schroeder JT, Sirdeshmukh R, Maitra A, Leach SD, Drake CG, Halushka MK, Prasad TS, Hruban RH, Kerr CL, Bader GD, Iacobuzio-Donahue CA, Gowda H and Pandey A. A draft map of the human proteome. Nature. 2014;509:575-81.
19. Keshishian H, Burgess MW, Gillette MA, Mertins P, Clauser KR, Mani DR, Kuhn EW, Farrell LA, Gerszten RE and Carr SA. Multiplexed, Quantitative Workflow for Sensitive Biomarker Discovery in Plasma Yields Novel Candidates for Early Myocardial Injury. Mol Cell Proteomics. 2015;14:2375-93.
20. Casasnovas JA, Alcaide V, Civeira F, Guallar E, Ibanez B, Borreguero JJ, Laclaustra M, Leon M, Penalvo JL, Ordovas JM, Pocovi M, Sanz G and Fuster V. Aragon workers' health study--design and cohort description. BMC Cardiovasc Disord. 2012;12:45.
21. Fernandez-Friera L, Fuster V, Lopez-Melgar B, Oliva B, Garcia-Ruiz JM, Mendiguren J, Bueno H, Pocock S, Ibanez B, Fernandez-Ortiz A and Sanz J. Normal LDL-Cholesterol Levels Are Associated With Subclinical Atherosclerosis in the Absence of Risk Factors. J Am Coll Cardiol. 2017;70:2979-2991.
22. Laclaustra M, Casasnovas JA, Fernandez-Ortiz A, Fuster V, Leon-Latre M, Jimenez-Borreguero LJ, Pocovi M, Hurtado-Roca Y, Ordovas JM, Jarauta E, Guallar E, Ibanez B and Civeira F. Femoral and Carotid Subclinical Atherosclerosis Association With Risk Factors and Coronary Calcium: The AWHS Study. J Am Coll Cardiol. 2016;67:1263-74.
23. Baldan-Martin M, Lopez JA, Corbacho-Alonso N, Martinez PJ, Rodriguez-Sanchez E, Mourino-Alvarez L, Sastre-Oliva T, Martin-Rojas T, Rincón R, Calvo E, Vazquez J, Vivanco F, Padial LR, Alvarez-Llamas G, Ruiz-Hurtado G, Ruilope LM and Barderas MG. Potential role of new molecular plasma signatures on cardiovascular risk stratification in asymptomatic individuals. Scientific Reports. 2018;8.
24. Bagwan N, Bonzon-Kulichenko E, Calvo E, Lechuga-Vieco AV, Michalakopoulos S, Trevisan-Herraz M, Ezkurdia I, Rodriguez JM, Magni R, Latorre-Pellicer A, Enriquez JA and Vazquez J. Comprehensive Quantification of the Modified Proteome Reveals Oxidative Heart Damage in Mitochondrial Heteroplasmy. Cell Rep. 2018;23:3685-3697 e4.
25. Navarro P, Trevisan-Herraz M, Bonzon-Kulichenko E, Nunez E, Martinez-Acedo P, Perez-Hernandez D, Jorge I, Mesa R, Calvo E, Carrascal M, Hernaez ML, Garcia F, Barcena JA, Ashman K, Abian J, Gil C, Redondo JM and Vazquez J. General statistical framework for quantitative proteomics by stable isotope labeling. Journal of proteome research. 2014;13:1234-47.
26. Navarro P and Vazquez J. A refined method to calculate false discovery rates for peptide identification using decoy databases. Journal of proteome research. 2009;8:1792-6.
27. Jorge I, Navarro P, Martinez-Acedo P, Nunez E, Serrano H, Alfranca A, Redondo JM and Vazquez J. Statistical model to analyze quantitative proteomics data obtained by 18O/16O labeling and linear ion trap mass spectrometry: application to the study of vascular endothelial growth factor-induced angiogenesis in endothelial cells. Molecular & cellular proteomics : MCP. 2009;8:1130-49.
28. Garcia-Marques F, Trevisan-Herraz M, Martinez-Martinez S, Camafeita E, Jorge I, Lopez JA, Mendez-Barbero N, Mendez-Ferrer S, Del Pozo MA, Ibanez B, Andres V, Sanchez-Madrid F, Redondo JM, Bonzon-Kulichenko E and Vazquez J. A Novel Systems-Biology Algorithm for the Analysis of Coordinated Protein Responses Using Quantitative Proteomics. Molecular & cellular proteomics : MCP. 2016;15:1740-60.
29. Bonzon-Kulichenko E, Garcia-Marques F, Trevisan-Herraz M and Vazquez J. Revisiting peptide identification by high-accuracy mass spectrometry: problems associated with the use of narrow mass precursor windows. Journal of proteome research. 2015;14:700-10.
30. Martinez-Bartolome S, Navarro P, Martin-Maroto F, Lopez-Ferrer D, Ramos-Fernandez A, Villar M, Garcia-Ruiz JP and Vazquez J. Properties of average score distributions of SEQUEST: the probability ratio method. Molecular & cellular proteomics : MCP. 2008;7:1135-45.
31. DeLong ER, DeLong DM and Clarke-Pearson DL. Comparing the areas under two or more correlated receiver operating characteristic curves: a nonparametric approach. Biometrics. 1988;44:837-45.
32. Kerr KF, Wang Z, Janes H, McClelland RL, Psaty BM and Pepe MS. Net reclassification indices for evaluating risk prediction instruments: a critical review. Epidemiology. 2014;25:114-21.
33. Pepe MS, Fan J, Feng Z, Gerds T and Hilden J. The Net Reclassification Index (NRI): a Misleading Measure of Prediction Improvement Even with Independent Test Data Sets. Stat Biosci. 2015;7:282-295.
34. Leening MJ, Vedder MM, Witteman JC, Pencina MJ and Steyerberg EW. Net reclassification improvement: computation, interpretation, and controversies: a literature review and clinician's guide. Ann Intern Med. 2014;160:122-31.
35. Saarikoski LA, Huupponen RK, Viikari JS, Marniemi J, Juonala M, Kahonen M and Raitakari OT. Adiponectin is related with carotid artery intima-media thickness and brachial flow-mediated dilatation in young adults--the Cardiovascular Risk in Young Finns Study. Ann Med. 2010;42:603-11.
36. Bhosale SD, Moulder R, Venalainen MS, Koskinen JS, Pitkanen N, Juonala MT, Kahonen MAP, Lehtimaki TJ, Viikari JSA, Elo LL, Goodlett DR, Lahesmaa R and Raitakari OT. Serum Proteomic Profiling to Identify Biomarkers of Premature Carotid Atherosclerosis. Sci Rep. 2018;8:9209.
37. Pencina MJ, D'Agostino RB, Sr., Larson MG, Massaro JM and Vasan RS. Predicting the 30-year risk of cardiovascular disease: the framingham heart study. Circulation. 2009;119:3078-84.
38. Ford ES, Giles WH and Mokdad AH. The distribution of 10-Year risk for coronary heart disease among US adults: findings from the National Health and Nutrition Examination Survey III. Journal of the American College of Cardiology. 2004;43:1791-6.
39. Trevisan-Herraz M, Bagwan N, Garcia-Marques F, Rodriguez JM, Jorge I, Ezkurdia I, Bonzon-Kulichenko E and Vazquez J. SanXoT: a modular and versatile package for the quantitative analysis of high-throughput proteomics experiments. Bioinformatics. 2018.
40. Oikonen M, Wendelin-Saarenhovi M, Siitonen N, Sainio A, Juonala M, Kahonen M, Lyytikainen LP, Seppala I, Lehtimaki T, Viikari JS, Jarvelainen H and Raitakari OT. Tissue inhibitor of matrix metalloproteinases 4 (TIMP4) in a population of young adults: relations to cardiovascular risk markers and carotid artery intima-media thickness. The Cardiovascular Risk in Young Finns Study. Scand J Clin Lab Invest. 2012;72:540-6.
41. Tsimikas S. A Test in Context: Lipoprotein(a): Diagnosis, Prognosis, Controversies, and Emerging Therapies. J Am Coll Cardiol. 2017;69:692-711.
42. Delbosc S, Bayles RG, Laschet J, Ollivier V, Ho-Tin-Noe B, Touat Z, Deschildre C, Morvan M, Louedec L, Gouya L, Guedj K, Nicoletti A and Michel JB. Erythrocyte Efferocytosis by the Arterial Wall Promotes Oxidation in Early-Stage Atheroma in Humans. Front Cardiovasc Med. 2017;4:43.
43. Rau JC, Deans C, Hoffman MR, Thomas DB, Malcom GT, Zieske AW, Strong JP, Koch GG and Church FC. Heparin cofactor II in atherosclerotic lesions from the Pathobiological Determinants of Atherosclerosis in Youth (PDAY) study. Exp Mol Pathol. 2009;87:178-83.
44. Orso E and Schmitz G. Lipoprotein(a) and its role in inflammation, atherosclerosis and malignancies. Clin Res Cardiol Suppl. 2017;12:31-37.
45. Greif M, Amoldt T, von Ziegler F, Ruemmler J, Becker C, Wakili R, D'Anastasi M, Schenzle J, Leber AW and Becker A. Lipoprotein (a) is independently correlated with coronary artery calcification. Eur J Intern Med. 2013;24:75-9.
46. Ellis KL, Boffa MB, Sahebkar A, Koschinsky ML and Watts GF. The renaissance of lipoprotein(a): Brave new world for preventive cardiology? Prog Lipid Res. 2017;68:57-82.
47. Ellis KL and Watts GF. Is Lipoprotein(a) Ready for Prime-Time Use in the Clinic? Cardiol Clin. 2018;36:287-298.
48. Levy AP, Hochberg I, Jablonski K, Resnick HE, Lee ET, Best L, Howard BV and Strong Heart S. Haptoglobin phenotype is an independent risk factor for cardiovascular disease in individuals with diabetes: The Strong Heart Study. J Am Coll Cardiol. 2002;40:1984-90.
49. Levy AP, Larson MG, Corey D, Lotan R, Vita JA and Benjamin EJ. Haptoglobin phenotype and prevalent coronary heart disease in the Framingham offspring cohort. Atherosclerosis. 2004;172:361-5.
50. Lee CW, Cheng TM, Lin CP and Pan JP. Plasma haptoglobin concentrations are elevated in patients with coronary artery disease. PLoS One. 2013;8:e76817.
51. Holme I, Aastveit AH, Hammar N, Jungner I and Walldius G. Haptoglobin and risk of myocardial infarction, stroke, and congestive heart failure in 342,125 men and women in the Apolipoprotein MOrtality RISk study (AMORIS). Ann Med. 2009;41:522-32.
52. Engstrom G, Lind P, Hedblad B, Stavenow L, Janzon L and Lindgarde F. Effects of cholesterol and inflammation-sensitive plasma proteins on incidence of myocardial infarction and stroke in men. Circulation. 2002;105:2632-7.
53. Holme I, Aastveit AH, Hammar N, Jungner I and Walldius G. Inflammatory markers, lipoprotein components and risk of major cardiovascular events in 65,005 men and women in the Apolipoprotein MOrtality RISk study (AMORIS). Atherosclerosis. 2010;213:299-305.
54. Brea D, Sobrino T, Blanco M, Fraga M, Agulla J, Rodriguez-Yanez M, Rodriguez-Gonzalez R, Perez de la Ossa N, Leira R, Forteza J, Davalos A and Castillo J. Usefulness of haptoglobin and serum amyloid A proteins as biomarkers for atherothrombotic ischemic stroke diagnosis confirmation. Atherosclerosis. 2009;205:561-7.
55. Sabatine MS, Morrow DA, Jablonski KA, Rice MM, Warnica JW, Domanski MJ, Hsia J, Gersh BJ, Rifai N, Ridker PM, Pfeffer MA, Braunwald E and Investigators P. Prognostic significance of the Centers for Disease Control/American Heart Association high-sensitivity C-reactive protein cut points for cardiovascular and other outcomes in patients with stable coronary artery disease. Circulation. 2007;115:1528-36.
56. Luczak M, Formanowicz D, Pawliczak E, Wanic-Kossowska M, Wykretowicz A and Figlerowicz M. Chronic kidney disease-related atherosclerosis - proteomic studies of blood plasma. Proteome science. 2011;9:25.
57. Battes LC, Akkerhuis KM, Cheng JM, Garcia-Garcia HM, Oemrawsingh RM, de Boer SP, Regar E, van Geuns RJ, Serruys PW, Boersma E and Kardys I. Circulating acute phase proteins in relation to extent and composition of coronary atherosclerosis and cardiovascular outcome: results from the ATHEROREMO-IVUS study. Int J Cardiol. 2014;177:847-53.
58. Kristensen LP, Larsen MR, Mickley H, Saaby L, Diederichsen AC, Lambrechtsen J, Rasmussen LM and Overgaard M. Plasma proteome profiling of atherosclerotic disease manifestations reveals elevated levels of the cytoskeletal protein vinculin. J Proteomics. 2014;101:141-53.
59. Kanagawa Y, Shigekiyo T, Aihara K, Akaike M, Azuma H and Matsumoto T. Molecular mechanism of type I congenital heparin cofactor (HC) II deficiency caused by a missense mutation at reactive P2 site: HC II Tokushima. Thromb Haemost. 2001;85:101-7.
60. Takamori N, Azuma H, Kato M, Hashizume S, Aihara K, Akaike M, Tamura K and Matsumoto T. High plasma heparin cofactor II activity is associated with reduced incidence of in-stent restenosis after percutaneous coronary intervention. Circulation. 2004;109:481-6.
61. Aihara K, Azuma H, Akaike M, Kurobe H, Takamori N, Ikeda Y, Sumitomo Y, Yoshida S, Yagi S, Iwase T, Ishikawa K, Sata M, Kitagawa T and Matsumoto T. Heparin cofactor II is an independent protective factor against peripheral arterial disease in elderly subjects with cardiovascular risk factors. J Atheroscler Thromb. 2009;16:127-34.
62. Aihara K, Azuma H, Takamori N, Kanagawa Y, Akaike M, Fujimura M, Yoshida T, Hashizume S, Kato M, Yamaguchi H, Kato S, Ikeda Y, Arase T, Kondo A and Matsumoto T. Heparin cofactor II is a novel protective factor against carotid atherosclerosis in elderly individuals. Circulation. 2004;109:2761-5.
63. Aihara K, Azuma H, Akaike M, Sata M and Matsumoto T. Heparin cofactor II as a novel vascular protective factor against atherosclerosis. J Atheroscler Thromb. 2009;16:523-31.
64. Ikeda Y, Aihara K-i, Yoshida S, Iwase T, Tajima S, Izawa-Ishizawa Y, Kihira Y, Ishizawa K, Tomita S, Tsuchiya K, Sata M, Akaike M, Kato S, Matsumoto T and Tamaki T. Heparin Cofactor II, a Serine Protease Inhibitor, Promotes Angiogenesis via Activation of the AMP-activated Protein Kinase-Endothelial Nitric-oxide Synthase Signaling Pathway. Journal of Biological Chemistry. 2012;287:34256-34263.
65. Kaetzel CS. The polymeric immunoglobulin receptor: bridging innate and adaptive immune responses at mucosal surfaces. Immunol Rev. 2005;206:83-99.
66. Wines BD and Hogarth PM. IgA receptors in health and disease. Tissue Antigens. 2006;68:103-14.
67. Hansson GK and Hermansson A. The immune system in atherosclerosis. Nat Immunol. 2011;12:204-12.
68. Tsiantoulas D, Diehl CJ, Witztum JL and Binder CJ. B cells and humoral immunity in atherosclerosis. Circ Res. 2014;114:1743-56.
69. Ohlmeier S, Mazur W, Linja-Aho A, Louhelainen N, Ronty M, Toljamo T, Bergmann U and Kinnula VL. Sputum proteomics identifies elevated PIGR levels in smokers and mild-to-moderate COPD. J Proteome Res. 2012;11:599-608.
70. Ngo D, Sinha S, Shen D, Kuhn EW, Keyes MJ, Shi X, Benson MD, O'Sullivan JF, Keshishian H, Farrell LA, Fifer MA, Vasan RS, Sabatine MS, Larson MG, Carr SA, Wang TJ and Gerszten RE. Aptamer-Based Proteomic Profiling Reveals Novel Candidate Biomarkers and Pathways in Cardiovascular Disease. Circulation. 2016;134:270-85.
71. de Hoog VC, Timmers L, Schoneveld AH, Wang JW, van de Weg SM, Sze SK, van Keulen JK, Hoes AW, den Ruijter HM, de Kleijn DP and Mosterd A. Serum extracellular vesicle protein levels are associated with acute coronary syndrome. Eur Heart J Acute Cardiovasc Care. 2013;2:53-60.
72. Muscari A, Bozzoli C, Gerratana C, Zaca F, Rovinetti C, Zauli D, La Placa M and Puddu P. Association of serum IgA and C4 with severe atherosclerosis. Atherosclerosis. 1988;74:179-86.
73. Muscari A, Bozzoli C, Puddu GM, Rovinetti C, Vallar G, Renzi C, Scarani P, Molinaro N and Puddu P. Increased serum IgA levels in subjects with previous myocardial infarction or other major ischemic events. Cardiology. 1993;83:383-9.
74. Kovanen PT, Manttari M, Palosuo T, Manninen V and Aho K. Prediction of myocardial infarction in dyslipidemic men by elevated levels of immunoglobulin classes A, E, and G, but not M. Arch Intern Med. 1998;158:1434-9.
75. Kaneko T, Amano H, Kawano S, Minowa K, Ando S, Watanabe T, Nakano S, Suzuki J, Morimoto S, Tokano Y and Takasaki Y. Increased serum concentration of BAFF/APRIL and IgA2 subclass in patients with mixed connective tissue disease complicated by interstitial lung disease. Mod Rheumatol. 2014;24:310-5.
76. Litinskiy MB, Nardelli B, Hilbert DM, He B, Schaffer A, Casali P and Cerutti A. DCs induce CD40-independent immunoglobulin class switching through BLyS and APRIL. Nat Immunol. 2002;3:822-9.
77. Burillo, E., Jorge, I., Martinez-Lopez, D., Camafeita, E., Blanco-Colio, L.M., Trevisan-Herraz, M., Ezkurdia, I., Egido, J., Michel, J.-B., Meilhac, O., et al. (2016). Quantitative HDL Proteomics Identifies Peroxiredoxin-6 as a Biomarker of Human Abdominal Aortic Aneurysm. Scientific Reports 6, 38477.1.
78. Fernandez-Alvira, J.M., Fuster, V., Pocock, S., Sanz, J., Fernandez-Friera, L., Laclaustra, M., Fernández-Jiménez, R., Mendiguren, J., Fernández-Ortiz, A., Ibáñez, B., et al. (2017). Predicting Subclinical Atherosclerosis in Low-Risk Individuals. Journal of the American College of Cardiology 70, 2463-2473.
79. Gomez-Serrano, M., Camafeita, E., Garcia-Santos, E., Lopez, J.A., Rubio, M.A., Sanchez-Pernaute, A., Torres, A., Vazquez, J., and Peral, B. (2016). Proteome-wide alterations on adipose tissue from obese patients as age-, diabetes- and gender-specific hallmarks. Sci Rep 6, 25756.
80. Jorge, I., Navarro, P., Martinez-Acedo, P., Nunez, E., Serrano, H., Alfranca, A., Redondo, J.M., and Vazquez, J. (2009). Statistical model to analyze quantitative proteomics data obtained by 18O/16O labeling and linear ion trap mass spectrometry: application to the study of vascular endothelial growth factor-induced angiogenesis in endothelial cells. Molecular & cellular proteomics : MCP 8, 1130-1149.
81. Kannel, W.B., Feinleib, M., McNamara, P.M., Garrison, R.J., and Castelli, W.P. (1979). An investigation of coronary heart disease in families. The Framingham offspring study. Am J Epidemiol 110, 281-290.
82. Latorre-Pellicer, A., Moreno-Loshuertos, R., Lechuga-Vieco, A.V., Sanchez-Cabo, F., Torroja, C., Acin-Perez, R., Calvo, E., Aix, E., Gonzalez-Guerra, A., Logan, A., et al. (2016). Mitochondrial and nuclear DNA matching shapes metabolism and healthy ageing. Nature 535, 561-565.
83. Martin-Alonso, M., Garcia-Redondo, A.B., Guo, D., Camafeita, E., Martinez, F., Alfranca, A., Mendez-Barbero, N., Pollan, A., Sanchez-Camacho, C., Denhardt, D.T., et al. (2015). Deficiency of MMP17/MT4-MMP proteolytic activity predisposes to aortic aneurysm in mice. Circulation research 117, e13-26.
84. Navarro, P., Trevisan-Herraz, M., Bonzon-Kulichenko, E., Nunez, E., Martinez-Acedo, P., Perez-Hernandez, D., Jorge, I., Mesa, R., Calvo, E., Carrascal, M., et al. (2014). General statistical framework for quantitative proteomics by stable isotope labeling. Journal of proteome research 13, 1234-1247.
85. Navarro, P., and Vazquez, J. (2009). A refined method to calculate false discovery rates for peptide identification using decoy databases. Journal of proteome research 8, 1792-1796.
86. Splansky, G.L., Corey, D., Yang, Q., Atwood, L.D., Cupples, L.A., Benjamin, E.J., D'Agostino, R.B., Sr., Fox, C.S., Larson, M.G., Murabito, J.M., et al. (2007). The Third Generation Cohort of the National Heart, Lung, and Blood Institute's Framingham Heart Study: design, recruitment, and initial examination. Am J Epidemiol 165, 1328-1335.

## Claims

1. A method for the screening, diagnosis and/or monitoring of subclinical atherosclerosis, preferably generalized subclinical atherosclerosis, in a subject, wherein said method comprises:
a) determining in a biological sample isolated from said subject the protein expression levels of:
i. PIGR and/or IGHA2; and
ii. optionally, one, two, three, four or five biomarkers, selected from APOA, HPT, HEP2, ITIH1 and C5;
b) comparing the levels of the biomarkers with a reference value;
c) wherein when the levels in the subject's sample of PIGR and/or IGHA2, and optionally APOA, HPT, HEP2, ITIH1 and/or C5, are increased with respect to the corresponding reference value is indicative of subclinical atherosclerosis.

2. The method according to claim 1 , wherein step a) comprises determining the protein expression levels of PIGR and/or IGHA2; and one, two, three, four or five biomarkers selected from the list consisting of APOA, HPT, HEP2, ITIH1 and C5.

3. The method according to any of claims 1 or 2, wherein step a) comprises determining the protein expression levels of a plurality of biomarkers selected from the group consisting of:
a. PIGR and/or IGHA2, and APOA;
b. PIGR and/or IGHA2, and HPT;
c. PIGR and/or IGHA2 , and HEP2 ;
d. PIGR and/or IGHA2, and C5;
e. PIGR and/or IGHA2, and ITIH1 ;
f. PIGR and/or IGHA2, APOA and HPT;
g. PIGR and/or IGHA2, APOA and HEP2;
h. PIGR and/or IGHA2, APOA and C5;
i. PIGR and/or IGHA2, APOA and ITIH1 ;
j. PIGR and/or IGHA2, HPT and HEP2;
k. PIGR and/or IGHA2, HPT and C5;
l. PIGR and/or IGHA2, HPT and ITIH1 ;
m. PIGR and/or IGHA2, HEP2 and C5;
n. PIGR and/or IGHA2, HEP2 and ITIH1 ;
o. PIGR and/or IGHA2, APO A, HPT and C5;
p. PIGR and/or IGHA2, APOA, HPT and HTH1 ;
q. PIGR and/or IGHA2, APOA, HEP2 and C5;
r. PIGR and/or IGHA2, APOA, HEP2 and ITIH1 ;
s. PIGR and/or IGHA2, HPT, HEP2 and C5;
t. PIGR and/or IGHA2, HPT, HEP2 and ITIH1 ; and
u. PIGR and/or IGHA2, APOA, HPT and HEP2.

4. The method according to any of claims 1 to 3, wherein said method further comprises determining in said biological sample the protein expression levels of IGHG1; wherein when the levels of IGHG1 in the subject's sample are decreased with respect to a reference value is indicative of subclinical atherosclerosis.

5. The method according to any of claims 1 to 3, wherein IGHA2 levels are relative levels with respect to any of IGHA1 , IGHG1 , IGHG2 or a combination thereof, preferably wherein IGHA2 levels are relative levels with respect to IGHA1 or with respect to the average levels of IGHG1 and IGHG2.

6. The method according to any of claims 1 to 5, wherein said method further comprises conducting a traditional cardiovascular risk score, preferably wherein said traditional cardiovascular risk score is selected from the group consisting of 10y FHS, 30-y FHS, ICHS and the BEWAT scores, more preferably wherein said cardiovascular risk score is 10-y FHS.

7. The method according to any of claims 1 to 6, wherein said method is a method for the screening and/or diagnosis of subclinical atherosclerosis in a subject classified as low risk when conducting a traditional cardiovascular risk score.

8. The method according to any of claims 1 to 7, wherein said biological sample isolated from the subject is a serum, blood or plasma sample, preferably is a serum sample.

9. The method according to any of claims 1 to 8, wherein said subject is a human subject.

10. The method according to any of claims 1 to 9, wherein steps b) and/or c) are implemented by a computer.

11. A data-processing apparatus comprising means adapted for carrying out the steps of a method ol claim 10.

12. A computer program comprising instructions which, when the program is executed by a computer, cause the computer to carry out the steps of the method of claim 10.

13. A computer-readable storage medium having stored thereon a computer program according to claim 12.

14. Use of a kit in a method for the screening, diagnosis and/or monitoring of a subject according to any of claims 1 to 10, wherein said kit comprises:
a. a reagent for determining the protein expression levels of PIGR and/or IGHA2; and
b. optionally, a reagent for determining the protein expression levels of one, two, three, four or five of APOA, HPT, HEP2, ITIH1 or C5;
c. optionally, further comprising instructions for the use of said reagents in determining said proteins expression levels in a biological sample isolated from a subject.

15. Use of a kit according to claim 14, the kit comprising reagents adequate for the determination of the protein expression levels of the following biomarkers:
i. PIGR and/or IGHA2;
ii. optionally, one, two, three, four or five biomarkers selected from APOA, HPT, HEP2, ITIH1 and C5;
wherein said kit comprises:
a. an affinity reagent for PIGR and/or IGHA2;
b. optionally, an affinity reagent for one, two, three four or five of APOA, HPT, HEP2, ITIH1 and C5;
c. optionally, further comprising instructions for the use of said reagents in determining said protein expression levels in a biological sample isolated from a subject.

## Patentansprüche

1. Verfahren für das Screening, die Diagnose und/oder die Überwachung von subklinischer Atherosklerose, vorzugsweise generalisierte subklinische Atherosklerose, in einem Subjekt, wobei das Verfahren umfasst.
a) Bestimmung der Proteinexpressionsspiegel in einer aus dem Subjekt isolierten biologischen Probe von.
i. PIGR und/oder IGHA2 und
ii. gegebenenfalls einem, zwei, drei, vier oder fünf Biomarkern, ausgewählt aus APOA, HPT, HEP2, ITIH1 und C5,
b) Vergleich der der Biomarkerspiegel mit einem Referenzwert,
c) wobei, wenn die Spiegel von PIGR und/oder IGHA2 und gegebenenfalls APOA, HPT, HEP2, ITIH1 und/oder C5 in der Probe des Subjekts gegenüber dem entsprechenden Referenzwert erhöht sind, dies auf eine subklinische Atherosklerose hinweist.

2. Verfahren nach Anspruch 1, wobei Schritt a) die Bestimmung der Proteinexpressionsspiegel von PIGR und/oder IGHA2 sowie von einem, zwei, drei, vier oder fünf Biomarkern umfasst, die aus der Liste bestehend aus APOA, HPT, HEP2, ITIH1 und C5 ausgewählt sind.

3. Verfahren nach einem der Ansprüche 1 oder 2, wobei Schritt a) die Bestimmung der Proteinexpressionsspiegel einer Vielzahl von Biomarkern, ausgewählt aus der Gruppe, bestehend aus.
a. PIGR und/oder IGHA2 und APOA,
b. PIGR und/oder IGHA2 und HPT,
c. PIGR und/oder IGHA2 und HEP2,
d. PIGR und/oder IGHA2 und C5,
e. PIGR und/oder IGHA2 und ITIH1,
f. PIGR und/oder IGHA2, APOA und HPT,
g. PIGR und/oder IGHA2, APOA und HEP2,
h. PIGR und/oder IGHA2, APOA und C5,
i. PIGR und/oder IGHA2, APOA und ITIH1,
j. PIGR und/oder IGHA2, HPT und HEP2,
k. PIGR und/oder IGHA2, HPT und C5,
l. PIGR und/oder IGHA2, HPT und ITIH1,
m. PIGR und/oder IGHA2, HEP2 und C5,
n. PIGR und/oder IGHA2, HEP2 und ITIH1,
o. PIGR und/oder IGHA2, APOA, HPT und C5,
p. PIGR und/oder IGHA2, APOA, HPT und HTH1 ,
q. PIGR und/oder IGHA2, APOA, HEP2 und C5,
r. PIGR und/oder IGHA2, APOA, HEP2 und ITIH1,
s. PIGR und/oder IGHA2, HPT, HEP2 und C5,
t. PIGR und/oder IGHA2, HPT, HEP2 und ITIH1, und
u. PIGR und/oder IGHA2, APOA, HPT und HEP2 umfasst.

4. Verfahren nach einem der Ansprüche 1 bis 3, wobei das Verfahren ferner die Bestimmung der Proteinexpressionsspiegel von IGHG1 in der biologischen Probe umfasst, wobei, wenn die Spiegel von IGHG1 in der Probe des Subjekts gegenüber dem Referenzwert vermindert sind, dies auf eine subklinische Atherosklerose hinweist.

5. Verfahren nach einem der Ansprüche 1 bis 3, wobei die IGHA2-Spiegel relative Werte in Bezug auf IGHA1, IGHG1, IGHG2 oder eine Kombination davon sind, vorzugsweise wobei die IGHA2-Spiegel relative Werte in Bezug auf IGHA1 oder in Bezug auf die durchschnittlichen Werte von IGHG1 und IGHG2 sind.

6. Verfahren nach einem der Ansprüche 1 bis 5, wobei das Verfahren ferner die Durchführung eines traditionellen kardiovaskulären Risikoscores umfasst, wobei der traditionelle kardiovaskuläre Risikoscore vorzugsweise aus der Gruppe ausgewählt ist, die aus den 10y FHS, 30y FHS, ICHS und den BEWAT-Scores besteht, wobei der kardiovaskuläre Risikoscore vorzugsweise der 10-y FHS ist.

7. Verfahren nach einem der Ansprüche 1 bis 6, wobei das Verfahren ein Verfahren zum Screening und/oder zur Diagnose von subklinischer Atherosklerose bei einem Subjekt ist, die bei der Durchführung eines herkömmlichen kardiovaskulären Risikoscores als geringes Risiko eingestuft wird.

8. Verfahren nach einem der Ansprüche 1 bis 7, wobei die aus dem Subjekt isolierte biologische Probe eine Serum-, Blut- oder Plasmaprobe, vorzugsweise eine Serumprobe ist.

9. Verfahren nach einem der Ansprüche 1 bis 8, wobei das Subjekt ein Mensch ist.

10. Verfahren nach einem der Ansprüche 1 bis 9, wobei die Schritte b) und/oder c) von einem Computer ausgeführt werden.

11. Datenverarbeitungsgerät mit Mitteln, die zur Durchführung der Schritte eines Verfahrens nach Anspruch 10 geeignet sind.

12. Computerprogramm mit Befehlen, die den Computer veranlassen, die Schritte des Verfahrens nach Anspruch 10 auszuführen, wenn das Programm von einem Computer ausgeführt wird.

13. Computerlesbares Speichermedium, auf dem ein Computerprogramm nach Anspruch 12 gespeichert ist.

14. Verwendung eines Kits in einem Verfahren zum Screening, zur Diagnose und/oder zur Überwachung eines Subjekts nach einem der Ansprüche 1 bis 10, wobei das Kit umfasst:
a. ein Reagens zur Bestimmung des Proteinexpressionsspiegels von PIGR und/oder IGHA2, und
b. gegebenenfalls ein Reagenz zur Bestimmung der Proteinexpressionsspiegel von einem, zwei, drei, vier oder fünf aus APOA, HPT, HEP2, ITIH1 oder C5,
c. gegebenenfalls ferner eine Anleitung für die Verwendung der Reagenzien zur Bestimmung der Proteinexpressionsspiegel in einer aus dem Subjekt isolierten biologischen Probe.

15. Verwendung eines Kits nach Anspruch 14, wobei das Kit Reagenzien umfasst, die für die Bestimmung der Proteinexpressionsspiegel der folgenden Biomarker geeignet sind:
i. PIGR und/oder IGHA2,
ii. gegebenenfalls einen, zwei, drei, vier oder fünf Biomarker, ausgewählt aus APOA, HPT,HEP2, ITIH1 und C5,
wobei der Kit umfasst.
a. ein Affinitätsreagenz für PIGR und/oder IGHA2,
b. gegebenenfalls ein Affinitätsreagenz für einen, zwei, drei, vier oder fünf aus APOA, HPT, HEP2, ITIH1 und C5,
c. gegebenenfalls ferner Anweisungen für die Verwendung der Reagenzien bei der Bestimmung der Proteinexpressionsspiegel in einer aus dem Subjekt isolierten biologischen Probe.

## Revendications

1. Procédé pour le dépistage, le diagnostic et/ou la surveillance de l'athérosclérose subclinique, de préférence l'athérosclérose subclinique généralisée, chez un sujet,
le procédé consistant à :
a) déterminer dans un échantillon biologique isolé du sujet des niveaux d'expression protéique de :
i. PIGR et/ou IGHA2 ; et
ii. éventuellement, un, deux, trois, quatre ou cinq biomarqueurs, choisis dans l'ensemble APOA, HPT, HEP2, ITIH1 et C5 ;
b) comparer les niveaux des biomarqueurs avec une valeur de référence ;
c) signaler une athérosclérose subclinique si les niveaux dans l'échantillon du sujet de PIGR et/ou IGHA2, et éventuellement APOA, HPT, HEP2, ITIH1 et/ou C5, sont augmentés par rapport à la valeur de référence correspondante.

2. Procédé selon la revendication 1,
dans lequel
l'étape a) consistant à déterminer des niveaux d'expression protéique de PIGR et/ou IGHA2 ; et un, deux, trois, quatre ou cinq biomarqueurs choisis dans l'ensemble APOA, HPT, HEP2, ITIH1 et C5.

3. Procédé selon l'une quelconque des revendications 1 ou 2,
dans lequel
l'étape a) consistant à déterminer des niveaux d'expression protéique d'une pluralité de biomarqueurs choisis dans le groupe comprenant :
a. PIGR et/ou IGHA2, et APOA ;
b. PIGR et/ou IGHA2, et HPT ;
c. PIGR et/ou IGHA2, et HEP2 ;
d. PIGR et/ou IGHA2, et C5 ;
e. PIGR et/ou IGHA2, et ITIH1 ;
f. PIGR et/ou IGHA2, APOA et HPT ;
g. PIGR et/ou IGHA2, APOA et HEP2 ;
h. PIGR et/ou IGHA2, APOA et C5 ;
i. PIGR et/ou IGHA2, APOA et ITIH1
j. PIGR et/ou IGHA2, HPT et HEP2 ;
k. PIGR et/ou IGHA2, HAPT et C5 ;
l. PIGR et/ou IGHA2, HPT et ITIH1 ;
m. PIGR et/ou IGHA2, HEP2 et C5 ;
n. PIGR et/ou IGHA2, HEP2 et ITIH1 ;
o. PIGR et/ou IGHA2, APO A, HPT et C5 ;
p. PIGR et/ou IGHA2, APOA, HPT et HTH1 ;
q. PIGR et/ou IGHA2, APOA, HEP2 et C5 ;
r. PIGR et/ou IGHA2, APOA, HEP2 et ITIH1 ;
s. PIGR et/ou IGHA2, HPT, HEP2 et C5 ;
t. PIGR et/ou IGHA2, HPT, HEP2 et ITIH1 ; et
u. PIGR et/ou IGHA2, APOA, HPT et HEP2.

4. Procédé selon l'une quelconque des revendications 1 à 3,
consistant en outre à :
- déterminer dans l'échantillon biologique, des niveaux d'expression protéique d'IGHG1, et
- indiquer une athérosclérose subclinique si les niveaux de IGHG1 dans l'échantillon du sujet sont diminués par rapport à une valeur de référence.

5. Procédé selon l'une quelconque des revendications 1 à 3,
dans lequel
les niveaux de IGHA2 sont des niveaux relatifs par rapport à l'un quelconque de IGHAI, IGHG1, IGHG2 ou une combinaison de ceux-ci, de préférence
les niveaux de IGHA2 sont des niveaux relatifs par rapport à IGHAI ou par rapport aux niveaux moyens de IGHG1 et IGHG2.

6. Procédé selon l'une quelconque des revendications 1 à 5,
consistant en outre à :
réaliser un score de risque cardiovasculaire traditionnel, de préférence le score de risque cardiovasculaire traditionnel étant choisi dans le groupe constitué par les scores fonctionnels FHS 10 ans, FHS 30 ans, ICHS et BEWAT, plus préférablement
le score de risque cardiovasculaire est FHS 10 ans.

7. Procédé selon l'une quelconque des revendications 1 à 6,
qui est un procédé de dépistage et/ou de diagnostic de l'athérosclérose subclinique chez un sujet classé à faible risque pour un score de risque cardiovasculaire traditionnel.

8. Procédé selon l'une quelconque des revendications 1 à 7,
selon lequel l'échantillon biologique isolé du sujet est un échantillon de sérum, de sang ou de plasma, de préférence un échantillon de sérum.

9. Procédé selon l'une quelconque des revendications 1 à 8,
selon lequel le sujet est un sujet humain.

10. Procédé selon l'une quelconque des revendications 1 à 9,
selon lequel les étapes b) et/ou c) sont mises en œuvre par un ordinateur.

11. Appareil de traitement de données comprenant des moyens adaptés pour exécuter les étapes d'un procédé de la revendication 10.

12. Programme d'ordinateur comprenant des instructions qui, lorsque le programme est exécuté par un ordinateur, font exécuter les étapes du procédé de la revendication 10 par l'ordinateur.

13. Support de stockage lisible par ordinateur comportant un programme informatique selon la revendication 12.

14. Utilisation d'un kit pour un procédé de dépistage, de diagnostic et/ou de suivi d'un sujet selon l'une quelconque des revendications 1 à 10, le kit comprenant :
a. un réactif pour déterminer les niveaux d'expression protéique de PIGR et/ou IGHA2 ; et
b. éventuellement, un réactif pour déterminer les niveaux d'expression des protéines d'un, deux, trois, quatre ou cinq de l'ensemble APOA, HPT, HEP2, ITIH1 ou C5 ;
c. éventuellement, comprenant en outre des instructions pour l'utilisation des réactifs dans la détermination des niveaux d'expression des protéines dans un échantillon biologique isolé d'un sujet.

15. Utilisation d'un kit selon la revendication 14,
le kit comprenant des réactifs adéquats pour déterminer des niveaux d'expression protéique des biomarqueurs suivants :
i. PIGR et/ou IGHA2 ;
ii. éventuellement, un, deux, trois, quatre ou cinq biomarqueurs choisis parmi l'ensemble APOA, HPT, HEP2, ITIH1 et C5 ;
le kit comprenant :
a. un réactif d'affinité pour PIGR et/ou IGHA2 ;
b. éventuellement, un réactif d'affinité pour un, deux, trois, quatre ou cinq de l'ensemble APOA, HPT, HEP2, ITIH1 et C5 ;
c. éventuellement, comprenant en outre des instructions pour l'utilisation desdits réactifs dans la détermination des niveaux d'expression de protéines dans un échantillon biologique isolé d'un sujet.
